# FASCICULE DE BREVET EUROPEEN

(11) **EP 3 634 957 B1**
(45) Date de publication et mention de la délivrance du brevet: **07.07.2021**
(21) Numéro de dépôt: 18727799.1
(22) Date de dépôt: 29.05.2018
(51) Int. Cl.: C07D 401/14, C07D 405/14, C07D 403/06, A61P 25/00, A61K 31/404

(54) **MODULATEURS DE RECEPTEURS NMDA, COMPOSITIONS LES COMPRENANT ET UTILISATION DE CES COMPOSÉS DANS LE TRAITEMENT DE MALADIES IMPLIQUANT LE SYSTEME NERVEUX CENTRAL**
NMDA-REZEPTOR-MODULATOREN, ZUSAMMENSETZUNGEN DAMIT UND VERWENDUNG DIESER VERBINDUNGEN ZUR BEHANDLUNG VON ERKRANKUNGEN IM ZUSAMMENHANG MIT DEM ZENTRALEN NERVENSYSTEM
NMDA RECEPTOR MODULATORS, COMPOSITIONS COMPRISING SAME AND USE OF THESE COMPOUNDS IN THE TREATMENT OF DISEASES INVOLVING THE CENTRAL NERVOUS SYSTEM

(30) Priorité: 06.06.2017 FR 1754993
(43) Date de publication de la demande: 15.04.2020
(73) Titulaire: Adpuerivitam, 92160 Antony (FR)
(72) Inventeur: ALAMI, Mouâd, 77600 Bussy-Saint-Georges (FR); BRION, Jean-Daniel, 95320 Saint-Leu-La-Foret (FR); MESSAOUDI, Samir, 91380 Chilly-Mazarin (FR); TOUCHET, Sabrina, 54500 Vandoeuvre-Les-Nancy (FR); GALVANI, Gilles, 92340 Bourg-La-Reine (FR); DULAC, Olivier, 75013 Paris (FR); GATAULLINA, Svetlana, 92160 Antony (FR); NOUS, Caroline, 91760 Itteville (FR)
(74) Mandataire: Novagraaf Technologies
(86) Numéro de dépôt international: PCT/EP2018/064093
(87) Numéro de publication internationale: WO 2018/224359

(56) Documents cités:
- WO-A2-2013/063120

## Description

### DOMAINE TECHNIQUE

La présente invention concerne le domaine de la prévention et du traitement de maladies impliquant les récepteurs NMDA du système nerveux central.
Elle concerne plus précisément des composés de type 1-(indol-3-yl)-3-hydroxy-3-(2-oxo-2-éthyl)-indol-2-ones, notamment à titre de principe actif et l'utilisation de tels composés dans la préparation de compositions pharmaceutiques. Ces compositions pharmaceutiques peuvent notamment être destinées à prévenir ou à traiter des pathologies impliquant les récepteurs NMDA du système nerveux central, en particulier l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure.

### ETAT DE LA TECHNIQUE

Les récepteurs N-méthyl-D-aspartate (NMDA) sont des récepteurs ionotropes spécifiques activés dans des conditions physiologiques par le glutamate et la glycine. Les récepteurs NMDA possèdent un rôle primordial dans la transmission synaptique excitatrice et l'excitotoxicité au sein du système nerveux central. Ils sont largement présents dans tout le système nerveux central et impliqués dans de nombreux processus physiologiques tels que la mémoire, la plasticité synaptique ou encore le guidage axonal. Ainsi, ces récepteurs apparaissent comme de très bonnes cibles thérapeutiques pour diverses maladies neurologiques telles que les accidents vasculaires cérébraux, la schizophrénie, la dépression majeure, les maladies de Parkinson et d'Alzheimer ainsi que la démence (cf. a) Chen, H.-S. V.; Lipton, A. S. The chemical biology of clinically_tolerated NMDA receptor antagonists. J. Neurochem. 2006, 97, 1611-1626. b) Hallett, P. J.; Standaert, D. G. Rationale for and use of NMDA receptor antagonists in Parkinson's disease. Pharmacol. Ther. 2004, 102, 155-174. c) Vance, K. M.; N., S.; Traynelis, S. F.; Furukawa, H. Ligand specific deactivation time course of GluN1/GluN2D NMDA receptors. Nat. Commun. 2011, 2, 294. d) Goff, D. C.; Cather, C.; Gottlieb, J. D.; Evins, A. E.; Walsh, J.; Raeke, L.; Otto, M. W.; Schoenfeld, D.; Green, M. F. Once-weekly Dcycloserine effects on negative symptoms and cognition in schizophrenia: an exploratory study. Schizophr. Res. 2008, 106, 320-327).
Sept sous-unités des récepteurs NMDA ont été identifiées à ce jour: une sous-unité GluN1, quatre sous-unités GluN2 (GluN2A/B/C/D) et deux sous-unités GluN3 (GluN3A/B). La sous-unité GluN3 ne permet pas la formation d'un récepteur fonctionnel ; cependant, elle peut s'assembler avec les sous-unités GluN1/GluN2. Pour être fonctionnel, un récepteur NMDA doit être tétramérique et composé de plusieurs sous-unités GluN1 en combinaison avec au moins une sous-unité GluN2 (A, B, C ou D) ce qui permet de générer une multitude de récepteurs NMDA différents avec des propriétés pharmacologiques et biologiques propres.
Les différentes sous-unités GluN2A/B/C/D des récepteurs NMDA sont réparties différemment suivant les régions du cerveau avec des niveaux d'expression qui varient remarquablement pendant le développement. Les sous-unités GluN1 sont exprimées seulement au sein du système nerveux central alors que les sous-unités GluN2 voient leur composition et expression varier pendant le développement et suivant les régions du corps. Les sous-unités GluN2B et GluN2C sont prédominantes dans le cerveau néonatal. Cependant, au cours du développement, elles sont progressivement complétées ou remplacées par des sous-unités GluN2A et dans certaines régions par des sous-unités GluN2C. Comme les quatre sous-unités GluN2 (GluN2A/B/C/D) sont responsables des propriétés pharmacologiques et biologiques des récepteurs NMDA, une action ciblée sur une seule de ces sous-unités permettrait une efficacité précise, en termes d'effet thérapeutique et de localisation dans le cerveau, permettant de limiter les effets indésirables dus à une action globale sur les récepteurs NMDA.

La pharmacologie des récepteurs NMDA est très diversifiée, principalement à cause de l'assemblage complexe de ces sous-unités qui en font des unités singulières. Cependant, la résolution de structures cristallines de complexe GluN1/GluN2/ligand ont permis le développement d'antagonistes sélectifs des différentes sous-unités GluN2. A ce jour, la plupart des antagonistes des récepteurs NMDA entrés en phases cliniques ont échoué à cause d'effets indésirables inacceptables, tels que des effets sédatifs et hallucinogènes, aux doses minimales efficaces. Ce fut le cas de la dizocilpine (MK-801), un antagoniste non-compétitif des récepteurs NMDA. Malgré des années d'effort à développer des molécules capables d'interagir spécifiquement avec les récepteurs NMDA, seulement quelques-unes sont parvenues en phase clinique. La mémantine, un antagoniste non-compétitif, est utilisée dans le traitement de la maladie d'Alzheimer et de la démence à corps de Lewy. La kétamine est administrée principalement pour initier et maintenir une anesthésie générale. Cependant, lors d'essais cliniques récents, la kétamine a permis de traiter la dépression résistante au traitement classique. L'amantadine quant à elle est indiquée en tant qu'antiviral ainsi que dans le traitement de la maladie de Parkinson.

Il existe donc un besoin pour de nouveaux composés antagonistes des récepteurs NMDA, et plus largement pour des modulateurs de ces récepteurs.

### DEFINITIONS

Pour faciliter la compréhension de l'invention, un certain nombre de termes et expressions sont définis ci-dessous:
De manière générale, le terme « substitué », précédé ou non du terme « éventuellement », et les substituants décrits dans les formules du présent document, désignent le remplacement d'un radical hydrogène dans une structure donnée avec le radical d'un substituant spécifié. Le terme « substitué » désigne par exemple le remplacement d'un radical hydrogène dans une structure donnée par un radical R. Lorsque plus d'une position peut être substituée, les substituants peuvent être les mêmes ou différents à chaque position.

On entend par « alkyle » au sens de la présente invention, un radical carboné saturé linéaire, ramifié ou cyclique, éventuellement substitué, comprenant 1 à 25 atomes de carbone, par exemple 1 à 10 atomes de carbone, par exemple 1 à 8 atomes de carbone, par exemple 1 à 6 atomes de carbone. Par exemple les groupes alkyle incluent, sans s'y limiter, méthyle, éthyle, n-propyle, isopropyle, n-butyle, sec-butyle, isobutyle, tert-butyle, n-pentyle, sec-pentyle, isopentyle, tert-pentyle, n-hexyle, sec-hexyle, etc.

On entend par « haloalkyle » au sens de la présente invention, un radical aklyle tel que défini ci-dessus, substitué par au moins un atome d'ahalogène. Par exemple les groupes haloalkyle incluent, sans s'y limiter, chlorométhyle, bromométhyle, trifluorométhyle, etc.

On entend par « aryle » au sens de la présente invention, un système aromatique comprenant au moins un cycle satisfaisant la règle d'aromaticité de Hückel. Ledit aryle est éventuellement substitué et peut comprendre de 6 à 50 atomes de carbone, par exemple 6 à 20 atomes de carbone, par exemple 6 à 10 atomes de carbone. Par exemple, on peut citer phényle, indanyle, indényle, naphthyle, phénanthryle et anthracyle.

On entend par « hétéroaryle » au sens de la présente invention, un système comprenant au moins un cycle aromatique de 5 à 50 chaînons parmi lesquels au moins un chaînon du cycle aromatique est un hétéroatome, notamment choisi dans le groupe comprenant le soufre, l'oxygène, l'azote, le bore. Ledit hétéroaryle est éventuellement substitué et peut comprendre de 1 à 50 atomes de carbone, de préférence 1 à 20 atomes de carbone, de préférence 3 à 10 atomes de carbone. Par exemple, on peut citer pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, et similaire. Par exemple, on peut citer pyridyle, quinolinyle, dihydroquinolinyle, isoquinolinyle, quinazolinyle, dihydroquinazolyle, et tetrahydroquinazolyle.

On entend par « arylalkyle » au sens de la présente invention, un substituant aryle lié au reste de la molécule via un radical alkyle. Une convention analogue est utilisée pour « hétéroarylalkyle ».

On entend par « alkoxyle » au sens de la présente invention, un substituant alkyle tel que défini précédemment lié au reste de la molécule via un atome d'oxygène. Par exemple, on peut citer méthoxyle, éthoxyle, etc..

Le terme «halogène» au sens de la présente invention, désigne un atome choisi parmi le fluor, le chlore, le brome et l'iode.

On entend par «indépendamment» au sens de la présente invention, le fait que les substituants, atomes ou groupes auxquels ce terme se réfère, sont choisis parmi la liste des variables indépendamment l'une de l'autre (à savoir, ils peuvent être identiques ou différents).

### DESCRIPTION DE CERTAINS MODES DE RÉALISATION AVANTAGEUX DE L'INVENTION

Ainsi, selon un premier aspect, l'invention a pour objet les composés isolés répondant à la formule **(I)** suivante ou un de ses sels pharmaceutiquement acceptables: 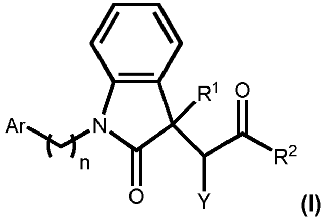 dans laquelle :
**R¹** représente H ou OH ; de préférence OH
**R²** représente CN, NH₂, OH ou un radical C₁₋₆alkyle linéaire, ramifié ou cyclique ; C₆₋₁₀aryle éventuellement substitué par un ou plusieurs substituants choisis parmi un atome d'halogène, un radical C₁₋₆alkyle linéaire, ramifié ou cyclique, un radical C₁₋₆alkoxyle linéaire, ramifié ou cyclique, un radical C₁₋₆haloalkyle linéaire, ramifié ou cyclique, un radical C₁₋₆haloalkoxyle linéaire, ramifié ou cyclique ; un radical C₆₋₁₀hétéroaryle éventuellement substitué par un ou plusieurs substituants choisis parmi un atome d'halogène, un radical C₁₋₆alkyle linéaire, ramifié ou cyclique, un radical C₁₋₆alkoxyle linéaire, ramifié ou cyclique, un radical C₁₋₆haloalkyle linéaire, ramifié ou cyclique, un radical C₁₋₆haloalkoxyle linéaire, ramifié ou cyclique ; ou un radical C₂₋₅hétérocycle saturé ou insaturé comprenant de 1 à 3 hétéroatome(s) choisi(s) dans le groupe comprenant O, N et S,
**Y** représente H ou un radical C₁₋₆alkyle linéaire, ramifié ou cyclique ; ou bien Y pris ensemble avec **R²** forme un cycle à 5 ou 6 chainons ;
**n** représente 0, 1, 2, 3 ou 4 ; de préférence n représente 2 ou 3
**Ar** représente un hétérocyle bicyclique de structure : 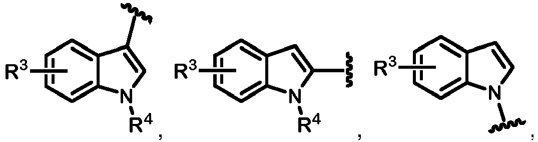 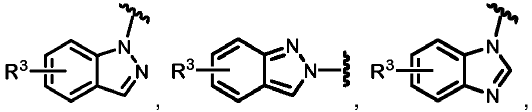 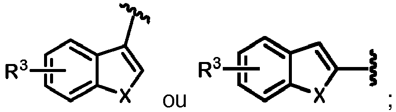 dans laquelle :
   **R³** représente H, un atome d'halogène, un radical -OR⁵ ou -NR^{6A}R^{6B}; dans lequel R⁵ représente un radical H, C₁₋₆alkyle linéaire, ramifié ou cyclique ; et R^{6A} et R^{6B} représentent indépendamment H, C₁₋₆alkyle linéaire, ramifié ou cyclique ou C₆₋₁₀aryle ;
   **R⁴** représente H ; un radical C₁₋₆alkyle linéaire, ramifié ou cyclique ; ou un radical -C(=O)R⁷ dans lequel R⁷ représente un radical C₁₋₆alkyle linéaire, ramifié ou cyclique ; et
   **X** représente O ou S.

Par exemple, n peut être avantageusement égale à 2 ou 3.

Par exemple R¹ peut avantageusement représenter OH.

Par exemple Y peut représenter H, méthyle, ou bien Y pris ensemble avec R² peut former un cycle à 5 chainons. Avantageusement, Y peut représenter H.

Lorsque forme avec R² un cycle à 5 ou 6 chainons, le carbone en alpha de la fonction cétone qui porte Y peut être un centre de chiralité, par exemple de configuration R ou S, ou bien racémique.

Par exemple Ar peut représenter hétérocyle bicyclique de structure suivante : 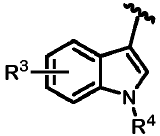 dans laquelle
R³ représente H ; un atome d'halogène choisi parmi F, Cl ou Br, de préférence Cl; un radical -OR⁵ où R⁵ représente H, méthyle ou éthyle ; ou un radical -NR^{6A}R^{6B} où R^{6A} et R^{6B} représentent indépendamment H, méthyle ou éthyle; et
R⁴ représente H ou méthyle.

Par exemple Ar peut également représenter un hétérocyle bicyclique de structure suivante : 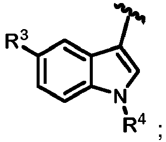 dans laquelle
R³ représente H ; un atome d'halogène choisi parmi F, Cl ou Br, de préférence Cl; un radical -OR⁵ où R⁵ représente H, méthyle ou éthyle ; ou un radical -NR^{6A}R^{6B} où R^{6A} et R^{6B} représentent indépendamment H, méthyle ou éthyle; et
R⁴ représente H ou méthyle.

A titre d'exemple, R² représente :
- méthyle, éthyle, propyle, n-butyle, iso-propyle, iso-butyle, cyclopropyle, cyclobutyle, cyclopentyle ou cyclohexyle ;
- un radical phényle éventuellement substitué par un ou plusieurs substituants choisis parmi F, Cl, Br, méthyle, éthyle, CN, CF₃, OH, méthoxy, NH₂ ou OCF₃; de préférence un radical phényle non substitué ou mono-substitué en ortho, méta ou para par un substituant choisi parmi F, Cl, Br, méthyle, éthyle, CN, CF₃, OH, méthoxy, NH₂ ou OCF₃, ou
- un radical pyridyle éventuellement substitué par un ou plusieurs substituants choisis parmi F, Cl, Br, méthyle, éthyle, CF₃, OH, méthoxy, NH₂ ou OCF₃; de préférence un radical pyridyle 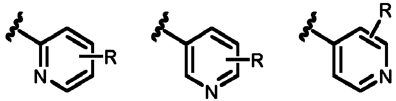 éventuellement substitué par un substituant R choisi parmi H, F, Cl, Br, méthyle, éthyle, CF₃, OH, méthoxy, NH₂ ou OCF₃, de préférence H ou
- un radical benzofuryle de structure 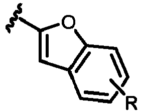 éventuellement substitué par un ou plusieurs substituants R choisis parmi F, Cl, Br, méthyle, éthyle, CN, CF₃, OH, méthoxy, NH₂ ou OCF₃, ou
- un radical C₄₋₅hétérocycle saturé ou insaturé comprenant de 1 à 3 hétéroatome choisi dans le groupe comprenant O et S,
- un radical furyle de structure 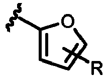 éventuellement substitué par un ou plusieurs substituants R choisis parmi F, Cl, Br, méthyle, éthyle, CN, CF₃, OH, méthoxy, NH₂ ou OCF₃, de préférence un radical furyl de structure 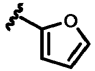
- un radical thiophene de structure 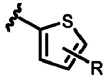 éventuellement substitué par un ou plusieurs substituants R choisis parmi F, Cl, Br, méthyle, éthyle, CN, CF₃, OH, méthoxy, NH₂ ou OCF₃, ou
- un radical benzofuryle de structure 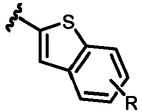 éventuellement substitué par un ou plusieurs substituants R choisis parmi F, Cl, Br, méthyle, éthyle, CN, CF₃, OH, méthoxy, NH₂ ou OCF₃, de préférence un radical benzofuryle de structure

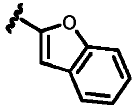

Avantageusement, les composés selon l'invention peuvent répondre à répondant à la formule **(I)** suivante ou un de ses sels pharmaceutiquement acceptables: 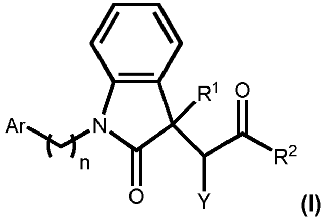 dans laquelle
**R¹** représente OH
Ar peut également représenter un hétérocyle bicyclique de structure suivante : 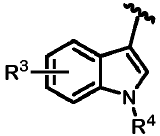 dans laquelle R³ représente H ou Cl; R⁴ représente H ou méthyle,
**R²** représente un radical phényle non substitué ou mono-substitué en ortho, méta ou para par un substituant choisi parmi F, Cl, Br, méthyle, éthyle, CN, CF₃, OH, méthoxy, NH₂ ou OCF₃,
**Y** représente H ou methyl, et
n est égal à 2

Avantageusement, les composés selon l'invention peuvent répondre à répondant à la formule **(I)** suivante ou un de ses sels pharmaceutiquement acceptables: 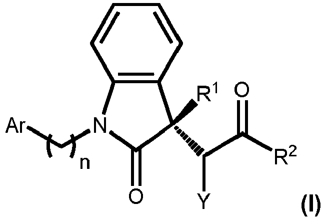 dans laquelle
**R¹** représente OH
**Ar** représente un un hétérocyle bicyclique de structure suivante : 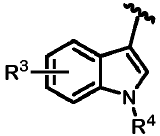 dans laquelle R³ représente H ou Cl; R⁴ représente H ou méthyle,
**R²** représente éthyl, n-butyle, iso-propyl, iso-butyl, un radical pyridyle non substitué, un radical C₃₋₆ alkyle cyclique, un radical furyl de structure 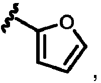 un radical thiophène de structure 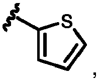 ou un radical benzofuryl de structure 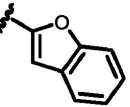
**Y** représente H, et
n est égal à 2

Avantageusement, les composés selon l'invention peuvent répondre à répondant à la formule **(I)** suivante ou un de ses sels pharmaceutiquement acceptables: 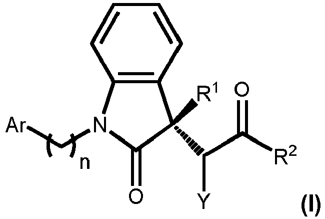 dans laquelle
**R¹** représente OH
**Ar** représente un un hétérocyle bicyclique de structure suivante : 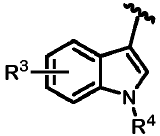 dans laquelle R³ représente H; R⁴ représente H,
**R²** représente un radical phényle mono-substitué en ortho ou méta par un substituant choisi parmi F, Cl
**Y** représente H, et
n est égal à 2

Avantageusement, les composés selon l'invention peuvent répondre à répondant à la formule **(I)** suivante ou un de ses sels pharmaceutiquement acceptables: 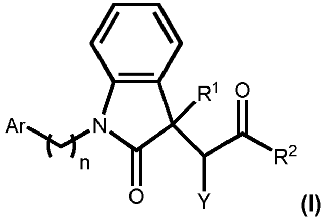 dans laquelle
**R¹** représente OH
Ar peut également représenter un hétérocyle bicyclique de structure suivante : 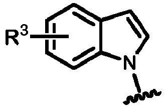 dans laquelle R³ représente H ou Cl,
**R²** peut représenter un radical phényle non substitué ou mono-substitué en ortho, méta ou para par un substituant choisi parmi F, Cl, Br, méthyle, éthyle, CN, CF₃, OH, méthoxy, NH₂ ou OCF₃,
**Y** représente H ou methyl, et
n est égal à 2

Avantageusement, les composés selon l'invention peuvent répondre à répondant à la formule **(I)** suivante ou un de ses sels pharmaceutiquement acceptables: 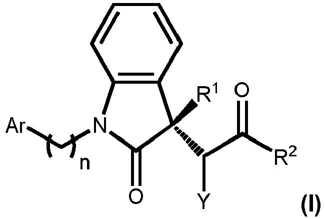 dans laquelle
**R¹** représente OH
**Ar** peut également représenter un hétérocyle bicyclique de structure suivante : 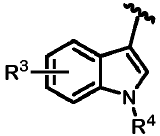 dans laquelle R³ représente H ou Cl; R⁴ représente H,
**R²** peut représenter un radical phényle non substitué ou mono-substitué en ortho, méta ou para par un substituant choisi parmi F, Cl, Br, méthyle, éthyle, CN, CF₃, OH, méthoxy, NH₂ ou OCF₃,
**Y** représente H ou methyl, et
n est égal à 3

Avantageusement, les composés selon l'invention peuvent répondre à l'une des structures suivantes:

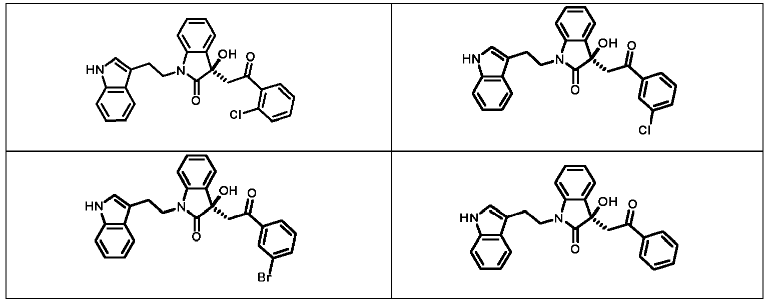

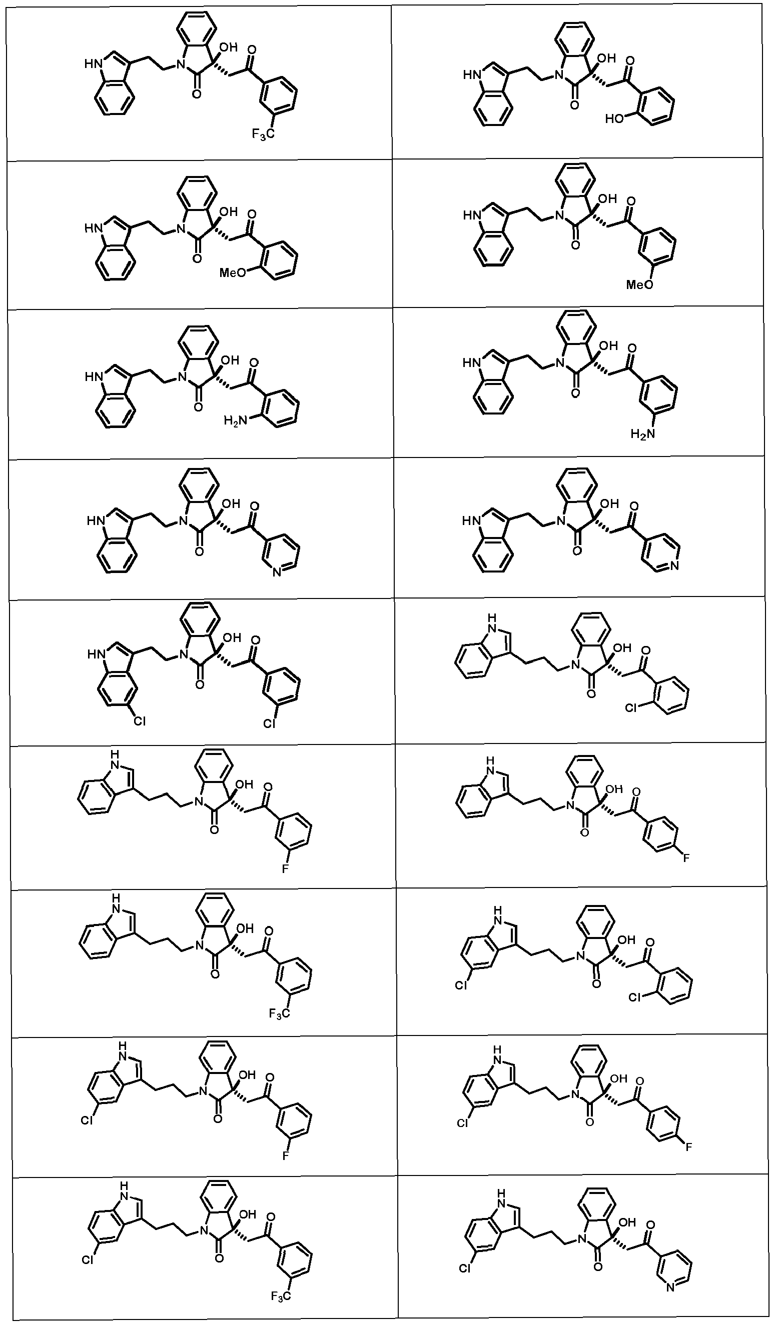

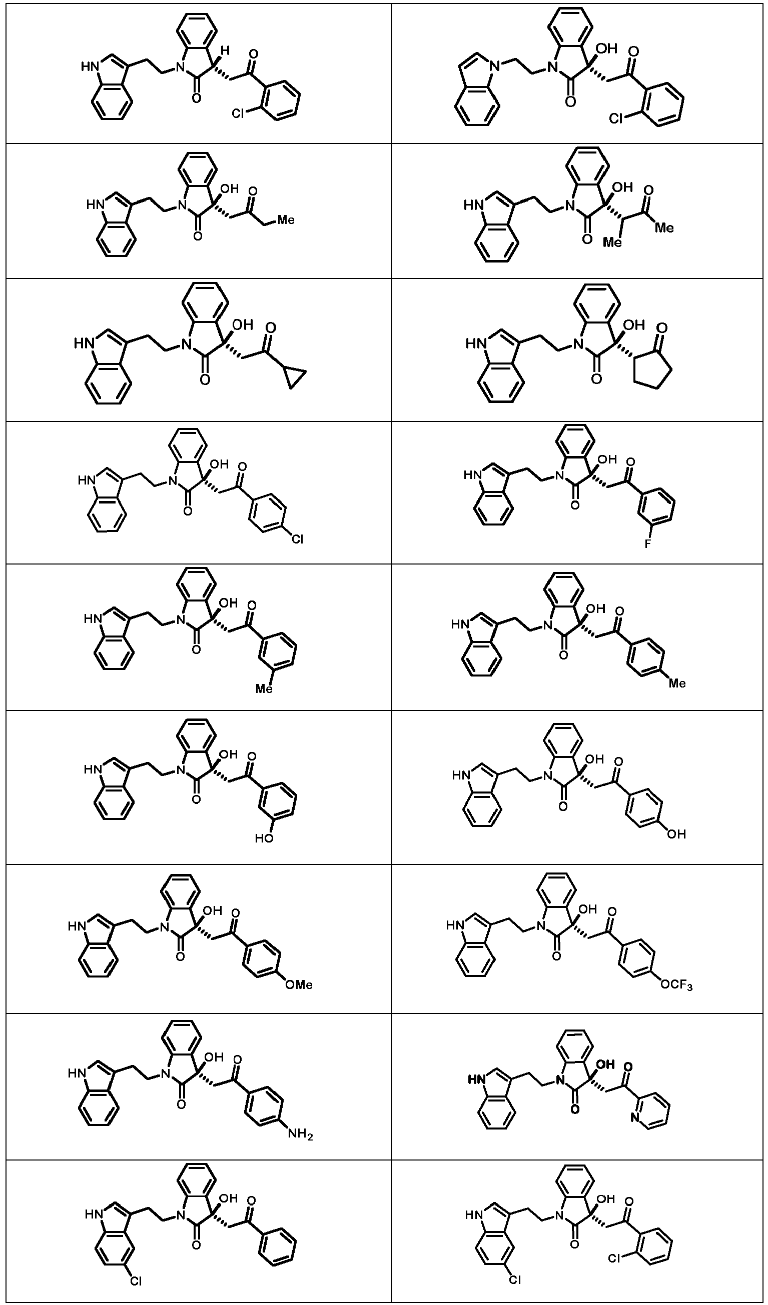

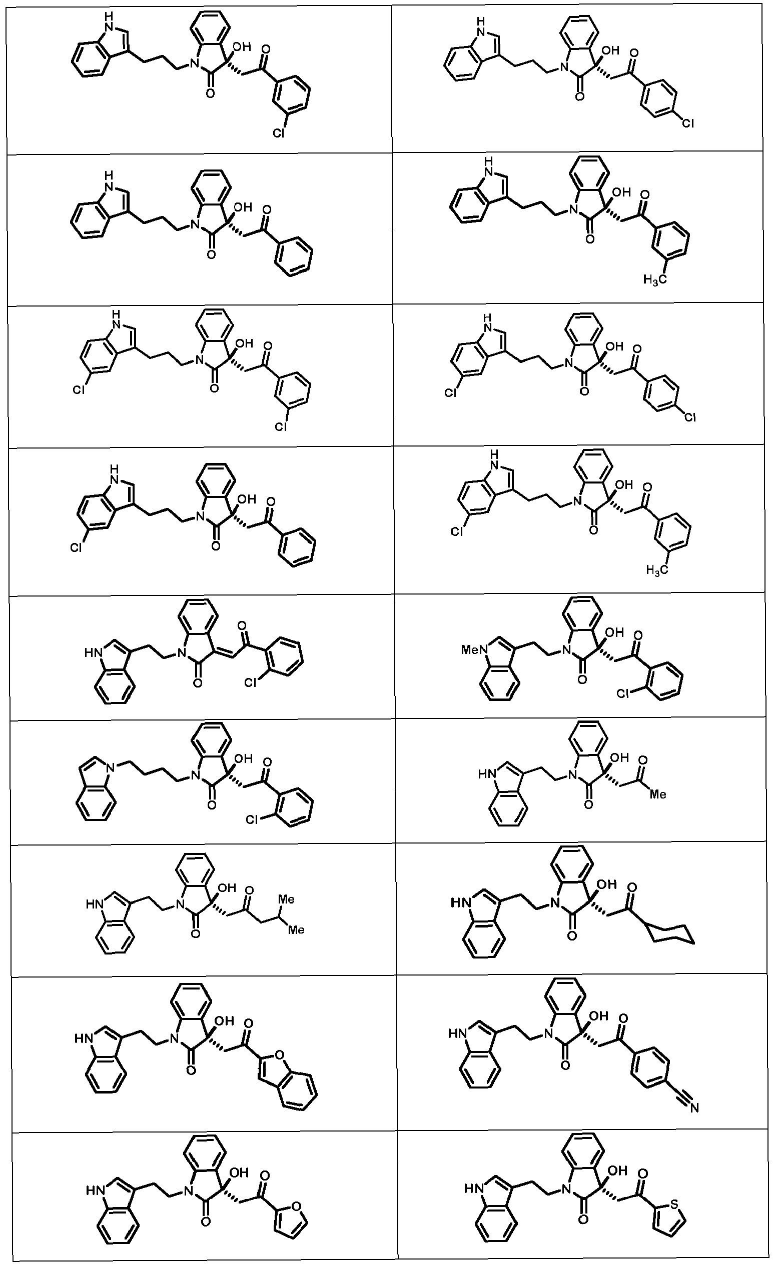

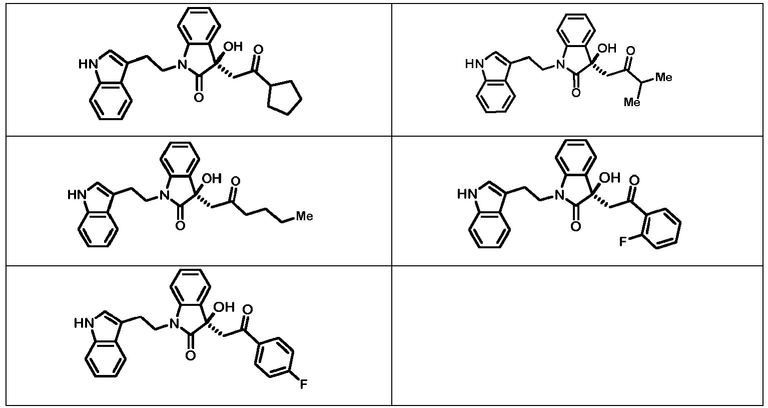

Le terme « isolé », lorsqu'il est utilisé pour caractériser les composés selon l'invention, désigne des composés qui sont (i) séparés d'au moins un composé avec lequel ils sont associés dans la nature, et/ou (ii) produits, préparés ou fabriqués par les soins de l'homme.
Des composés selon l'invention pourraient comprendre un ou plusieurs centres asymétriques et peuvent donc exister sous diverses formes isomères, par exemple, énantiomères et / ou diastéréomères. Ainsi, les composés selon l'invention et des compositions pharmaceutiques de ceux-ci peuvent être sous la forme d'un énantiomère individuel, diastéréomère ou isomère géométrique, ou peuvent être sous la forme d'un mélange de stéréo-isomères. Avantageusement, les composés selon l'invention peuvent être des composés énantiomériquement purs. Alternativement, les composés selon l'invention peuvent être sous forme de mélanges de stéréoisomères ou de diastéréomères.
En outre, sauf indication contraire, des composés tels que décrits dans le présent document peuvent avoir une ou plusieurs doubles liaisons qui peuvent exister sous forme d'isomères Z ou E. L'invention englobe en outre les composés tels que décrits dans la présente, sous forme d'isomères individuels essentiellement dépourvus d'autres isomères et, alternativement, sous forme de mélanges de divers isomères, par exemple des mélanges racémiques de stéréoisomères. En plus des composés mentionnés ci-dessus en tant que tels, la présente invention englobe également les dérivés pharmaceutiquement acceptables de ces composés et des compositions comprenant un ou plusieurs composés selon l'invention et un ou plusieurs excipients pharmaceutiquement acceptables ou des additifs.
Les composés selon l'invention peuvent être préparés par cristallisation d'un composé de formule (I) ou l'une quelconque des sous-formules décrites dans la présente invention. Ceux-ci peuvent exister sous la forme d'un polymorphe unique ou une combinaison de polymorphes du composé de formule générale (I) faisant partie de la présente invention. Par exemple, différents polymorphes peuvent être identifiés et / ou préparés en utilisant différents solvants ou différents mélanges de solvants pour la recristallisation ; en effectuant la cristallisation à des températures différentes ; ou en utilisant différents modes de refroidissement, allant de très rapide à un refroidissement très lent pendant la cristallisation. Les polymorphes peuvent également être obtenus par chauffage ou fusion du composé, puis par refroidissement progressif ou rapide. La présence de polymorphes peut être déterminée par spectroscopie RMN du solide, spectroscopie IR, calorimétrie différentielle à balayage, diffractométrie aux rayons X et / ou d'autres techniques appropriées. Ainsi, la présente invention englobe les composés selon l'invention, leurs dérivés, leurs formes tautomères, leurs stéréoisomères, leurs polymorphes, leurs sels pharmaceutiquement acceptables, leurs formes solvatées pharmaceutiquement acceptables et des compositions pharmaceutiquement acceptables les contenant.

### 2) Stratégies générales de synthèse

La personne du métier dispose d'une solide littérature de la chimie des indoles de laquelle il peut tirer parti, en combinaison avec les informations contenues dans ce document, pour des renseignements sur les stratégies de synthèse, les groupes protecteurs, et d'autres matériaux et des méthodes utiles pour la synthèse des composés de cette invention, y compris les composés contenant les différents substituants R₁, R₂, R₃, R₄, R₅, et R₆.
Les différents documents de brevets et autres références citées dans le présent document fournissent des informations générales utiles sur la préparation des composés similaires aux composés selon l'invention décrits dans la présente, ou intermédiaires pertinents.
De plus, la personne du métier pourra se référer à l'enseignement et aux exemples spécifiques fournis dans le présent document, se rapportant à divers exemples de composés et leurs intermédiaires, pour mettre en œuvre la présente invention dans toute sa portée.
Comme cela est décrit ci-dessus, la présente invention porte sur de nouveaux composés, spécifiquement les composés ayant la structure générale suivante : 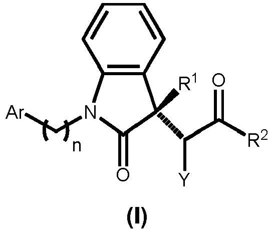 laquelle R₁, R₂, Ar, n et Y sont tels que définis précédemment.

Selon un autre aspect de l'invention, des procédés de préparation des composés de formules (I) sont fournis, des modes de réalisation de ces procédés étant généralement représentés dans les schémas 1 ou 2 ci-dessous, le schéma 2 correspondant au schéma 1 complété par une réaction de déshydratation et de réduction de l'oléfine :

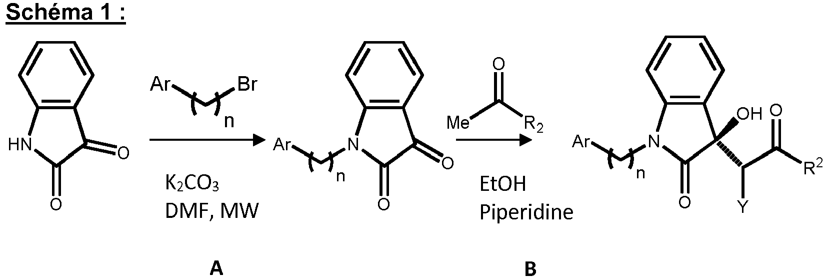

Par exemple, les étapes A et B peuvent être réalisées selon le procédé décrit respectivement dans Nguyen Dinh Thanh; Nguyen Thi Kim Giang "Reaction of N-alkylisatins with 4-(2,3,4,6-tetra-O-acetyl-β-D-glucopyranosyl)thiosemicarbazide" Hindawi Publishing Corporation Journal of Chemistry 2013, et López-Alvarado, P.; Avendaño, C. "New Diastereoselective Synthesis of 3-Alkylidene-1-methyloxindoles" Synthesis, 2002, 1, 104-110.

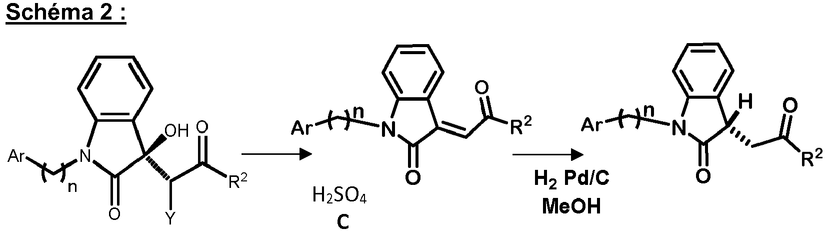

Par exemple, l'étape C peut être réalisée selon le procédé décrit dans le document Nagle, A. A.; Reddy, S. A.; Bertrand, H.; Tajima, H.; Dang, T.-M.; Wong, S.-C.; Hayes, J. D.; Wells, G.; Chew, E.-H. "3-(2-Oxoethylidene)indolin-2-one Derivatives Activate Nrf2 and Inhibit NF-kB: Potential Candidates for Chemoprevention" ChemMedChem 2014, 9, 1763-1774.

Par exemple, lorsque le groupement Ar est un indole, les composés selon l'invention peuvent être préparés selon le schéma 3A, 3B ou 3C ci-dessous : 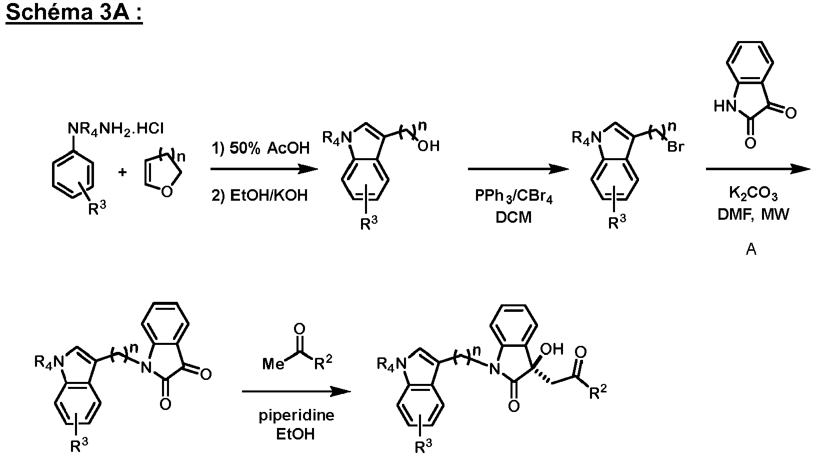 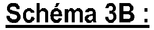 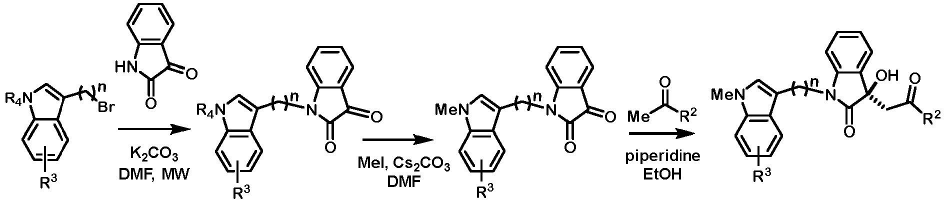 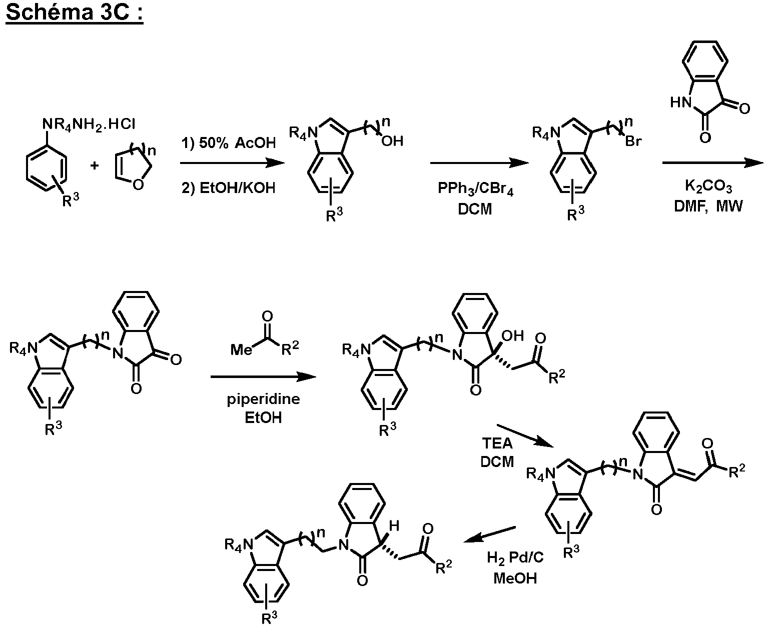 Par exemple, lorsque le groupement Ar est un groupement de formule 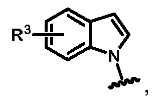 les composés selon l'invention peuvent être préparés selon le schéma 4 ci-dessous :

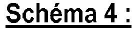

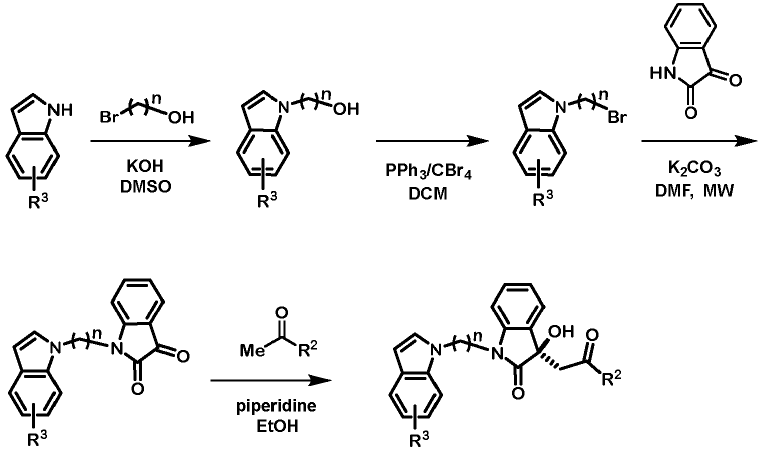

Par exemple, lorsque le groupement Ar est un groupement de formule 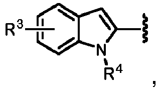 les composés selon l'invention peuvent être préparés selon le schéma 5 ci-dessous :

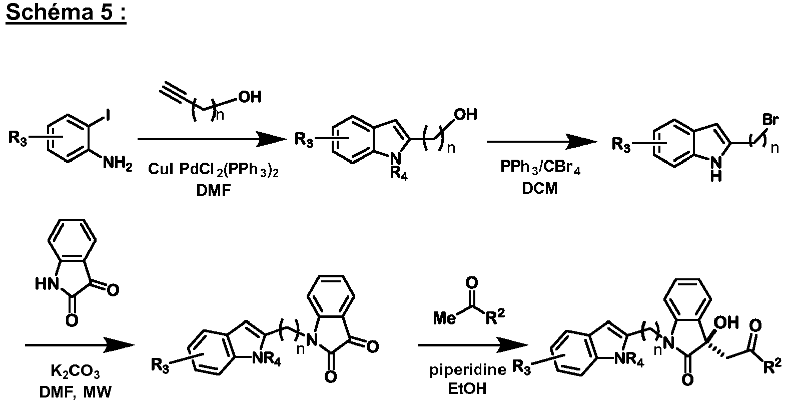

Par exemple, lorsque le groupement Ar est un groupement de formule 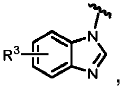 les composés selon l'invention peuvent être préparés selon le schéma 6 ci-dessous :

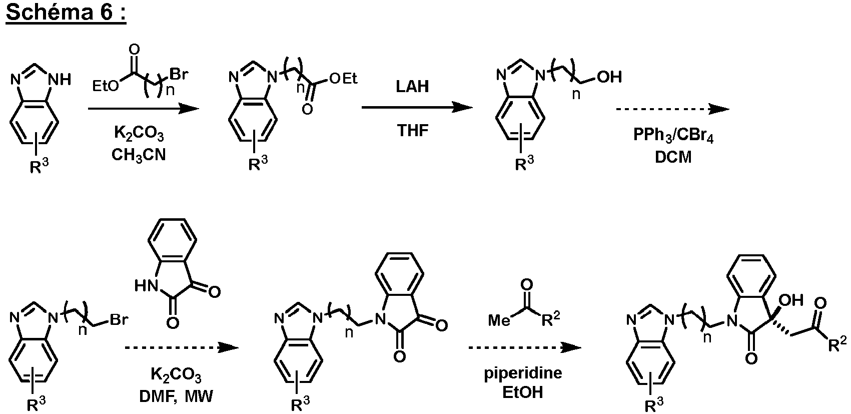

Par exemple, lorsque le groupement Ar est un groupement de formule 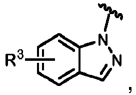 les composés selon l'invention peuvent être préparés selon le schéma 7 ci-dessous :

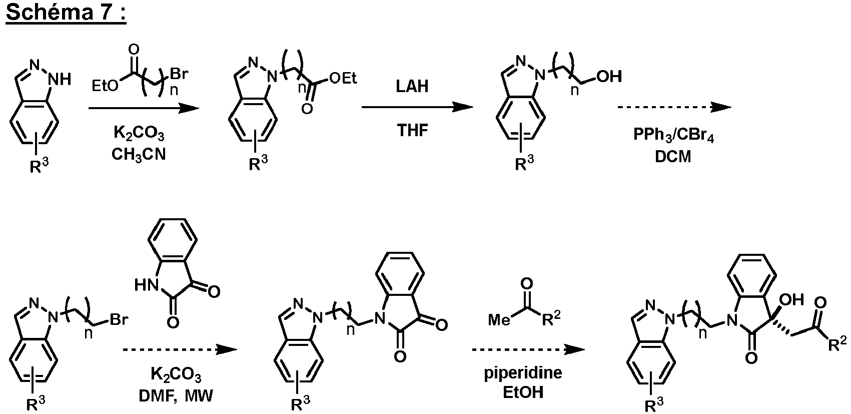

Par exemple, lorsque le groupement Ar est un groupement de formule 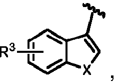 les composés selon l'invention peuvent être préparés, lorsque X représente O, selon le schéma 8 ci-dessous :

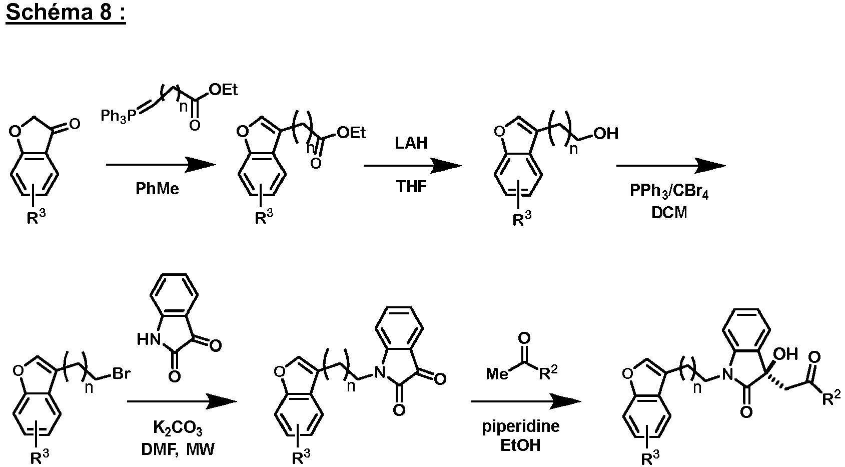

Par exemple, lorsque le groupement Ar est un groupement de formule 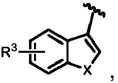 les composés selon l'invention peuvent être préparés, lorsque X représente S, selon le schéma 9 ci-dessous :

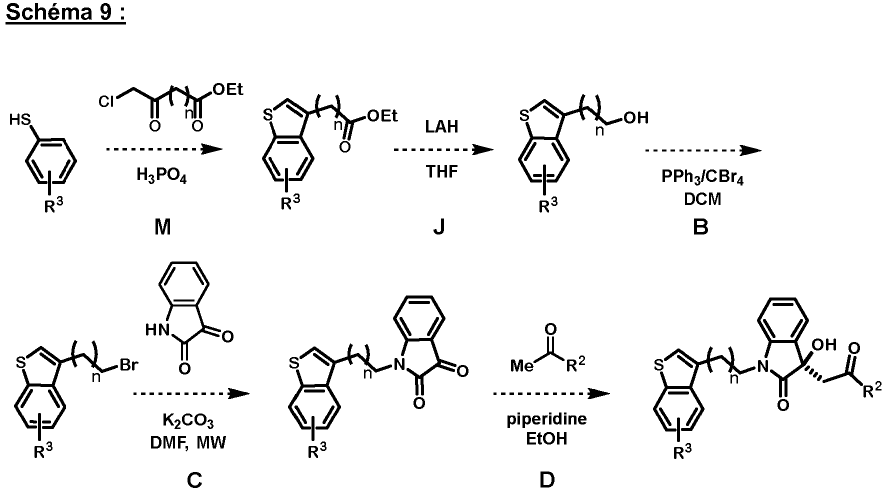

Par exemple, lorsque le groupement Ar est un groupement de formule 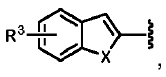 les composés selon l'invention peuvent être préparés, lorsque X représente O, selon le schéma 10 ci-dessous :

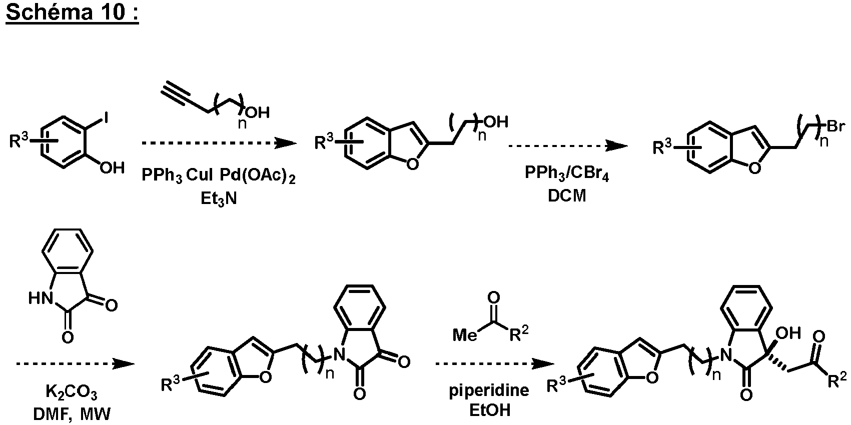

Par exemple, lorsque le groupement Ar est un groupement de formule 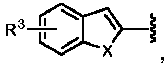 les composés selon l'invention peuvent être préparés, lorsque X représente S, selon le schéma 11 ci-dessous :

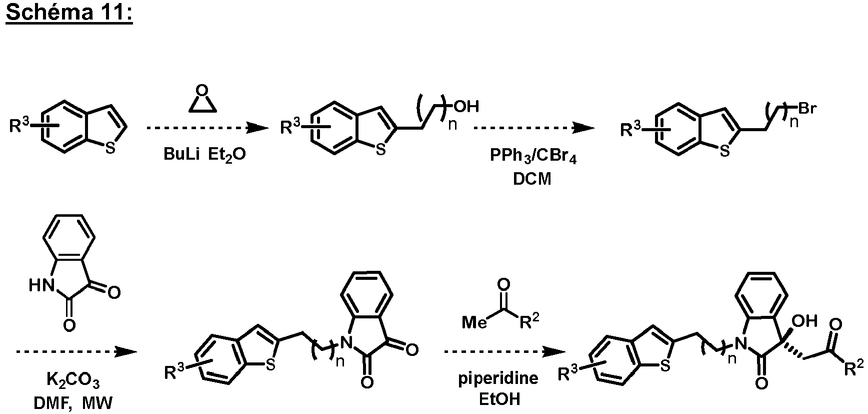

Les synthons précédents, ainsi que leurs homologues, peuvent être préparés par des méthodes connues de l'art. Par exemple, le lecteur pourra se référer aux publications répertoriées dans le tableau suivant :

| **Synthons** | **Références** |
|---|---|
| 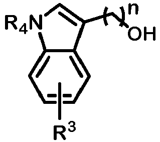 | approach for the synthesis of indole-3-propanol and its acetates from dihydropyran" Monatsh Chem. 2008, 139,1475-1478. |
| | Campos, K. R.; Woo, J. C. S.; Lee, S.; Tillyer, R. D. "A General Synthesis of Substituted Indoles from Cyclic Enol Ethers and Enol Lactones" Org. Lett. 2004, 6, 79-82. |
| 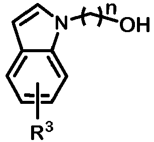 | Yang, J.-M.; Li, P.-H.; Wei, Y.; Tang, X.-Y.; Shi, M. « Gold(I)-catalyzed highly stereoselective synthesis of polycyclic indolines: the construction of four contiguous stereocenters » Chem. Commun. 2016, 52, 346-349. |
| | Kounosuke, O.; Abe, J.; Kanai, M. « Manganese-catalyzed aerobic dehydrogenative cyclization toward ring-fused indole skeletons » Org. Biomol. Chem. 2013, 11, 4569-4572. |
| 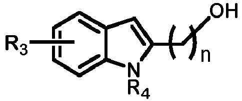 | Campaigne, E.; Homfeld, E. « Benzo[b]thiophene derivatives. XXV.Condensation and reductive alkylation of 3-aminoalkylbenzo[b]thiophenes with formaldehyde » J. Heterocycl. Chem. 1979, 16, 1321-1324. |
| 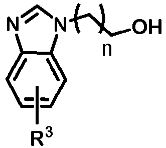 | |
| 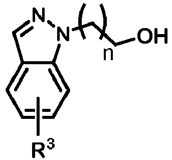 | Zhang , H. C.; Ye , H.; Moretto , A. F.; Brumfield, K. K.; Maryanoff, B. E. « Facile Solid-Phase Construction of Indole Derivatives Based on a Traceless, Activating Sulfonyl Linker » Org. Lett. 2000, 2, 89-92. |
| | Lee, S. S.; Shen, W.; Zheng, X. ; Jacobsen, I. C. « Preparation of tetrahydropyridinylthiophenecarboxylic acid derivatives for use as hepatitis c virus polymerase inhibitors »WO2014055142 A1. |
| 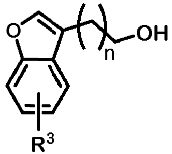 | Caroff, E.; Keller, M.; Kimmerlin, T.; Meyer, E.; « Preparation of 4-(benzoimidazol-2-yl)thiazole compounds and related aza derivatives as modulators of the CXCR3 receptor » WO2013114332 A1. |
| | Kruegel, A. C.; Rakshit, S.; Li, X.; Sames, D.; « Constructing Iboga Alkaloids via C-H Bond Functionalization: Examination of the Direct and Catalytic Union of Heteroarenes and Isoquinuclidine Alkenes » J. Org. Chem. 2015, 80, 2062-2071. |
| 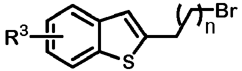 | Van Epps, D. E.; Jiang, G.-L.; Collette, A. L.; Horan, R.L.; Chen, J. S.; Altman, G. H.; Im, W.-B. « Compositions and improved soft tissue replacement methods » WO2013123272 A1. |
| | Contour-Galcéra, M.-O. ; Sidhu, A.; Plas, P. ; Roubert, P. "3-Thio-1,2,4-triazoles, novel somatostatin sst2/sst5 agonists" Bioorg. Med. Chem. Lett. 2005, 15, 3555-3559. |
| 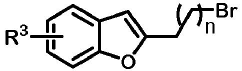 | Rong, Z.; Wang, W.; Jiang, Z.j.; Wang, K.; Zheng, X.-I.; Fu, H.-y.; Chen, H.; Li, R.-x. « One-pot synthesis of 2-substituted benzo[b]furans via Pd-tetraphosphinecatalyzed coupling of 2-halophenols with alkynes » Chem. Commun. 2014, 50, 6023-6026. |
| | Banerjee, T. S.; Paul, S.; Sinha, S.; Das, S. « Synthesis of iboga-like isoquinuclidines: Dual opioid receptors agonists having antinociceptive properties » Bioorg. Med. Chem. 2014, 22, 6062-6070. |
| 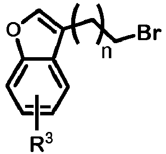 | Kruegel, A. C.; Rakshit, S.; Li, X.; Sames, D.; « Constructing Iboga Alkaloids via C-H Bond Functionalization: Examination of the Direct and Catalytic Union of Heteroarenes and Isoquinuclidine Alkenes » J. Org. Chem. 2015, 80, 2062-2071. |
| | Contour-Galcéra, M.-O. ; Sidhu, A. ; Plas, P. ; Roubert, P. "3-Thio-1,2,4-triazoles, novel somatostatin sst2/sst5 agonists" Bioorg. Med. Chem. Lett. 2005, 15, 3555-3559. |
| | Campaigne, E.; Homfeld, E. « Benzo[b]thiophene derivatives. XXV.Condensation and reductive alkylation of 3-aminoalkylbenzo[b]thiophenes with formaldehyde » J. Heterocycl. Chem. 1979, 16, 1321-1324. |
| 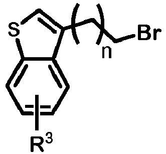 | Zhang , H. C.; Ye , H.; Moretto , A. F.; Brumfield, K. K.; Maryanoff, B. E. « Facile Solid-Phase Construction of Indole Derivatives Based on a Traceless, Activating Sulfonyl Linker » Org. Lett. 2000, 2, 89-92. |
| | Contour-Galcéra, M.-O. ; Sidhu, A. ; Plas, P. ; Roubert, P. "3-Thio-1,2,4-triazoles, novel somatostatin sst2/sst5 agonists" Bioorg. Med. Chem. Lett. 2005, 15, 3555-3559. |

### 3) Compositions et utilisations

Comme indiqué précédemment, la présente invention fournit des composés modulateurs des récepteurs NMDA et, par conséquent, les présents composés sont utiles pour le traitement des maladies, troubles et pathologies, impliquant ces récepteurs du système nerveux central, y compris mais sans s'y limiter, l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure. En conséquence, selon un autre aspect de la présente invention, des compositions pharmaceutiquement acceptables sont fournies, lesquelles comprennent l'un quelconque des composés tels que décrits précédemment, et comprennent éventuellement un support pharmaceutiquement acceptable, un adjuvant ou un véhicule. Avantageusement, ces compositions peuvent comprendre en outre éventuellement un ou plusieurs agents thérapeutiques supplémentaires, notamment ceux connus et utilisés en particulier pour le traitement de l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure.
Dans la présente invention, le terme «sel pharmaceutiquement acceptable» désigne des sels appropriés pour une utilisation pharmaceutique humaine ou vétérinaire sans toxicité, irritation, réponse allergique ou autre effet délétère impropre à une utilisation médicamenteuse et présentent un rapport bénéfice / risque raisonnable. Un «sel pharmaceutiquement acceptable» désigne tout sel non toxique ou un sel d'un ester d'un composé de la présente invention qui, après administration à un sujet, est capable de fournir, directement ou indirectement, un composé de la présente invention ou un métabolite ou un résidu de celui-ci, modulateur des récepteurs NMDA. Tel qu'il est utilisé ici, le terme «métabolite ou un résidu de celui-ci, modulateur des récepteurs NMDA» signifie qu'un métabolite ou résidu de celui-ci est également modulateur des récepteurs NMDA, comme les composés de la présente invention.
Les sels pharmaceutiquement acceptables sont bien connus dans le domaine médical. Par exemple, S. M. Berge et al., décrivent des sels pharmaceutiquement acceptables en détail dans J. Pharmaceutical Sciences, 1977, 66, 1-19, lequel est incorporé dans le présent document par référence. Les sels pharmaceutiquement acceptables des composés de la présente invention comprennent ceux dérivés d'acides et de bases inorganiques et organiques appropriés. Des exemples de sels d'addition pharmaceutiquement acceptables non toxiques, comprennent des sels d'un groupe aminé formé avec des acides inorganiques tels que l'acide chlorhydrique, l'acide bromhydrique, l'acide phosphorique, l'acide sulfurique ou avec des acides organiques tels que l'acide acétique, l'acide oxalique, l'acide maléique, l'acide tartrique, l'acide citrique, l'acide succinique ou l'acide malonique ou en utilisant d'autres procédés utilisés dans l'art tels que l'échange d'ions. D'autres sels pharmaceutiquement acceptables comprennent l'adipate, l'alginate, l'ascorbate, l'aspartate, le benzènesulfonate, le benzoate, le bisulfate, le borate, le butyrate, le camphorate, le camphorsulfonate, le citrate, le cyclopentylpropionate, le gluconate, le dodécylsulfate, l'éthanesulfonate, le formiate, le fumarate, le glucoheptonate, le glycérophosphate, le gluconate, l'hémisulfate, l'heptanoate, l'hexanoate, l'iodhydrate, le 2-hydroxy-éthanesulfonate, le lactobionate, le lactate, le laurate, le laurylsulfate, le malate, le maléate, le malonate, le méthanesulfonate, le 2-naphtalènesulfonate, le nicotinate, le nitrate, l'oléate, l'oxalate, le palmitate, le pamoate, le pectinate, le persulfate, le 3 -phénylpropionate, le phosphate, le pivalate, le propionate, le stéarate, le succinate, le sulfate, le tartrate, le p-toluènesulfonate, l'undécanoate, le valérate et analogues. Les sels dérivés de bases appropriés comprennent les sels de métal alcalin, métal alcalino-terreux, d'ammonium et les sels d'ammonium quaternaires N⁺(alkyle en C₁₋₄)₄. La présente invention envisage également la quaternisation de tous les groupes contenant un azote basique des composés divulgués dans le présent mémoire. Des produits solubles ou dispersibles dans l'eau ou un milieu huileux peuvent être obtenus par une telle quaternisation. Des sels de métaux alcalins ou alcalino-terreux représentatifs comprennent le sodium, le lithium, le potassium, le calcium, le magnésium, et analogues. D'autres sels pharmaceutiquement acceptables non toxiques comprennent, le cas échéant, les sels d'ammonium, d'ammonium quaternaire et des cations d'amines formés en utilisant des contre-ions tels qu'un halogénure, un hydroxyde, un carboxylate, un sulfate, un phosphate, un nitrate, un sulfonate alkyle inférieur et arylsulfonate.
Comme décrit ci-dessus, les compositions pharmaceutiquement acceptables de la présente invention comprennent en outre un support pharmaceutiquement acceptable, un adjuvant ou un véhicule, qui, tel que défini dans la présente, inclut n'importe quel solvant, diluant ou autre véhicule liquide, dispersion ou auxiliaire de suspension, agent tensio-actif, agent isotonique, agent épaississant ou émulsifiant, conservateur, liant solide, lubrifiant et analogue, adaptés à la forme posologique particulière souhaitée. Remington Pharmaceutical Sciences, seizième édition, E. W. Martin (Mack Publishing Co., Easton, Pa., 1980) décrit différents supports utilisés dans la formulation de compositions pharmaceutiquement acceptables et des techniques connues pour leur préparation. Sauf dans le cas où un milieu support conventionnel s'avèrerait incompatible avec les composés selon l'invention, par exemple en produisant un quelconque effet biologique indésirable ou bien en interagissant de manière délétère avec tout autre composant (s) de la composition pharmaceutiquement acceptable, son utilisation est envisagée comme tombant dans le cadre de la présente invention. Quelques exemples de matériaux qui peuvent servir de supports pharmaceutiquement acceptables comprennent, mais ne sont pas limités à, des échangeurs d'ions, l'alumine, le stéarate d'aluminium, la lécithine, des protéines sériques telles que l'albumine sérique humaine, des substances tampons telles que les phosphates, la glycine, l'acide sorbique, ou sorbate de potassium, des mélanges de glycérides partiels d'acides gras végétaux saturés, l'eau, des sels ou des electrolytes tels que le sulfate de protamine, le phosphate disodique, l'hydrogénophosphate de potassium, le chlorure de sodium, des sels de zinc, la silice colloïdale, le trisilicate de magnésium, la polyvinylpyrrolidone, des polyacrylates, des cires, les polymères de polyéthylène-polyoxypropylène, des sucres tels que le lactose, le glucose et le saccharose ; des amidons tels que l'amidon de maïs et de pomme de terre ; la cellulose et ses dérivés tels que la carboxyméthylcellulose sodique, l'éthylcellulose et l'acétate de cellulose; la tragacanthe en poudre ; malt ; gélatine ; talc ; des excipients tels que le beurre de cacao et les cires pour suppositoires ; des huiles telles que l'huile d'arachide, l'huile de graine de coton ; l'huile de carthame ; huile de sésame ; huile d'olive ; huile de maïs et l'huile de soja ; glycols ; un tel propylène glycol ou le polyéthylène glycol ; des esters tels que l'oléate d'éthyle et le laurate d'éthyle ; la gélose ; des agents tels que l'hydroxyde de magnésium et l'hydroxyde d'aluminium en tampon ; l'acide alginique ; une solution saline isotonique ; la solution de Ringer ; l'alcool éthylique, et de tampon de phosphate de solutions, ainsi que d'autres lubrifiants compatibles non toxiques tels que le laurylsulfate de sodium et le stéarate de magnésium, ainsi que des agents colorants, des agents de démoulage, des agents d'enrobage, des agents édulcorants, aromatisants et parfumants, des conservateurs et des antioxydants peuvent également être présents dans la composition, selon le jugement du galéniste.

Selon un autre aspect, la présente invention se rapporte à l'un quelconque des composés tels que décrits précédemment, ou composition pharmaceutique le comprenant, pour son utilisation comme médicament, notamment dans le traitement de maladies ou conditions impliquant un récepteur N-Méthyl-D-Aspartate du système nerveux central, telles que l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure.

Selon un autre aspect, la présente invention se rapporte à une méthode de traitement ou prévention de maladies ou pathologies impliquant un récepteur N-Méthyl-D-Aspartate du système nerveux central, telles que l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure, comprenant l'administration d'une quantité efficace d'un composé ou d'une composition pharmaceutiquement acceptable selon la présente invention, à un sujet.

Dans le présent document, une "quantité efficace" d'un composé ou d'une composition pharmaceutiquement acceptable selon l'invention se réfère à une quantité efficace pour traiter ou diminuer la gravité d'une maladie ou affection associée aux récepteurs NMDA. Les composés et compositions, selon la méthode de traitement de la présente invention peuvent être administrés en utilisant toute quantité et toute voie d'administration efficace pour traiter ou diminuer la gravité d'une maladie ou affection associée aux récepteurs NMDA. La quantité exacte requise variera d'un sujet à un autre, en fonction de l'espèce, l'âge et l'état général du sujet, de la gravité de l'infection, du composé particulier et de son mode d'administration.
Les composés selon l'invention sont de préférence formulés sous une forme posologique unitaire pour faciliter l'administration et l'uniformité du dosage. Dans le présent document, l'expression «forme posologique unitaire» fait référence à une unité physiquement distincte de composé approprié pour le patient à traiter. Cependant, il sera entendu que la posologie quotidienne totale des composés et compositions selon la présente invention sera décidée par le médecin traitant. Le niveau de dose efficace spécifique pour un patient ou sujet animal ou humain particulier dépendra d'une variété de facteurs comprenant le trouble ou la maladie traité et la sévérité du trouble ou de la maladie ; l'activité du composé spécifique employé ; la composition spécifique employée ; l'âge, le poids corporel, la santé générale, le sexe et le régime alimentaire du patient/sujet ; la période d'administration, la voie d'administration et le taux d'élimination du composé spécifique employé ; la durée du traitement ; les médicaments utilisés en combinaison ou incidemment avec le composé spécifique utilisé et des facteurs analogues bien connus dans les arts médicaux. Le terme «patient», tel qu'il est utilisé ici, désigne un animal, de préférence un mammifère, et de manière préférée, un être humain.
Les compositions pharmaceutiquement acceptables de la présente invention peuvent être administrées à des humains et autres animaux par voie orale, rectale, parentérale, intracisternale, intravaginale, intrapéritoneale, topique (comme par des poudres, des pommades ou des gouttes), buccale, sous forme de pulvérisation orale ou nasale, ou similaire, selon la gravité de l'infection à traiter. Par exemple, les composés selon l'invention peuvent être administrés par voie orale ou parentérale à des doses d'environ 0,01 mg / kg à environ 50 mg / kg et de préférence d'environ 1 mg / kg à environ 25 mg / kg de poids corporel du sujet par jour, une ou plusieurs fois par jour, pour obtenir l'effet thérapeutique souhaité. Les formes galéniques liquides pour l'administration orale comprennent, mais sans s'y limiter, des émulsions pharmaceutiquement acceptables, des microémulsions, des solutions, des suspensions, des sirops et des élixirs. En plus des composés actifs, les formes galéniques liquides peuvent contenir des diluants inertes couramment utilisés dans l'art tels que, par exemple, l'eau ou d'autres solvants, des agents et des émulsifiants tels que l'alcool éthylique, l'alcool isopropylique, le carbonate d'éthyle, l'acétate d'éthyle, l'alcool benzylique, le benzoate de benzyle, le propylèneglycol, le 1,3-butylène glycol, le diméthylformamide, les huiles (en particulier, les graines de coton, d'arachide, de maïs, de germe, d'olive, et de sésame), le glycérol, l'alcool tétrahydrofurfurylique, des polyéthylèneglycols et des esters d'acides gras de sorbitan et des mélanges de ceux-ci. Outre les diluants inertes, les compositions orales peuvent également comprendre des adjuvants tels que des agents mouillants, des agents émulsifiants et de suspension, des édulcorants, des aromatisants et des agents parfumants.
Les formes galéniques solides pour une administration orale comprennent des capsules, des comprimés, des pilules, des poudres et des granulés. Dans de telles formes galéniques solides, le composé actif est mélangé avec au moins un support inerte, excipient ou support pharmaceutiquement acceptable tel que le citrate de sodium ou le phosphate dicalcique et / ou a) des charges ou des diluants tels que les amidons, le lactose, le saccharose, le glucose, le mannitol et l'acide silicique, b) des liants tels que, par exemple, la carboxyméthylcellulose, l'alginate, la gélatine, la polyvinylpyrrolidone, le saccharose et la gomme arabique, c) des humectants tels que le glycérol, d) des agents délitants tels que l'agar-agar, le carbonate de calcium, l'amidon de pomme de terre ou de manioc, l'acide alginique, certains silicates et le carbonate de sodium, e) des accélérateurs d'absorption tels que des composés d'ammonium quaternaire, f) des agents mouillants tels que, par exemple, l'alcool cétylique et le monostéarate de glycérol, g) des absorbants tels que le kaolin et l'argile bentonite et h) des lubrifiants tels que le talc, le stéarate de calcium, le stéarate de magnésium, des polyéthylèneglycols solides, le laurylsulfate de sodium et des mélanges de ceux-ci. Dans le cas des capsules, des comprimés et des pilules, la forme galéniques peut également comprendre des agents tampons.
Des compositions solides d'un type similaire peuvent également être employées comme charges dans des capsules de gélatine molle ou dure en utilisant des excipients tels que le lactose ou sucre de lait ainsi que des polyéthylèneglycols de haut poids moléculaire de polyéthylène et analogues. Les formes galéniques solides de comprimés, dragées, gélules, pilules et granules peuvent être préparées avec des enrobages et des enveloppes tels que des enrobages entériques et d'autres enrobages bien connus dans l'art pharmaceutique, la formulation. Elles peuvent éventuellement contenir des agents opacifiants et peuvent également être d'une composition telle qu'elles libèrent l'ingrédient actif (s) uniquement, ou préférentiellement, dans une certaine partie du tractus intestinal, facultativement de manière retardée. Des exemples de compositions d'enrobage qui peuvent être utilisées comprennent des substances polymères et des cires. Des compositions solides d'un type similaire peuvent également être employées comme charges dans des capsules de gélatine molle et dure en utilisant des excipients tels que le lactose ou sucre de lait ainsi que des polyéthylèneglycols de haut poids moléculaire de polyéthylène et analogues.
Les composés et les compositions pharmaceutiquement acceptables de la présente invention peuvent également être utilisés dans les thérapies combinées, à savoir les composés et les compositions pharmaceutiquement acceptables peuvent être administrés simultanément avec, avant ou postérieurement à un ou plusieurs autres agents thérapeutiques, ou procédures médicales. La combinaison particulière de thérapies (thérapies ou procédures) à employer dans un schéma d'association prendra en compte la compatibilité des produits thérapeutiques souhaités et / ou des procédures et l'effet thérapeutique souhaité à atteindre. Les thérapies utilisées peuvent viser la même maladie (par exemple, un composé selon l'invention peut être administré simultanément avec un autre agent utilisé pour traiter la même maladie), ou elles peuvent viser différents effets thérapeutiques (par exemple, le contrôle d'effets indésirables).
Par exemple, des agents thérapeutiques connus pour traiter des maladies, troubles et conditions, impliquant ces récepteurs du système nerveux central y compris, mais sans s'y limiter l'épilepsie grave/résistante, la schizophrénie, les maladies de Parkinson et d'Alzheimer, la démence ou la dépression majeure, peuvent être combinés avec les composés de la présente invention pour traiter ces mêmes maladies. Des exemples de thérapies ou agents thérapeutiques qui peuvent être utilisés en combinaison avec les composés de la présente invention comprennent en particulier l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure.

### TROUSSES (« KITS OF PARTS »)

La présente invention concerne également un kit pour la pratique et la mise en oeuvre des méthodes de traitement conformes à la présente invention. D'une manière générale, le pack ou kit pharmaceutique comprend un ou plusieurs récipients rempli(s) d'un ou plusieurs des ingrédients des compositions pharmaceutiques selon l'invention. De tels kits sont particulièrement adaptés pour l'administration de formes orales solides telles que des comprimés ou des capsules. Un tel kit comprend de préférence un certain nombre de doses unitaires, et peut également inclure une plaquette sur laquelle les unités de dosage sont disposées dans l'ordre préconisé de leur utilisation. Si cela est souhaité, un aide-mémoire peut être fourni, par exemple sous la forme de chiffres, de lettres ou d'autres marquages ou avec un insert de calendrier, désignant les jours dans le programme de traitement, dans lequel les doses peuvent être administrées. Alternativement, les doses de placebo ou des suppléments de calcium alimentaire, que ce soit sous une forme similaire ou distincte des dosages des compositions pharmaceutiques, peuvent être inclus pour fournir un kit dans lequel une dose est prise tous les jours. Le kit peut également comprendre une notice explicative, comprenant notamment des informations relatives à la forme prescrite par une agence gouvernementale réglementant la fabrication, l'utilisation ou la vente de produits pharmaceutiques.

### EQUIVALENTS

Les exemples représentatifs qui suivent sont destinés à illustrer l'invention et ne sont pas destinés à limiter la portée de l'invention, ni ne doivent être interprétés comme tels. En effet, diverses modifications de l'invention et de nombreux autres modes de réalisation de celle-ci, en plus de celles présentées et décrites ici, apparaîtront à l'homme du métier à partir de l'ensemble du contenu de ce document, y compris les exemples qui suivent. Les exemples qui suivent contiennent des informations importantes supplémentaires, d'exemplification et d'enseignement qui peuvent être adaptés à la pratique de cette invention dans ses divers modes de réalisation et les équivalents de ceux-ci.
Les exemples suivants sont donnés à titre indicatif et sans aucun caractère limitatif de l'invention.
Des avantages autres que ceux décrits dans la présente demande pourront encore apparaître à l'homme du métier à la lecture des exemples ci-dessous donnés à titre illustratif.

### EXEMPLES

### Exemple 1: procédure générale A pour la préparation de 3-(Hydroxyalkyl)-(1H)-indole-3-yl

Dans un tube de type micro-ondes de taille appropriée, sous atmosphère d'argon, une solution d'acide acétique à 50% dans l'eau (18 mL) a été ajoutée au chlorhydrate de phénylhydrazine souhaité (1 équiv., 8,4 mmol) à température ambiante, c'est-à-dire à 20°C. Le dihydropyrane ou le dihydrofurane (1 équiv., 8,4 mmol) a été ajouté goutte à goutte sur 2 minutes à la solution précédente. Le flacon a été scellé et chauffé à 50 °C pendant 10 min puis à 100 °C pendant 55 min. Le mélange réactionnel a été désactivé par l'addition d'une solution saturée d'hydrogénocarbonate de sodium (10 mL). La phase aqueuse a été extraite trois fois avec de l'acétate d'éthyle (3 x 20 mL) et les phases organiques ont été combinées puis lavées avec de la saumure (10mL), séchées sur sulfate de magnésium anhydre, filtrées sur verre fritté et concentrées sous pression réduite (50 mbar) à l'évaporateur rotationnel. Le brut obtenu a été ensuite dilué dans de l'éthanol (20 mL) et 10 pastilles d'hydroxyde de potassium ont été ajoutées à température ambiante c'est-à-dire à 20°C et agité pendant 2 h. Le mélange réactionnel a été désactivé par l'addition d'eau (10 mL). La phase aqueuse a été extraite trois fois avec de l'acétate d'éthyle (3 x 20 mL) et les phases organiques ont été combinées puis lavées avec de l'eau (3 x 10 mL) jusqu'à pH neutre, de la saumure (10 mL), séchées sur sulfate de magnésium, filtrées sur verre fritté et concentrées sous pression réduite (50 mbar) à l'évaporateur rotationnel. La purification a été réalisée par chromatographie éclair sur colonne [25 g, débit 18 mL/min, cyclohexane/EtOAc 100/0 à 50/50] pour donner le 3-(Hydroxyalkyl)-(1*H*)-indole-3-yl souhaité.

### Exemple 2: procédure générale B pour la préparation de 3-(Bromoalkyl)-(1H)-indole-3-vl

Dans un ballon de taille appropriée, du tétrabromure de carbone (1,5 équiv., 12,6 mmol) à été ajouté à une solution le 3-(2-Hydroxyéthyl)-(1*H*)-indole-3-yl (1 équiv, 8,4 mmol) dans du dichlorométhane anhydre (150 ml) sous atmosphère d'argon à 0 °C. Puis la triphénylphosphine (1,5 équiv., 12,6 mmol) a été ajoutée en 5 portions égales sur une période de 10 min à 0 °C. Le mélange réactionnel a été agité à température ambiante c'est-à-dire à 20°C pendant 1h puis évaporer sous pression réduite sous pression réduite (50 mbar) à l'évaporateur rotationnel. La purification a été réalisée par chromatographie éclair sur colonne telle que décrite dans l'exemple 1 pour donner le 3-(Bromoalkyl)-(1*H*)-indole-3-yl souhaité.

### Exemple 3: procédure générale C pour la préparation 1-(3-alkyl-(1H)-indol-3-yl)-indol-2,3-dione

Dans un tube de type micro-ondes de taille appropriée, l'isatin (1 équiv., 3,40 mmol), le 3-(2-Bromoéthyl)-(1*H*)-indol-3-yl (1,02 équiv., 3,47 mmol) et le carbonate de potassium (1,4 équiv., 4,76 mmol) ont été mélangés dans du diméthylformamide anhydre (15 ml) sous atmosphère d'argon. Le flacon a été scellé et irradié sous irradiation micro-onde à 130°C pendant 15 min. Le mélange réactionnel a été désactivé par addition d'acétate d'éthyle. La phase organique a été lavée successivement avec une solution saturée de chlorure d'ammonium puis avec de la saumure, séchées sur sulfate de magnésium, filtrées et concentrées sous pression réduite tel que décrit dans l'exemple 1. La purification a été réalisée par chromatographie éclair sur colonne telle que décrite dans l'exemple 1 pour donner le 1-(3-alkyl-(1*H*)-indol-3-yl)-indol-2,3-dione souhaitée.

### Exemple 4: procédure générale D pour la préparation de 1-(3-alkyl-(1H)-indol-3-yl)-3-hydroxy-3-(2-oxo-2-phényléthyl)-indol-2-one

Dans un tube de type micro-ondes de taille appropriée, le 1-(3-éthyl-(1*H*)-indol-3-yl)-indol-2,3-dione (1 équiv., 0,28 mmol), l'acétophénone (1,4 équiv., 0,39 mmol) et la piperidine (500 µL) ont été mélangés dans de l'éthanol absolu (3 ml) sous atmosphère d'argon. Le flacon a été scellé et agité à température ambiante c'est-à-dire à 20°C pendant 18 heures. Le mélange réactionnel a été désactivé par addition d'acétate d'éthyle. La phase organique a été lavée successivement avec une solution saturée de chlorure d'ammonium puis de la saumure, séchées sur sulfate de magnésium, filtrées et concentrées sous pression réduite tel que décrit dans l'exemple 1. La purification a été réalisée par chromatographie éclair sur colonne telle que décrite dans l'exemple 1 pour donner 1-(3-alkyl-(1*H*)-indol-3-yl)-3-hydroxy-3-(2-oxo-2-phényléthyl)-indol-2-one souhaitée.

### Exemple 5: procédure générale E pour la préparation 1-(3-alkyl-1-méthyl-(1H)-indol-3-yl)-indol-2,3-dione

Dans un ballon de taille appropriée, le iodure de méthyle (1,5 équiv., 0,52 mmol) et le carbonate de césium (2 équiv., 0,69 mmol) ont été ajouté à une solution de 1-(3-éthyl-(1*H*)-indol-3-yl)-indol-2,3-dione (1 équiv., 0,34 mmol) dans du diméthylformamide anhydre (5 ml) sous atmosphère d'argon à température ambiante. Le mélange réactionnel est agité à température ambiante ambiante c'est-à-dire à 20°C pendant 3h puis désactivé par addition d'acétate d'éthyle. La phase organique a été lavée successivement avec une solution saturée de chlorure d'ammonium puis de la saumure, séchées sur sulfate de magnésium, filtrées et concentrées sous pression réduite tel que décrit dans l'exemple 1. La purification a été réalisée par chromatographie éclair sur colonne telle que décrite dans l'exemple 1 pour donner le 1-(3-alkyl-1-méthyl-(1*H*)-indol-3-yl)-indol-2,3-dione souhaitée.

### Exemple 6: procédure générale F pour la préparation de (E)-1-(2-(1H-indol-3-yl)ethyl)-3-(2-(2-chlorophenyl)-2-oxoethylidene)indolin-2-one APV-ST505

Dans un ballon de taille appropriée, la triéthylamine (2 équiv., 0,58 mmol) a été ajoutée à une solution de 1-(3-Ethyl-(1*H*)-indol-3-yl)-3-hydroxy-3-(2-oxo-2-(2-chlorophényl)-éthyl)-indol-2-one (1 équiv., 0,29 mmol) dans le dichlorométhane (5 mL) à température ambiante. Le mélange réactionnel est agité à température ambiante ambiante c'est-à-dire à 20°C pendant 12h. La phase organique a été lavée successivement avec une solution saturée de chlorure d'ammonium (2 x 2mL) puis de la saumure (2mL), séchée sur sulfate de magnésium, filtrée sur verre fritté et concentrées sous pression réduite tel que décrit dans l'exemple 1 pour donner le (*E*)-1-(2-(1H-indol-3-yl)ethyl)-3-(2-(2-chlorophenyl)-2-oxoethylidene)indolin-2-one souhaitée.

Le composé APV-ST505 a été préparé suivant le mode opératoire général F en utilisant du 1-(3-Ethyl-(1*H*)-indol-3-yl)-3-hydroxy-3-(2-oxo-2-(2-chlorophényl)-éthyl)-indol-2-one (130 mg, 0,29 mmol) et de la triéthylamine (40 µL, 0,56 mmol). Le composé indiqué dans le titre a été obtenu sous forme d'un solide rouge (130 mg). Rdt = quantitatif.
RMN ¹H (300 MHz, CDCl₃, δ ppm) 8,58 (d, *J* = 7,7 Hz, 1H), 8,02 (s, 1H), 7,68 (d, J = 8,3 Hz, 2H), 7,64 (s, 1H), 7,51-7,45 (m, 2H), 7-45-7,29 (m, 3H) 7,22 (t, *J* = 7,2 Hz, 1H), 7,15 (t, *J* = 7,2 Hz, 1H), 7,10-7,00 (m, 2H), 6,75 (d, *J* = 7,8 Hz, 1H), 4,06 (t, *J* = 7,6 Hz, 2H), 3,17 (t, *J* = 7,6 Hz, 2H).
RMN ¹³C (75 MHz, DMSO-d₆, δ ppm) 191,9, 166,8, 145,6, 138,0, 136,2, 135,5, 133,5, 133,4, 132,4, 130,6, 130,3, 129,3, 127,8, 127,4, 127,3, 127,0, 123,1, 122,3, 121,0, 119,3, 118,3, 118,0, 111,4, 110,5, 109,4, 22,9.
HRMS (ESI, m/z) calculé pour C₂₆H₁₉N₂O₂NaCl [M+Na]⁺ : 449,1033 déterminé 449,1033.

### Exemple 7: procédure générale G pour la préparation 1-(2-(1H-indol-3-yl)ethyl)-3-(2-(2-chlorophenyl)-2-oxoethyl)indolin-2-one

Dans un ballon de taille appropriée, du palladium sur charbon actif (8 mg, 10% poids/poids) a été ajouté à une solution de (*E*)-1-(2-(1H-indol-3-yl)ethyl)-3-(2-(2-chlorophenyl)-2-oxoethylidene)indolin-2-one (80 mg, 0,19 mmol) dans du méthanol (13 ml) sous atmosphère d'argon à température ambiante c'est-à-dire à 20°C. Le mélange est ensuite placé sous atmosphère d'hydrogène et agité à température ambiante c'est-à-dire à 20°C pendant 1h. Le brut a été filtré sur un lit de célite et ce dernier a été lavé par l'acétate d'éthyle. Le filtrat a été concentré sous pression réduite pour donner le 1-(2-(1H-indol-3-yl)ethyl)-3-(2-(2-chlorophenyl)-2-oxoethyl)indolin-2-one souhaité.

### 1-(2-(1H-indol-3-yl)ethyl)-3-(2-(2-chlorophenyl)-2-oxoethyl)indolin-2-one (APV-GG065)

Le composé APV-GG065 a été préparé suivant le mode opératoire général G en utilisant du (*E*)-1-(2-(1H-indol-3-yl)ethyl)-3-(2-(2-chlorophenyl)-2-oxoethylidene)indolin-2-one (80 mg, 0,19 mmol) (80 mg, 0,19 mmol) et du Paladium sur Charbon le Pd/C (8 mg). Le composé indiqué dans le titre a été obtenu sous forme d'un solide beige (60 mg). Rdt = 74 %.
RMN ¹H (300 MHz, CDCl₃, δ ppm) 8,00 (s, 1H), 7,59 (d, *J* = 7,8 Hz, 1H), 7,50 (d, *J* = 7,7 Hz, 1H), 7,45-7,38 (m, 2H) 7,38-7,27 (m, 3H), 7,25-7,07 (m, 4H), 7,01 (t, *J* = 7,5 HZ, 1H), 6,84 (d, *J* = 7,8 Hz, 1H), 4,10-3,98 (m, 3H), 3,65 (dd, *J* = 18,4, 3,7 Hz, 1H), 3,23 (dd, *J* = 18,4, 8,5 Hz, 1H), 3,17 (t, *J* = 7,6 Hz, 2H). RMN ¹³C (75 MHz, CDCl₃, δ ppm) 200,2, 177,3, 143,9, 138,6, 136,4, 132,3, 131,3, 130,9, 129,5, 128,9, 128,3, 127,2, 124,7, 122,5, 122,4, 122,3, 119,7, 118,7, 112,7, 111,4, 108,5, 102,4, 44,1, 41,6, 41,1, 23,4. HRMS (ESI, m/z) calculé pour C₂₆H₂₁N₂O₂NaCl [M+Na]⁺ : 451,1189 déterminé 451,1194.

### Exemple 8: synthèse de composés selon l'invention (composé issu de la synthèse D)

### 1-(3-Ethyl-(1H)-indol-3-yl)-3-hydroxy-3-(2-oxo-2-phényléthyl)-indol-2-one (APV-ST288)

Le composé APV-ST288 a été préparé suivant le mode opératoire général D en utilisant la 1-(3-Ethyl-(1*H*)-indol-3-yl)-indol-2,3-dione (80 mg, 0,28 mmol) et l'Acétophenone (45 µL, 0,39 mmol). Le composé indiqué dans le titre a été obtenu après chromatographie éclair suivie d'une trituration dans le pentane sous forme d'un solide beige (91 mg). Rdt = 80 %. Tf = 159,7 - 161,5 °C. RMN ¹H (300 MHz, DMSO-d₆, δ ppm) 10,89 (s, 1H), 7,90 (d, *J* = 7,6 Hz, 2H), 7,66 (d, *J* = 7,8 Hz, 1H), 7,61 (d, *J* = 7,1 Hz, 1H), 7,51 (d, *J* = 7,5 Hz, 1H), 7,48 (d, *J* = 7,6 Hz, 1H), 7,43 - 7,35 (m, 2H), 7,33 (s, 1H), 7,26 (dd, *J* = 7,6, 7,6 Hz, 1H), 7,16 - 6,91 (m, 4H), 6,20 (s, 1H), 4,15 (d, *J* = 17,5 Hz, 1H), 4,06 - 3,85 (m, 2H), 3,68 (d, *J* = 17,4 Hz, 1H), 3,08 (t, *J* = 7,8 Hz, 2H). RMN ¹³C (75 MHz, DMSO-d₆, δ ppm) 196,9, 177,0, 144,1, 136,7, 136,6, 133,9, 131,6, 129,6, 129,2, 129,2, 128,4, 128,4, 127,6, 123,9, 123,6, 122,2, 121,5, 118,9, 118,6, 111,9, 111,3, 108,8, 73,2, 46,6, 40,0, 23,2. IR-TF (neat, cm⁻¹)3359,2954, 1702, 1678, 1615, 1344, 1221, 1170, 1061, 982, 741, 689. HRMS (ESI, m/z) calculé pour C₂₆H₂₃N₂O₃ [M+H]⁺ : 411,1630 déterminé 411,1705 et pour C₂₆H₂₂N₂O₃Na [M+Na]⁺ : 433,1528 déterminé 433,1526. *Conditions de chromatographie éclair:* colonne 25 g, débit 18 mL/min, cyclohexane/EtOAc 100/0 à 50/50. R*_{f}* = 0,35 cyclohexane/EtOAc 50/50.

### 1-(3-Ethyl-(1H)-indol-3-yl)-3-hydroxy-3-(2-oxo-2-(2-chlorophényl)-éthyl)-indol-2-one (APV-ST357)

Le composé APV-ST357 a été préparé suivant le mode opératoire général D en utilisant la 1-(3-Ethyl-(1*H*)-indol-3-yl)-indol-2,3-dione (80 mg, 0,28 mmol) et la 2'-Chloroacétophenone (71 µL, 0,39 mmol). Le composé indiqué dans le titre a été obtenu après chromatographie éclair suivie d'une trituration dans le pentane sous forme d'un solide beige (100 mg). Rdt = 81 %. Tf= 100,5- 101,9 °C. RMN ¹H (300 MHz, DMSO-d₆, δ ppm) 10,88 (s, 1H), 7,64 (d, *J* = 7,4 Hz, 1H), 7,54 - 7,44 (m, 3H), 7,44 - 7,34 (m, 3H), 7,34 - 7,24 (m, 2H), 7,18 - 6,93 (m, 4H), 6,26 (s, 1H), 4,03 - 3,82 (m, 3H), 3,63 (d, *J* = 16,7 Hz, 1H), 3,05 (t, *J* = 7,6 Hz, 2H). RMN ¹³C (75 MHz, DMSO-d₆, δ ppm) 199,0, 176,6, 143,8, 138,4, 136,7, 132,9, 130,9, 130,9, 130,1, 129,8, 129,7, 127,8, 127,5, 124,3, 123,6, 122,3, 121,5, 118,9, 118,5, 111,9, 111,3, 108,9, 73,2, 50,3, 40,6, 23,2. IR-TF (neat, cm⁻¹) 3381, 2920, 1693, 1615, 1469, 1353, 1336, 1163, 1061, 741. HRMS (ESI, m/z) calculé pour C₂₆H₂₂N₂O₃Cl [M+H]⁺ : 445,1319 déterminé 445,1326 et pour C₂₆H₂₁N₂O₃ClNa [M+Na]⁺ : 467,1138 déterminé 467,1133. *Conditions de chromatographie éclair*: colonne 25 g, débit 18 mL/min, cyclohexane/EtOAc 100/0 à 50/50. R*_{f}* = 0,50 cyclohexane/EtOAc 50/50.

### 1-(3-Ethyl-(1H)-indol-3-yl)-3-hydroxy-3-(2-oxo-2-(3-chlorophényl)-éthyl)-indol-2-one (APV-ST292)

Le composé APV-ST292 a été préparé suivant le mode opératoire général D en utilisant la 1-(3-Ethyl-(1*H*)-indol-3-yl)-indol-2,3-dione (80 mg, 0,28 mmol) et la 3'-Chloroacétophenone (51 µL, 0,39 mmol). Le composé indiqué dans le titre a été obtenu après chromatographie éclair suivie d'une trituration dans le pentane sous forme d'un solide beige (88 mg). Rdt = 72 %. Tf = 130,2 - 133,4 °C, RMN ¹H (300 MHz, CDCl₃, δ ppm) 8,23 (s, 1H), 7,81 (s, 1H), 7,69 (d, *J* = 7,7 Hz, 1H), 7,57 - 7,47 (m, 2H), 7,41 - 7,33 (m, 3H), 7,29 (d, *J* = 8,2 Hz, 1H), 7,18 (dd, *J* = 7,6, 7,6 Hz, 1H), 7,12 - 7,01 (m, 3H), 6,88 (d, *J* = 7,9 Hz, 1H), 4,20 - 3,95 (m, 3H), 3,64 (d, *J* = 17,4 Hz, 1H), 3,32 (d, *J* = 17,5 Hz, 1H), 3,22 (t, *J* = 7,2 Hz, 2H). RMN ¹³C (75 MHz, CDCl₃, δ ppm) 196,7, 176,1, 143,2,137,8, 136,2, 135,0, 133,6,130,0, 130,0, 129,9, 128,2, 127,5, 126,3, 124,1, 122,9, 122,7, 122,0, 119,3, 118,4, 112,2, 111,4, 108,9, 74,2, 44,6, 41,0, 22,9. IR-TF (neat, cm⁻¹) 3332, 1702, 1683, 1616, 1413, 1337, 1215, 1170, 1066, 748. HRMS (ESI, m/z) calculé pour C₂₆H₂₂N₂O₃Cl [M+H]⁺ : 445,1319 déterminé 445,1306 et pour C₂₆H₂₁N₂O₃ClNa [M+Na]⁺ : 467,1138 déterminé 467,1139. *Conditions de chromatographie éclair:* colonne 25 g, débit 18 mL/min, cyclohexane/EtOAc 100/0 à 50/50. R*_{f}* = 0,46 cyclohexane/EtOAc 50/50.

### 1-(3-Ethyl-(1H)-indol-3-yl)-3-hydroxy-3-(2-oxo-2-(4-chlorophényl)-éthyl)-indol-2-one (APV-ST358)

Le composé APV-ST358 a été préparé suivant le mode opératoire général D en utilisant la 1-(3-Ethyl-(1*H*)-indol-3-yl)-indol-2,3-dione (80 mg, 0,28 mmol) et la 4'-Chloroacétophenone (51 µL, 0,39 mmol). Le composé indiqué dans le titre a été obtenu après chromatographie éclair suivie d'une trituration dans le pentane sous forme d'un solide beige (91 mg). Rdt = 74 %. Tf = 176,9 - 178,1 °C. RMN ¹H (300 MHz, DMSO-d₆, δ ppm) 10,89 (s, 1H), 7,91 (d, *J* = 8,4 Hz, 2H), 7,66 (d, *J* = 7,6 Hz, 1H), 7,55 (d, *J* = 8,3 Hz, 2H), 7,43 - 7,35 (m, 2H), 7,33 (d, *J* = 1,5 Hz, 1H), 7,27 (dd, *J* = 7,7, 7,7 Hz, 1H), 7,17 - 6,91 (m, 4H), 6,23 (s, 1H), 4,13 (d, *J* = 17,4 Hz, 1H), 4,07 - 3,84 (m, 2H), 3,67 (d, *J* = 17,4 Hz, 1H), 3,08 (t, *J* = 7,8 Hz, 2H). RMN ¹³C (75 MHz, DMSO-d₆, δ ppm) 196,0, 176,9, 144,0, 138,9, 136,8, 135,3, 131,5, 130,4, 130,4, 129,6, 129,2, 129,2, 127,6, 124,0, 123,6, 122,2, 121,5, 118,9, 118,6, 112,0, 111,3, 108,8, 73,2, 46,6, 40,6, 23,2. IR-TF (neat, cm⁻¹) 3334, 1703, 1658, 1613, 1589, 1381, 1355, 1338, 1219, 1169, 1006, 837, 739. HRMS (ESI, m/z) calculé pour C₂₆H₂₂N₂O₃Cl [M+H]⁺ : 445,1319 déterminé 445,1330 et pour C₂₆H₂₁N₂O₃ClNa [M+Na]⁺ : 467,1138 déterminé 467,1134. *Conditions de chromatographie éclair:* colonne 25 g, débit 18 mL/min, cyclohexane/EtOAc 100/0 à 50/50. R*_{f}* = 0,50 cyclohexane/EtOAc 50/50.

### 1-(3-Ethyl-(1H)-indol-3-yl)-3-hydroxy-3-(2-oxo-2-(pyridin-2-yl)-éthyl)-indol-2-one (APV-ST430)

Le composé APV-ST430 a été préparé suivant le mode opératoire général D en utilisant la 1-(3-Ethyl-(1*H*)-indol-3-yl)-indol-2,3-dione (80 mg, 0,28 mmol) et la 2-Acétylpyridine (42 µL, 0,39 mmol). Le composé indiqué dans le titre a été obtenu après chromatographie éclair suivie d'une trituration dans le pentane sous forme d'un solide beige (95 mg). Rdt = 84 %. Tf = 156,8 - 158,5 °C. RMN ¹H (300 MHz, DMSO-d₆, δ ppm) 10,88 (s, 1H), 8,75 (d, *J* = 4,1 Hz, 1H), 7,98 - 7,89 (m, 1H), 7,77 (d, *J* = 7,8 Hz, 1H), 7,69 - 7,62 (m, 2H), 7,37 (d, *J* = 7,8 Hz, 1H), 7,34 - 7,29 (m, 2H), 7,29 - 7,21 (m, 1H), 7,14 - 6,98 (m, 3H), 6,96 - 6,89 (m, 1H), 6,25 (s, 1H), 4,37 (d, *J* = 17,9 Hz, 1H), 4,04 - 3,86 (m, 2H), 3,74 (d, *J* = 17,8 Hz, 1H), 3,07 (t, *J* = 8,0 Hz, 2H). RMN ¹³C (75 MHz, DMSO-d₆, δ ppm) 198,2, 176,9, 152,6, 149,7, 144,0, 138,1, 136,7, 131,5, 129,6, 128,4, 127,5, 123,9, 123,6, 122,2, 121,6, 121,5, 118,9, 118,6, 111,9, 111,3, 108,8, 73,2, 45,7, 40,5, 23,3. IR-TF (neat, cm⁻¹) 3350, 3250, 1680, 1613, 1461, 1353, 1170, 1065, 994, 772, 739. HRMS (ESI, m/z) calculé pour C₂₅H₂₁N₃O₃Na [M+Na]⁺ : 434,1481 déterminé 434,1473. *Conditions de chromatographie éclair* : colonne 10 g, débit 8 mL/min, cyclohexane/EtOAc 100/0 à 40/60. R*_{f}* = 0,16 cyclohexane/EtOAc 50/50.

### 1-(3-Ethyl-(1H)-indol-3-yl)-3-hydroxy-3-(2-oxo-2-(pyridin-3-yl)-éthyl)-indol-2-one (APV-ST296)

Le composé APV-ST296 a été préparé suivant le mode opératoire général D en utilisant la 1-(3-Ethyl-(1*H*)-indol-3-yl)-indol-2,3-dione (80 mg, 0,28 mmol) et la 3-Acétylpyridine (43 µL, 0,39 mmol). Le composé indiqué dans le titre a été obtenu après chromatographie éclair suivie d'une trituration dans le pentane sous forme d'un solide beige (76 mg). Rdt = 68 %. Tf = 124,2 - 126,1 °C. RMN ¹H (300 MHz, CDCl₃, δ ppm) 8,92 (s, 1H), 8,64 (d, *J* = 4,5 Hz, 1H), 8,51 (s, 1H), 8,05 (d, *J* = 8,0 Hz, 1H), 7,48 (d, *J* = 7,8 Hz, 1H), 7,40 - 7,26 (m, 4H), 7,18 - 7,11 (m, 1H), 7,09 - 6,99 (m, 3H), 6,87 (d, *J* = 7,8 Hz, 1H), 4,84 (br. s, 1H), 4,15 - 3,94 (m, 2H), 3,68 (d, *J* = 17,2 Hz, 1H), 3,38 (d, *J* = 17,2 Hz, 1H), 3,18 (t, *J* = 7,1 Hz, 2H). RMN ¹³C (75 MHz, CDCl₃, δ ppm) 196,5, 176,4, 153,5, 149,4, 143,2, 136,2, 135,6, 131,8, 130,0, 129,9, 127,5, 124,0, 123,6, 122,9, 122,7, 121,9, 119,2, 118,3, 112,0, 111,5, 109,0, 74,0, 45,0, 40,9, 22,8. IR-TF (neat, cm⁻¹) 3321, 2942, 1716, 1690, 1613, 1468, 1355, 1229, 116, 740, 701. HRMS (ESI, m/z) calculé pour C₂₅H₂₂N₃O₃ [M+H]⁺ : 412,1661 déterminé 412,1667 et pour C₂₅H₂₁N₃O₃Na [M+Na]⁺ : 434,1481 déterminé 434,1486. *Conditions de chromatographie éclair:* colonne 25 g, débit 18 mL/min, cyclohexane/EtOAc 100/0 à 50/50. R*_{f}* = 0,05 cyclohexane/EtOAc 50/50.

### 1-(3-Ethyl-(1H)-indol-3-yl)-3-hydroxy-3-(2-oxo-2-(pyridin-4-yl)-éthyl)-indol-2-one (APV-ST431)

Le composé APV-ST431 a été préparé suivant le mode opératoire général D en utilisant la 1-(3-Ethyl-(1*H*)-indol-3-yl)-indol-2,3-dione (80 mg, 0,28 mmol) et la 4-Acétylpyridine (42 µL, 0,39 mmol). Le composé indiqué dans le titre a été obtenu après chromatographie éclair suivie d'une trituration dans le pentane sous forme d'un solide beige (104 mg). Rdt = 92 %. Tf = 126,5- 127,8 °C. RMN ¹H (300 MHz, DMSO-d₆, δ ppm) 10,88 (s, 1H), 8,77 (d, *J* = 5,0 Hz, 1H), 7,75 (d, *J* = 5,1 Hz, 2H), 7,64 (d, *J* = 7,6 Hz, 1H), 7,38 (d, *J* = 7,7 Hz, 2H), 7,32 - 7,22 (m, 2H), 7,15 - 6,92 (m, 5H), 6,26 (s, 1H), 4,14 (d, *J* = 17,4 Hz, 1H), 4,03 - 3,83 (m, 2H), 3,67 (d, *J* = 17,5 Hz, 1H), 3,06 (t, *J* = 7,7 Hz, 2H). RMN ¹³C (75 MHz, DMSO-d₆, δ ppm) 197,3, 176,7, 151,2, 151,2, 143,9, 142,4, 136,7, 131,2, 129,7, 129,7, 127,5, 124,1, 123,6, 122,3, 121,5, 121,5, 118,9, 118,6, 111,9, 111,2, 108,9, 73,1, 46,8, 40,5, 23,2. IR-TF (neat, cm⁻¹) 3340, 3098, 1697, 16313, 1468, 1355, 1225, 1166, 1063, 741. HRMS (ESI, m/z) calculé pour C₂₅H₂₂N₃O₃ [M+H]⁺ : 412,1661 déterminé 412,1667 et pour C₂₅H₂₁N₃O₃Na [M+Na]⁺ : 434,1481 déterminé 434,1483. *Conditions de chromatographie éclair* : colonne 10 g, débit 8 mL/min, cyclohexane/EtOAc 100/0 à 40/60. R*_{f}* = 0,04 cyclohexane/EtOAc 50/50.

### 1-(3-Ethyl-(1H)-indol-3-yl)-3-hydroxy-3-(2-oxo-2-(2-hydroxyphényl)-éthyl)-indol-2-one (APV-ST415)

Le composé APV-ST415 a été préparé suivant le mode opératoire général D en utilisant la 1-(3-Ethyl-(1*H*)-indol-3-yl)-indol-2,3-dione (80 mg, 0,28 mmol) et la 2'-Hydroxyacétophenone (47 µL, 0,39 mmol). Le composé indiqué dans le titre a été obtenu après chromatographie éclair suivie d'une trituration dans le pentane sous forme d'un solide beige (116 mg). Rdt = 99 %. Tf = 114,8 - 116,7 °C. RMN ¹H (300 MHz, DMSO-d₆, δ ppm) 11,32 (s, 1H), 10,88 (s, 1H), 7,78 (d, *J* = 8,2 Hz, 1H), 7,64 (d, *J* = 7,6 Hz, 1H), 7,53 - 7,43 (m, 1H), 7,41 - 7,33 (m, 2H), 7,30 (s, 1H), 7,26 (d, *J*= 7,7 Hz, 1H), 7,15 - 6,88 (m, 6H), 6,20 (s, 1H), 4,15 (d, *J* = 17,6 Hz, 1H), 4,04-3,84 (m, 2H), 3,72 (d, *J* = 17,7 Hz, 1H), 3,05 (t, *J* = 7,8 Hz, 2H). RMN ¹³C (75 MHz, DMSO-d₆, δ ppm) 201,6, 176,9, 160,5, 144,0, 136,7, 136,4, 131,5, 131,1, 129,6, 127,5, 124,0, 123,6, 122,2, 121,5, 121,6, 119,7, 118,9, 118,6, 118,0, 111,9, 111,3, 108,8, 73,2, 48,2, 40,5, 23,1. IR-TF (neat, cm⁻¹) 3336, 2911, 1701, 1640, 1613, 1370, 1276, 1157, 740. HRMS (ESI, m/z) calculé pour C₂₆H₂₃N₂O₄ [M+H]⁺ : 427,1658 déterminé 427,1649 et pour C₂₆H₂₂N₂O₄Na [M+Na]⁺ : 449,1477 déterminé 449,1479. *Conditions de chromatographie éclair:* colonne 25 g, débit 18 mL/min, cyclohexane/EtOAc 100/0 à 50/50. R*_{f}* = 0,49 cyclohexane/EtOAc 50/50.

### 1-(3-Ethyl-(1H)-indol-3-yl)-3-hydroxy-3-(2-oxo-2-(3-hydroxyphényl)-éthyl)-indol-2-one (APV-ST360)

Le composé APV-ST360 a été préparé suivant le mode opératoire général D en utilisant la 1-(3-Ethyl-(1*H*)-indol-3-yl)-indol-2,3-dione (100 mg, 0,34 mmol) et la 3'-Hydroxyacétophenone (65 mg, 0,48 mmol). Le composé indiqué dans le titre a été obtenu après chromatographie éclair suivie d'une trituration dans le pentane sous forme d'un solide beige (119 mg). Rdt = 82 %. Tf = 145,3 - 147,2 °C. RMN ¹H (300 MHz, DMSO-d₆, δ ppm) 10,88 (s, 1H), 9,76 (s, 1H), 7,65 (d, *J* = 7,6 Hz, 1H), 7,42-7,18 (m, 7H), 7,15 - 6,91 (m, 5H), 6,16 (s, 1H), 4,07 (d, *J =* 17,5 Hz, 1H), 4,03 - 3,83 (m, 2H), 3,61 (d, *J* = 17,5 Hz, 1H), 3,06 (t, *J* = 7,8 Hz, 2H). RMN ¹³C (75 MHz, DMSO-d₆, δ ppm) 196,7, 177,0, 158,0, 144,1, 137,9, 136,7, 131,6, 130,3, 129,6, 127,6, 123,9, 123,6, 122,1, 121,5, 121,0, 119,4, 118,9, 118,6, 114,4, 111,9, 111,3, 108,8, 73,1, 46,6, 40,5, 23,2. IR-TF (neat, cm⁻¹) 3357, 2910, 1680, 1614, 1469, 1452, 1339, 1166, 739. HRMS (ESI, m/z) calculé pour C₂₆H₂₃N₂O₄ [M+H]⁺ : 427,1658 déterminé 427,1645 et pour C₂₆H₂₂N₂O₄Na [M+Na]⁺ : 449,1477 déterminé 449,1486. *Conditions de chromatographie éclair:* colonne 25 g, débit 18 mL/min, cyclohexane/EtOAc 100/0 à 50/50. R*_{f}* = 0,15 cyclohexane/EtOAc 50/50.

### 1-(3-Ethyl-(1H)-indol-3-yl)-3-hydroxy-3-(2-oxo-2-(4-hydroxyphényl)-éthyl)-indol-2-one (APV-ST428)

Le composé APV-ST428 a été préparé suivant le mode opératoire général D en utilisant la 1-(3-Ethyl-(1*H*)-indol-3-yl)-indol-2,3-dione (80 mg, 0,28 mmol) et la 4'-Hydroxyacétophenone (53 mg, 0,39 mmol). Le composé indiqué dans le titre a été obtenu après chromatographie éclair suivie d'une trituration dans le pentane sous forme d'un solide beige (52 mg). Rdt = 44 %. Tf = 111,7 - 113,5 °C. RMN ¹H (300 MHz, DMSO-d₆, δ ppm) 10,87 (s, 1H), 10,36 (s, 1H), 7,76 (d, *J* = 8,1 Hz, 2H), 7,64 (d, *J* = 7,8 Hz, 1H), 7,39 - 7,31 (m, 2H), 7,27 - 7,21 (m, 1H), 7,14 - 6,89 (m, 5H), 6,81 (d, *J* = 8,1 Hz, 2H), 6,10 (s, 1H), 4,02 (d, *J* = 17,3 Hz, 1H), 3,99 - 3,80 (m, 2H), 3,55 (d, *J* = 17,1 Hz, 1H), 3,05 (t, *J* = 7,9 Hz, 2H). RMN ¹³C (75 MHz, DMSO-d₆, δ ppm) 194,9, 177,1, 162,7, 144,1, 136,7, 131,8, 131,0, 131,0, 129,4, 128,3, 127,6, 123,8, 123,6, 122,0, 121,5, 118,9, 118,6, 115,6, 115,6, 111,9, 111,4, 108,7, 73,2, 46,1, 40,5, 23,2. IR-TF (neat, cm⁻¹) 3400, 2901, 1691, 1669, 1599, 1579, 1356, 1232, 1167, 844, 794. HRMS (ESI, m/z) calculé pour C₂₆H₂₂N₂O₄Na [M+Na]⁺ : 449,1477 déterminé 449,1466. *Conditions de chromatographie éclair* : colonne 10 g, débit 8 mL/min, cyclohexane/EtOAc 100/0 à 50/50. R*_{f}* = 0,11 cyclohexane/EtOAc 50/50.

### 1-(3-Ethyl-(1H)-indol-3-yl)-3-hydroxy-3-(2-oxo-2-(2-aminophényl)-éthyl)-indol-2-one (APV-ST432)

Le composé APV-ST432 a été préparé suivant le mode opératoire général D en utilisant la 1-(3-Ethyl-(1*H*)-indol-3-yl)-indol-2,3-dione (80 mg, 0,28 mmol) et la 2'-Aminoacétophenone (47 µL, 0,39 mmol). Le composé indiqué dans le titre a été obtenu après chromatographie éclair suivie d'une trituration dans le pentane sous forme d'un solide beige (82 mg). Rdt = 70 %. Tf = 149,6 - 151,5 °C. RMN ¹H (300 MHz, DMSO-d₆, δ ppm) 10,89 (s, 1H), 7,81 (d, *J* = 8,1 Hz, 1H), 7,66 (d, *J* = 7,7 Hz, 1H), 7,41 - 7,31 (m, 3H), 7,30 - 7,19 (m, 2H), 7,14 - 6,92 (m, 6H), 6,70 (d, *J* = 8,4 Hz, 1H), 6,61 - 6,53 (m, 1H), 6,10 (s, 1H), 4,07 (d, *J* = 17,2 Hz, 1H), 4,03 - 3,86 (m, 2H), 3,63 (d, *J* = 17,2 Hz, 1H), 3,08 (t, *J* = 7,8 Hz, 2H). RMN ¹³C (75 MHz, DMSO-d₆, δ ppm) 198,6, 177,2, 151,5, 144,2, 136,7, 134,8, 132,0, 131,8, 129,4, 127,6, 123,8, 123,6, 122,0, 121,5, 118,9, 118,6, 117,4, 116,5, 115,0, 111,9, 111,4, 108,7, 73,3, 47,0, 40,5, 23,1. IR-TF (neat, cm⁻¹) 3329, 2910, 1712, 1613, 1359, 1163, 740. HRMS (ESI, m/z) calculé pour C₂₆H₂₄N₃O₃ [M+H]⁺ : 426,1818 déterminé 426,1826 et pour C₂₆H₂₃N₃O₃Na [M+Na]⁺ : 448,1637 déterminé 488,1636. *Conditions de chromatographie éclair* : colonne 10 g, débit 8 mL/min, cyclohexane/EtOAc 100/0 à 50/50. R*_{f}* = 0,38 cyclohexane/EtOAc 50/50.

### 1-(3-Ethyl-(1H)-indol-3-yl)-3-hydroxy-3-(2-oxo-2-(3-aminophényl)-éthyl)-indol-2-one (APV-ST362)

Le composé APV-ST362 a été préparé suivant le mode opératoire général D en utilisant la 1-(3-Ethyl-(1*H*)-indol-3-yl)-indol-2,3-dione (80 mg, 0,28 mmol) et la 3'-Aminoacétophenone (53 mg, 0,39 mmol). Le composé indiqué dans le titre a été obtenu après chromatographie éclair suivie d'une trituration dans le pentane sous forme d'un solide beige (105 mg). Rdt = 89 %. Tf = 190,3 - 192,1 °C. RMN ¹H (300 MHz, DMSO-d₆, δ ppm) 10,88 (s, 1H), 7,65 (d, *J* = 7,6 Hz, 1H), 7,40 - 7,22 (m, 4H), 7,17 - 6,90 (m, 7H), 6,78 (d, *J* = 8,2 Hz, 1H), 6,12 (s, 1H), 5,30 (s, 2H), 4,01 (d, *J* = 17,6 Hz, 1H), 4,10 - 3,81 (m, 2H), 3,57 (d, *J* = 17,5 Hz, 1H), 3,06 (t, *J* = 7,9 Hz, 2H). RMN ¹³C (75 MHz, DMSO-d₆, δ ppm) 196,7, 176,6, 149,0, 143,7, 136,9, 136,3, 131,2, 129,1, 129,1, 127,1, 123,4, 123,2, 121,6, 121,0, 118,7, 118,4, 118,1, 115,5, 112,4, 111,5, 110,9, 108,3, 72,7, 46,1, 40,1, 22,7. IR-TF (neat, cm⁻¹) 3361, 2903, 1694, 1618, 1468, 1654, 1350, 1169, 1061, 738. HRMS (ESI, m/z) calculé pour C₂₆H₂₄N₃O₃ [M+H]⁺ : 426,1818 déterminé 426,1812. *Conditions de chromatographie éclair:* colonne 25 g, débit 18 mL/min, cyclohexane/EtOAc 100/0 à 50/50. R*_{f}* = 0,08 cyclohexane/EtOAc 50/50.

### 1-(3-Ethyl-(1H)-indol-3-yl)-3-hydroxy-3-(2-oxo-2-(4-aminophényl)-éthyl)-indol-2-one (APV-ST433)

Le composé APV-ST433 a été préparé suivant le mode opératoire général D en utilisant la 1-(3-Ethyl-(1*H*)-indol-3-yl)-indol-2,3-dione (80 mg, 0,28 mmol) et la 4'-Aminoacétophenone (53 mg, 0,39 mmol). Le composé indiqué dans le titre a été obtenu après chromatographie éclair suivie d'une trituration dans le pentane sous forme d'un solide beige (68 mg). Rdt = 58 %. Tf = 141,6 - 143,4 °C. RMN ¹H (300 MHz, DMSO-d₆, δ ppm) 10,87 (s, 1H), 7,65 (d, *J* = 7,7 Hz, 1H), 7,60 (d, *J* = 8,5 Hz, 2H), 7,40 - 7,20 (m, 4H), 7,14 - 7,01 (m, 2H), 6,99 - 6,89 (m, 2H), 6,53 (d, *J* = 8,5 Hz, 2H), 6,11 - 6,01 (m, 3H), 4,03 - 3,80 (m, 2H), 3,93 (d, *J* = 17,0 Hz, 1H), 3,47 (d, *J*= 17,0 Hz, 1H), 3,05 (t, *J* = 7,9 Hz, 2H). RMN ¹³C (75 MHz, DMSO-d₆, δ ppm) 193,7, 177,2, 154,3, 144,2, 136,7, 132,0, 130,8, 130,8, 129,3, 127,6, 124,5, 123,7, 123,6, 122,0, 121,5, 118,9, 118,6, 112,9, 112,9, 111,9, 111,4, 108,6, 73,3, 45,7, 40,5, 23,2. IR-TF (neat, cm⁻¹) 3357, 2942, 1712, 1614, 1591, 1562, 1355, 1229, 1176, 740. HRMS (ESI, m/z) calculé pour C₂₆H₂₄N₃O₃ [M+H]⁺ : 426,1818 déterminé 426,1810. *Conditions de chromatographie éclair* : colonne 10 g, débit 8 mL/min, cyclohexane/EtOAc 100/0 à 50/50. R*_{f}* = 0,10 cyclohexane/EtOAc 50/50.

### 1-(3-Ethyl-(1H)-indol-3-yl)-3-hydroxy-3-(2-oxo-2-(3-méthylphényl)-éthyl)-indol-2-one (APV-ST373)

Le composé APV-ST373 a été préparé suivant le mode opératoire général D en utilisant la 1-(3-Ethyl-(1*H*)-indol-3-yl)-indol-2,3-dione (80 mg, 0,28 mmol) et la 3'-Méthylacétophenone (51 µL, 0,39 mmol). Le composé indiqué dans le titre a été obtenu après chromatographie éclair suivie d'une trituration dans le pentane sous forme d'un solide beige (85 mg). Rdt = 73 %. Tf = 107,9 - 109,9 °C. RMN ¹H (300 MHz, DMSO-d₆, δ ppm) 10,89 (s, 1H), 7,71 (s, 1H), 7,69 - 7,63 (m, 1H), 7,46 - 7,30 (m, 5H), 7,30 - 7,22 (m, 1H), 7,14 - 6,91 (m, 5H), 6,16 (s, 1H), 4,13 (d, *J* = 17,5 Hz, 1H), 4,07 - 3,84 (m, 2H), 3,65 (d, *J* = 17,5 Hz, 1H), 3,07 (t, *J* = 7,8 Hz, 2H), 2,35 (s, 3H). RMN ¹³C (75 MHz, DMSO-d₆, δ ppm) 197,0, 177,0, 144,1, 138,6, 136,7, 136,6, 134,5, 131,6, 129,6, 129,0, 128,8, 127,6, 125,6, 123,9, 123,6, 122,1, 121,5, 118,9, 118,6, 111,9, 111,3, 108,8, 73,2, 46,6, 40,5, 23,2, 21,2. IR-TF (neat, cm⁻¹) 3380, 3001, 1688, 1612, 1565, 1422, 1341, 1200, 1067, 741. HRMS (ESI, m/z) calculé pour C₂₇H₂₅N₂O₃ [M+H]⁺ : 425,1865 déterminé 425,1864 et pour C₂₇H₂₄N₂O₃Na [M+Na]⁺ : 447,1685 déterminé 447,1685. *Conditions de chromatographie éclair:* colonne 25 g, débit 18 mL/min, cyclohexane/EtOAc 100/0 à 50/50. R*_{f}* = 0,45 cyclohexane/EtOAc 50/50.

### 1-(3-Ethyl-(1H)-indol-3-yl)-3-hydroxy-3-(2-oxo-2-(4-méthylphényl)-éthyl)-indol-2-one (APV-ST426)

Le composé APV-ST426 a été préparé suivant le mode opératoire général D en utilisant la 1-(3-Ethyl-(1*H*)-indol-3-yl)-indol-2,3-dione (80 mg, 0,28 mmol) et la 4'-Méthylacétophenone (52 mg, 0,39 mmol). Le composé indiqué dans le titre a été obtenu après chromatographie éclair suivie d'une trituration dans le pentane sous forme d'un solide beige (101 mg). Rdt = 86 %. Tf = 198,4 -200,1 °C. RMN ¹H (300 MHz, DMSO-d₆, δ ppm) 10,88 (s, 1H), 7,79 (d, *J* = 7,9 Hz, 2H), 7,65 (d, *J* = 7,5 Hz, 1H), 7,42 - 7,22 (m, 6H), 7,14 - 6,90 (m, 4H), 6,16 (s, 1H), 4,10 (d, *J* = 17,4 Hz, 1H), 4,03 - 3,83 (m, 2H), 3,63 (d, *J* = 17,4 Hz, 1H), 3,06 (t, *J* = 7,8 Hz, 2H), 2,36 (s, 3H). RMN ¹³C (75 MHz, DMSO-d₆, δ ppm) 196,4, 177,0, 144,3, 144,1, 136,7, 134,2, 131,6, 129,7, 129,7, 129,5, 128,5, 128,5, 127,6, 123,9, 123,6, 122,1, 121,5, 118,9, 118,6, 111,9, 111,3, 108,8, 73,2, 46,4, 40,5, 23,2, 21,6. IR-TF (neat, cm⁻¹) 3318, 2935, 1703, 1675, 1605, 1340, 1183, 1169, 1066, 739. HRMS (ESI, m/z) calculé pour C₂₇H₂₄N₂O₃Na [M+Na]⁺ : 447,1685 déterminé 447,1673. *Conditions de chromatographie éclair* : colonne 10 g, débit 8 mL/min, cyclohexane/EtOAc 100/0 à 50/50. R*_{f}* = 0,45 cyclohexane/EtOAc 50/50.

### 1-(3-Ethyl-(1H)-indol-3-yl)-3-hydroxy-3-(2-oxo-2-(2-méthoxyphényl)-éthyl)-indol-2-one (APV-ST402)

Le composé APV-ST402 a été préparé suivant le mode opératoire général D en utilisant la 1-(3-Ethyl-(1*H*)-indol-3-yl)-indol-2,3-dione (80 mg, 0,28 mmol) et la 2'-Méthoxyacétophenone (53 µL, 0,39 mmol). Le composé indiqué dans le titre a été obtenu après chromatographie éclair suivie d'une trituration dans le pentane sous forme d'un solide beige (102 mg). Rdt = 84 %. Tf= 108,4 - 110,3 °C. RMN ¹H (300 MHz, DMSO-d₆, δ ppm) 10,89 (s, 1H), 7,66 (d, *J* = 7,6 Hz, 1H), 7,56 - 7,48 (m, 1H), 7,39 (d, *J* = 7,9 Hz, 1H), 7,35 - 7,23 (m, 4H), 7,19 - 6,90 (m, 6H), 6,12 (s, 1H), 4,07 - 3,83 (m, 6H), 3,71 (d, *J* = 17,8 Hz, 1H), 3,07 (t, *J* = 7,7 Hz, 2H). RMN ¹³C (75 MHz, DMSO-d₆, δ ppm) 198,0, 177,0, 158,9, 144,1, 136,7, 134,6, 131,6, 129,8, 129,5, 127,6, 127,4, 123,8, 123,6, 122,1, 121,5, 120,9, 118,9, 118,6, 112,9, 111,9, 111,4, 108,7, 73,2, 56,3, 51,8, 40,5, 23,2. IR-TF (neat, cm⁻¹) 3355, 2942, 1701, 1614, 156, 1467, 1339, 1244, 1163, 1016, 755. HRMS (ESI, m/z) calculé pour C₂₇H₂₅N₂O₄ [M+H]⁺ : 441,1814 déterminé 441,1814 et pour C₂₇H₂₄N₂O₄Na [M+Na]⁺ : 463,1634 déterminé 463,1640. *Conditions de chromatographie éclair:* colonne 25 g, débit 18 mL/min, cyclohexane/EtOAc 100/0 à 50/50. R*_{f}* = 0,24 cyclohexane/EtOAc 50/50.

### 1-(3-Ethyl-(1H)-indol-3-yl)-3-hydroxy-3-(2-oxo-2-(3-méthoxyphényl)-éthyl)-indol-2-one (APV-ST403)

Le composé APV-ST403 a été préparé suivant le mode opératoire général D en utilisant la 1-(3-Ethyl-(1*H*)-indol-3-yl)-indol-2,3-dione (80 mg, 0,28 mmol) et la 3'-Méthoxyacétophenone (53 µL, 0,39 mmol). Le composé indiqué dans le titre a été obtenu après chromatographie éclair suivie d'une trituration dans le pentane sous forme d'un solide beige (110 mg). Rdt = 91 %. Tf = 193,3 - 195,2 °C. RMN ¹H (300 MHz, DMSO-d₆, δ ppm) 10,88 (s, 1H), 7,65 (d, *J* = 7,6 Hz, 1H), 7,52 (d, *J* = 7,5 Hz, 1H), 7,46 - 7,31 (m, 5H), 7,30 - 7,22 (m, 1H), 7,22 - 7,16 (m, 1H), 7,14 - 6,92 (m, 4H), 6,16 (s, 1H), 4,14 (d, *J* = 17,6 Hz, 1H), 3,99 - 3,86 (m, 2H), 3,79 (s, 3H), 3,67 (d, *J* = 17,6 Hz, 1H), 3,06 (t, *J* = 7,8 Hz, 2H). RMN ¹³C (75 MHz, DMSO-d₆, δ ppm) 196,7, 177,0, 159,8, 144,1, 137,9, 136,7, 131,6, 130,3, 129,6, 127,6, 123,9, 123,6, 122,2, 121,5, 121,0, 120,2, 118,8, 118,6, 112,5, 111,9, 111,3, 108,8, 73,6, 55,8, 46,7, 40,6, 23,2. IR-TF (neat, cm⁻¹) 3326, 2923, 1704, 1674, 1613, 1412, 1339, 1256, 1069, 981, 746. HRMS (ESI, m/z) calculé pour C₂₇H₂₅N₂O₄ [M+H]⁺ : 441,1814 déterminé 441,1815 et pour C₂₇H₂₄N₂O₄Na [M+Na]⁺ : 463,1634 déterminé 463,1642. *Conditions de chromatographie éclair* : colonne 25 g, débit 18 mL/min, cyclohexane/EtOAc 100/0 à 50/50. R*_{f}* = 0,30 cyclohexane/EtOAc 50/50.

### 1-(3-Ethyl-(1H)-indol-3-yl)-3-hydroxy-3-(2-oxo-2-(4-méthoxyphényl)-éthyl)-indol-2-one (APV-ST429)

Le composé APV-ST429 a été préparé suivant le mode opératoire général D en utilisant la 1-(3-Ethyl-(1*H*)-indol-3-yl)-indol-2,3-dione (80 mg, 0,28 mmol) et la 4'-Méthoxyacétophenone (58 mg, 0,39 mmol). Le composé indiqué dans le titre a été obtenu après chromatographie éclair suivie d'une trituration dans le pentane sous forme d'un solide beige (82 mg). Rdt = 68 %. Tf = 183,8 - 185,6 °C. RMN ¹H (300 MHz, DMSO-d₆, δ ppm) 10,88 (s, 1H), 7,87 (d, *J* = 8,7 Hz, 2H), 7,65 (d, *J* = 7,6 Hz, 1H), 7,42 - 7,30 (m, 3H), 7,28 - 7,22 (m, 1H), 7,14 - 6,90 (m, 6H), 6,14 (s, 1H), 4,07 (d, *J* = 17,2 Hz, 1H), 4,00 - 3,85 (m, 2H), 3,83 (s, 3H), 3,60 (d, *J* = 17,3 Hz, 1H), 3,06 (t, *J* = 7,8 Hz, 2H). RMN ¹³C (75 MHz, DMSO-d₆, δ ppm) 195,2, 177,0, 163,7, 144,1, 136,7, 131,7, 130,77, 130,8, 129,6, 129,5, 127,6, 123,9, 123,6, 122,1, 121,5, 118,9, 118,6, 114,3, 114,3, 111,9, 111,3, 108,8, 73,2, 56,0, 46,2, 40,5, 23,2. IR-TF (neat, cm⁻¹) 3319, 2904, 1705, 1667, 1597, 1340, 1234, 1171, 1067, 1025, 852, 742. HRMS (ESI, m/z) calculé pour C₂₇H₂₄N₂O₄Na [M+Na]⁺ : 463,1634 déterminé 463,1622. *Conditions de chromatographie éclair* : colonne 10 g, débit 8 mL/min, cyclohexane/EtOAc 100/0 à 50/50. R*_{f}* = 0,23 cyclohexane/EtOAc 50/50.

### 1-(3-Ethyl-(1H)-indol-3-yl)-3-hydroxy-3-(2-oxo-2-(3-bromophényl)-éthyl)-indol-2-one (APV-ST374)

Le composé APV-ST374 a été préparé suivant le mode opératoire général D en utilisant la 1-(3-Ethyl-(1*H*)-indol-3-yl)-indol-2,3-dione (80 mg, 0,28 mmol) et la 3'-Bromoacétophenone (51 µL, 0,39 mmol). Le composé indiqué dans le titre a été obtenu après chromatographie éclair suivie d'une trituration dans le pentane sous forme d'un solide beige (91 mg). Rdt = 68 %. Tf = 148,1 - 149,7 °C. RMN ¹H (300 MHz, DMSO-d₆, δ ppm) 10,88 (s, 1H), 8,01 (s, 1H), 7.90 (d, *J* = 7.7 Hz, 1H), 7.82 (d, *J* = 8.1 Hz, 1H), 7.65 (d, *J* = 7,6 Hz, 1H), 7,52 - 7,43 (m, 1H), 7,38 (d, *J* = 7,6 Hz, 2H), 7,31 (d, *J* = 1,5 Hz, 1H), 7,31 - 7,23 (m, 1H), 7,17 - 6,92 (m, 4H), 6,20 (s, 1H), 4,14 (d, *J* = 17,5 Hz, 1H), 4,06 - 3,82 (m, 2H), 3,65 (d, *J* = 17,4 Hz, 1H), 3,06 (t, *J* = 7,8 Hz, 2H). RMN ¹³C (75 MHz, DMSO-d₆, δ ppm) 196,0, 176,8, 144,0, 138,6, 136,7, 136,5, 131,4, 131,4, 131,4, 131,0, 129,6, 127,5, 124,1, 123,6, 122,6, 122,2, 121,5, 118,9, 118,6, 111,9, 111,3, 108,8, 73,2, 46,6, 40,5, 23,2. IR-TF (neat, cm⁻¹) 3328, 2914, 1704, 1679, 1614, 1468, 1339, 1165, 1074, 740. HRMS (ESI, m/z) calculé pour C₂₆H₂₂N₂O₃Br [M+H]⁺ : 489,0814 déterminé 489,0824 et pour C₂₆H₂₁N₂O₃BrNa [M+Na]⁺ : 511,0633 déterminé 511,0635. *Conditions de chromatographie éclair:* colonne 25 g, débit 18 mL/min, cyclohexane/EtOAc 100/0 à 50/50. R*_{f}* = 0,45 cyclohexane/EtOAc 50/50.

### 1-(3-Ethyl-(1H)-indol-3-yl)-3-hydroxy-3-(2-oxo-2-(4-(trifluorométhoxy)-phényl)-éthyl)-indol-2-one (APV-ST427)

Le composé APV-ST427 a été préparé suivant le mode opératoire général D en utilisant la 1-(3-Ethyl-(1*H*)-indol-3-yl)-indol-2,3-dione (80 mg, 0,28 mmol) et la 4'-(Trifluorométhoxy)acétophenone (62 µL, 0,39 mmol). Le composé indiqué dans le titre a été obtenu après chromatographie éclair suivie d'une trituration dans le pentane sous forme d'un solide beige (109 mg). Rdt = 80 %. Tf = 154,4 - 156,2 °C. RMN ¹H (300 MHz, DMSO-d₆, δ ppm) 10,88 (s, 1H), 8,03 (d, *J* = 8,6 Hz, 2H), 7,64 (d, *J* = 7,7 Hz, 1H), 7,47 (d, *J* = 8,6 Hz, 2H), 7,41 - 7,33 (m, 2H), 7,31 (br. s, 1H), 7,29 - 7,22 (m, 1H), 7,15 - 6,90 (m, 4H), 6,21 (s, 1H), 4,14 (d, *J* = 17,4 Hz, 1H), 4,07 - 3,78 (m, 2H), 3,67 (d, *J* = 17,4 Hz, 1H), 3,06 (t, *J* = 7,9 Hz, 2H). RMN ¹³C (75 MHz, DMSO-d₆, δ ppm) 195,8, 176,9, 152,1, 144,0, 136,7, 135,4, 131,4, 131,0, 131,0, 129,6, 127,5, 124,0, 123,6, 122,2, 121,5, 121,1, 121,1, 118,9, 118,6, 111,9, 111,3, 108,8, 73,2, 46,6, 40,5, 23,2. NB : Le carbone en alpha des fluors (-CF3) n'est pas visible. RMN ¹⁹F (188 MHz, DMSO-d₆, δ ppm) -57,0. IR-TF (neat, cm⁻¹) 3334, 1703, 1681, 1260, 1214, 1168, 1066, 741. HRMS (ESI, m/z) calculé pour C₂₇H₂₂N₂O₄F₃ [M+H]⁺ : 495,1532 déterminé 495,1541 et pour C₂₇H₂₁N₂O₄F₃Na [M+Na]⁺ : 517,1351 déterminé 517,1347. *Conditions de chromatographie éclair :* colonne 10 g, débit 8 mL/min, cyclohexane/EtOAc 100/0 à 50/50. R*_{f}* = 0,49 cyclohexane/EtOAc 50/50.

### 1-(3-Ethyl-(1H)-indol-3-yl)-3-hydroxy-3-(2-oxo-2-(3-(trifluoromethyl)-phényl)-éthyl)-indol-2-one (APV-ST363)

Le composé APV-ST363 a été préparé suivant le mode opératoire général D en utilisant la 1-(3-Ethyl-(1*H*)-indol-3-yl)-indol-2,3-dione (80 mg, 0,28 mmol) et la 3'-(trifluorométhyl)acétophenone (59 µL, 0,39 mmol). Le composé indiqué dans le titre a été obtenu après chromatographie éclair suivie d'une trituration dans le pentane sous forme d'un solide beige (92 mg). Rdt = 70 %. Tf = 113,2 - 114,8 °C. RMN ¹H (300 MHz, DMSO-d₆, δ ppm) 10,89 (s, 1H), 8,21 (d, *J* = 8,0 Hz, 1H), 8,14 (s, 1H), 8,00 (d, *J* = 7,7 Hz, 1 H), 7,81 - 7,69 (m, 1H), 7,64 (d, *J* = 7,6 Hz, 1H), 7,43 - 7,34 (m, 2H), 7,32 - 7,24 (m, 2H), 7,13 - 6,92 (m, 4H), 6,23 (s, 1H), 4,22 (d, *J* = 17,4 Hz, 1H), 4,05 - 3,80 (m, 2H), 3,72 (d, *J* = 17,4 Hz, 1H), 3,05 (t, *J* = 7,8 Hz, 2H). RMN ¹³C (75 MHz, DMSO-d₆, δ ppm)195,7, 176,3, 143,4, 136,8, 136,3, 132,1, 130,9, 130,1, 129,9 - 129,5 (m, CH couplé fluor), 129,2, 127,1, 124,5 - 124,2 (m, CH couple fluor), 123,7, 123,2, 121,8, 121,0, 118,4, 118,1, 111,5, 110,8, 108,4, 72,7, 46,2, 40,1, 22,8. NB : Les carbones quaternaires en alpha et beta des fluors ne sont pas visible (-C-CF3). RMN ¹⁹F (188 MHz, DMSO-d₆, δ ppm) -61,7. IR-TF (neat, cm⁻¹)3307, 1690, 1615, 1468, 1352, 1201, 1168, 1127, 1072, 744. HRMS (ESI, m/z) calculé pour C₂₇H₂₂N₂O₃F₃ [M+H]⁺ : 479,1583 déterminé 479,1588 et pour C₂₇H₂₁N₂O₃F₃Na [M+Na]⁺ : 501,1402 déterminé 501,1400. *Conditions de chromatographie éclair* : colonne 10 g, débit 8 mL/min, cyclohexane/EtOAc 100/0 à 50/50. R*_{f}* = 0,19 cyclohexane/EtOAc 50/50.

### 1-(3-Ethyl-(1H)-indol-3-yl)-3-hydroxy-3-(2-oxo-2-(2-fluorophényl)-éthyl)-indol-2-one (APV-ST416)

Le composé APV-ST416 a été préparé suivant le mode opératoire général D en utilisant la 1-(3-Ethyl-(1*H*)-indol-3-yl)-indol-2,3-dione (80 mg, 0,28 mmol) et la 2'-Fluoroacétophenone (47 µL, 0,39 mmol). Le composé indiqué dans le titre a été obtenu après chromatographie éclair suivie d'une trituration dans le pentane sous forme d'un solide beige (89 mg). Rdt = 75 %. Tf = 180,0 - 181,8 °C. RMN ¹H (300 MHz, DMSO-d₆, δ ppm) 10,88 (s, 1H), 7,76 - 7,54 (m, 3H), 7,47 - 7,20 (m, 6H), 7,14 - 6,91 (m, 4H), 6,19 (s, 1H), 4,01 (d, *J* = 17,9 Hz, 1H), 4,10 - 3,82 (m, 2H), 3,68 (d, *J* = 17,8, 1H), 3,05 (t, *J* = 7,9 Hz, 2H). RMN ¹³C (75 MHz, DMSO-d₆, δ ppm) 194,78, 176,77, 161,48 (d, *J* = 254,2 Hz), 144,02, 136,72, 135,84 (d, *J* = 9,0 Hz), 131,36, 130,51, 129,63, 127,53, 125,25 (d, *J* = 3,4 Hz), 123,91, 123,62, 122,17 (d, *J* = 8,0 Hz),121,47, 118,87, 118,55, 117,33 (d, *J* = 23,2 Hz), 111,92, 111,28, 108,83, 72,95, 50,83, 40,53, 23,19. NB : Le carbone quaternaire situé en alpha du C=O et C-F n'est pas visible. RMN ¹⁹F (188 MHz, DMSO-d₆, δ ppm) -107,9. IR-TF (neat, cm⁻¹) 3330, 1702, 1608, 1470, 1450, 1342, 1172, 1060, 745. HRMS (ESI, m/z) calculé pour C₂₆H₂₂N₂O₃F [M+H]⁺ : 429,1614 déterminé 429,1614. *Conditions de chromatographie éclair:* colonne 25 g, débit 18 mL/min, cyclohexane/EtOAc 100/0 à 50/50. R*_{f}* = 0,47 cyclohexane/EtOAc 50/50.

### 1-(3-Ethyl-(1H)-indol-3-yl)-3-hydroxy-3-(2-oxo-2-(3-fluorophényl)-éthyl)-indol-2-one (APV-ST372)

Le composé APV-ST372 a été préparé suivant le mode opératoire général D en utilisant la 1-(3-Ethyl-(1*H*)-indol-3-yl)-indol-2,3-dione (80 mg, 0,28 mmol) et la 3'-Fluoroacétophenone (48 µL, 0,39 mmol). Le composé indiqué dans le titre a été obtenu après chromatographie éclair suivie d'une trituration dans le pentane sous forme d'un solide beige (88 mg). Rdt = 75 %. Tf = 107,5 - 109,2 °C. RMN ¹H (300 MHz, DMSO-d₆, δ ppm) 10,89 (s, 1H), 7,76 (d, *J* = 7,6 Hz, 1H), 7,69 - 7,62 (m, 2H), 7,60 - 7,44 (m, 2H), 7,37 (d, *J* = 7,6 Hz, 2H), 7,32 - 7,23 (m, 2H), 7,16 - 6,90 (m, 4H), 6,20 (s, 1H), 4,14 (d, *J* = 17,5 Hz, 1H), 4,04 - 3,84 (m, 2H), 3,66 (d, *J* = 17,5 Hz, 1H), 3,06 (t, *J* = 7,8 Hz, 2H). RMN ¹³C (75 MHz, DMSO-d₆, δ ppm) 195,54, 176,40, 162,12 (d, *J_{C-F}* = 245,3 Hz), 143,54, 138,30 (d, *J_{C-F}* = 6,2 Hz), 136,27, 130,97, 130,93 (d, *J_{C-F}* = 6,8 Hz), 129,19, 127,09, 124,27 (d, *J_{C-F}* = 3,4 Hz), 123,56, 123,19, 121,74, 121,02, 120,36 (d, *J_{C-F}* = 21,3 Hz), 118,43, 118,11, 114,46 (d, *J_{C-F}* = 22,4 Hz), 111,48, 110,83, 108,38, 72,70, 46,24, 40,07, 22,76. RMN ¹⁹F (188 MHz, DMSO-d₆, δ ppm) -109,6. IR-TF (neat, cm⁻¹) 3314, 2943, 1682, 1614, 1468, 1340, 1247, 1167, 1060, 739. HRMS (ESI, m/z) calculé pour C₂₆H₂₂N₂O₃F [M+H]⁺ : 429,1614 déterminé 429,1607 et pour C₂₆H₂₁N₂O₃FNa [M+Na]⁺ : 451,1434 déterminé 451,1432. *Conditions de chromatographie éclair* : colonne 25 g, débit 18 mL/min, cyclohexane/EtOAc 100/0 à 50/50. R*_{f}* = 0,46 cyclohexane/EtOAc 50/50.

### 1-(3-Ethyl-(1H)-indol-3-yl)-3-hydroxy-3-(2-oxo-2-(4-fluorophényl)-éthyl)-indol-2-one (APV-ST417)

Le composé APV-ST417 a été préparé suivant le mode opératoire général D en utilisant la 1-(3-Ethyl-(1*H*)-indol-3-yl)-indol-2,3-dione (80 mg, 0,28 mmol) et la 4'-Fluoroacétophenone (47 µL, 0,39 mmol). Le composé indiqué dans le titre a été obtenu après chromatographie éclair suivie d'une trituration dans le pentane sous forme d'un solide beige (100 mg). Rdt = 85 %. Tf = 113,3 - 115,2 °C. RMN ¹H (300 MHz, DMSO-d₆, δ ppm) 10,88 (s, 1H), 8,02 - 7,93 (m, 2H), 7,64 (d, *J* = 7,2 Hz, 1H), 7,40 - 7,22 (m, 6H), 7,14 - 6,90 (m, 4H), 6,17 (s, 1H), 4,12 (d, *J* = 17,4 Hz, 1H), 4,02 - 3,83 (m, 2H), 3,64 (d, *J* = 17,4 Hz, 1H), 3,06 (d, *J* = 8,0 Hz, 2H), RMN ¹³C (75 MHz, DMSO-d₆, δ ppm) 195,1, 176,4, 165,1 (d, *J_{C-F}* = 252,0 Hz), 143,5, 136,3, 132,9 (d, *J_{C-F}* = 2,5 Hz), 131,1, 131,0 (d, *J_{C-F}* = 9,1 Hz, 2*CH), 129,1, 127,1, 123,5, 123,2, 121,7, 121,0, 118,4, 118,1, 115,7 (d, *J_{C-F}* = 21,9 Hz, 2*CH), 111,6, 110,8, 108,3, 72,7, 46,0, 40,1, 22,7. RMN ¹⁹F (188 MHz, DMSO-d₆, δ ppm) -103,7. IR-TF (neat, cm⁻¹)3371, 3057, 1682, 1614, 1596, 1340, 1227, 1157, 841, 739. HRMS (ESI, m/z) calculé pour C₂₆H₂₂N₂O₃F [M+H]⁺ : 429,1614 déterminé 429,1609 et pour C₂₆H₂₁N₂O₃FNa [M+Na]⁺ : 451,1434 déterminé 451,1430. *Conditions de chromatographie éclair:* colonne 25 g, débit 18 mL/min, cyclohexane/EtOAc 100/0 à 50/50. R*_{f}* = 0,38 cyclohexane/EtOAc 50/50.

### 1-(3-Ethyl-1-méthyl-(1H)-indol-3-yl)-3-hydroxy-3-(2-oxo-2-(2-chlorophényl)-éthyl)-indol-2-one (APV-ST521)

Le composé APV-ST521 a été préparé suivant le mode opératoire général D en utilisant la 1-(3-Ethyl-1-méthyl-(1*H*)-indol-3-yl)-indol-2,3-dione (45 mg, 0,15 mmol) et la 2'-Chloroacétophenone (39 µL, 0,21 mmol). Le composé indiqué dans le titre a été obtenu après chromatographie éclair suivie d'une trituration dans le pentane sous forme d'un solide beige (40 mg). Rdt = 60 %. Tf = 149,8 - 151,8 °C. RMN ¹H (300 MHz, DMSO-d₆, δ ppm) 7,64 (d, *J* = 7,7 Hz, 1H), 7,52 - 7,36 (m, 6H), 7,28 (d, *J* = 6,0 Hz, 2H), 7,20 - 7,13 (m, 1H), 7,09 - 6,95 (m, 3H), 6,24 (s, 1H), 3,98 - 3,83 (m, 3H), 3,62 (d, *J* = 16,5 Hz, 1H), 3,32 (s, 3H), 3,01 (t, *J* = 7,6 Hz, 2H). RMN ¹³C (75 MHz, DMSO-d₆, δ ppm) 198,6, 176,1, 143,3, 137,9, 136,7, 132,5, 130,4, 130,4, 129,6, 129,3, 129,2, 127,6, 127,4, 127,3, 123,8, 121,9, 121,1, 118,5, 118,3, 110,1, 109,7, 108,5, 72,8, 49,8, 40,1, 32,3, 22,5. IR-TF (neat, cm⁻¹) 3334, 1686, 1614, 1470, 1394, 1370, 1118, 992, 831, 731. HRMS (ESI, m/z) calculé pour C₂₇H₂₃N₂O₃ClNa [M+Na]⁺ : 481,1295 déterminé 481,1292. *Conditions de chromatographie éclair:* colonne 12 g, débit 15 mL/min, cyclohexane/EtOAc 100/0 à 60/40. R*_{f}* = 0,54 cyclohexane/EtOAc 50/50.

### 1-(3-Propyl-(1H)-indol-3-yl)-3-hydroxy-3-(2-oxo-2-phényléthyl)-indol-2-one (APV-ST389)

Le composé APV-ST389 a été préparé suivant le mode opératoire général D en utilisant la 1-(3-Propyl-(1*H*)-indol-3-yl)-indol-2,3-dione (80 mg, 0,26 mmol) et l'Acétophenone (43 µL, 0,37 mmol). Le composé indiqué dans le titre a été obtenu après chromatographie éclair suivie d'une trituration dans le pentane sous forme d'un solide beige (100 mg). Rdt = 90 %. Tf = 120,9 - 122,8 °C. RMN ¹H (300 MHz, DMSO-d₆, δ ppm) 10,78 (s, 1H), 7,87 (d, *J* = 7,5 Hz, 2H), 7,65 - 7,54 (m, 2H), 7,52 - 7,45 (m, 2H), 7,35 (d, *J* = 7,6 Hz, 2H), 7,26 - 7,17 (m, 2H), 7,10 - 7,04 (m, 1H), 6,97 (d, *J* = 7,7 Hz, 2H), 6,95 - 6,89 (m, 1H), 6,15 (s, 1H), 4,14 (d, *J=* 17,6 Hz, 1H), 3,85 - 3,72 (m, 2H), 3,68 (d, *J* = 17,6 Hz, 1H), 2,83 (t, *J* = 7,6 Hz, 2H), 2,09 - 1,96 (m, 2H). RMN ¹³C (75 MHz, DMSO-d₆, δ ppm) 196,9, 177,1, 144,3, 136,8, 136,5, 133,9, 131,7, 129,5, 129,2, 129,2, 128,3, 128,3, 127,6, 123,8, 122,7, 122,1, 121,3, 118,8, 118,5, 114,5, 111,8, 108,8, 73,1, 46,5, 39,7, 28,3, 22,6. IR-TF (neat, cm⁻¹) 3294, 2926, 1682, 1613, 1468, 1339, 1213, 1162, 1065, 739. HRMS (ESI, m/z) calculé pour C₂₇H₂₅N₂O₃ [M+H]⁺ : 425,1865 déterminé 425,1870. *Conditions de chromatographie éclair:* colonne 25 g, débit 18 mL/min, cyclohexane/EtOAc 100/0 à 50/50. R*_{f}* = 0,32 cyclohexane/EtOAc 50/50.

### 1-(3-Propyl-(1H)-indol-3-yl)-3-hydroxy-3-(2-oxo-2-(2-chlorophényl)-éthyl)-indol-2-one (APV-ST388)

Le composé APV-ST388 a été préparé suivant le mode opératoire général D en utilisant la 1-(3-Propyl-(1H)-indol-3-yl)-indol-2,3-dione (80 mg, 0,26 mmol) et la 2'-Chloroacétophenone (48 µL, 0,37 mmol). Le composé indiqué dans le titre a été obtenu après chromatographie éclair suivie d'une trituration dans le pentane sous forme d'un solide beige (83 mg). Rdt = 69 %. Tf = 126,2 - 127,8 °C, RMN ¹H (300 MHz, DMSO-d₆, δ ppm) 10,76 (s, 1H), 7,53 (d, *J* = 7,8 Hz, 1H), 7,49 - 7,43 (m, 3H), 7,40 - 7,32 (m, 3H), 7,29 - 7,23 (m, 1H), 7,17 (br, s, 1H), 7,09 - 7,03 (m, 1H), 7,00 - 6,92 (m, 3H), 6,22 (s, 1H), 3,93 (d, *J* = 17,0 Hz, 1H), 3,83 - 3,67 (m, 2H), 3,62 (d, *J* = 16,8 Hz, 1H), 2,87 - 2,76 (m, 2H), 2,06 - 1,96 (m, 2H), RMN ¹³C (75 MHz, DMSO-d₆, δ ppm) 199,0, 176,7, 144,1, 138,4, 136,8, 132,9, 131,0, 130,9, 130,1, 129,7, 129,7, 127,8, 127,6, 124,1, 122,7, 122,2, 121,3, 118,8, 118,5, 114,4, 111,8, 109,0, 73,2, 50,3, 39,7, 28,2, 22,6. IR-TF (neat, cm⁻¹) 3293, 3229, 2929, 1695, 1680, 1616, 1468, 1354, 1164, 1059, 739. HRMS (ESI, m/z) calculé pour C₂₇H₂₄N₂O₃Cl [M+H]⁺ : 459,1475 déterminé 459,1472. *Conditions de chromatographie éclair:* colonne 25 g, débit 18 mL/min, cyclohexane/EtOAc 100/0 à 50/50. R*_{f}* = 0,38 cyclohexane/EtOAc 50/50.

### 1-(3-Propyl-(1H)-indol-3-yl)-3-hydroxy-3-(2-oxo-2-(3-chlorophényl)-éthyl)-indol-2-one (APV-ST387)

Le composé APV-ST387 a été préparé suivant le mode opératoire général D en utilisant la 1-(3-Propyl-(1H)-indol-3-yl)-indol-2,3-dione (80 mg, 0,26 mmol) et la 3'-Chloroacétophenone (48 µL, 0,37 mmol). Le composé indiqué dans le titre a été obtenu après chromatographie éclair suivie d'une trituration dans le pentane sous forme d'un solide beige (93 mg). Rdt = 72 %. Tf = 126,0 - 127,9 °C. RMN ¹H (300 MHz, DMSO-d₆, δ ppm) 10,77 (s, 1H), 7,85 (s, 1H), 7,84 (d, *J* = 7,1 Hz, 1H), 7,69 (d, *J* = 7,5 Hz, 1H), 7,55 (d, *J* = 7,6 Hz, 1H), 7,50 (d, *J* = 8,2 Hz, 1H), 7,36 (d, *J =* 3,2 Hz, 1H), 7,33 (d, *J* = 3,7 Hz, 1H), 7,27 - 7,20 (m, 1H), 7,17 (d, *J* = 2,3 Hz, 1H), 7,10 - 7,03 (m, 1H), 6,97 (d, *J* = 7,9 Hz, 2H), 6,92 (d, *J* = 7,8 Hz, 1H), 6,15 (s, 1H), 4,14 (d, *J* = 17,5 Hz, 1H), 3,86 - 3,60 (m, 2H), 3,66 (d, *J* = 17,5 Hz, 1H), 2,89 - 2,76 (m, 2H), 2,09 - 1,92 (m, 2H), RMN ¹³C (75 MHz, DMSO-d₆, δ ppm) 196,0, 177,05, 144,2, 138,3, 136,8, 134,1, 133,6, 131,5, 131,2, 129,6, 128,0, 127,6, 127,1, 123,9, 122,7, 122,1, 121,3, 118,8, 118,5, 114,5, 111,8, 108,8, 73,1, 46,6, 39,7, 28,3, 22,6. IR-TF (neat, cm⁻¹) 3341, 2920, 1687, 1613, 1468, 1338, 1209, 1163, 1082, 781, 740. HRMS (ESI, m/z) calculé pour C₂₇H₂₄N₂O₃Cl [M+H]⁺ : 459,1475 déterminé 459,1478. *Conditions de chromatographie éclair:* colonne 25 g, débit 18 mL/min, cyclohexane/EtOAc 100/0 à 50/50. R*_{f}* = 0,37 cyclohexane/EtOAc 50/50.

### 1-(3-Propyl-(1H)-indol-3-yl)-3-hydroxy-3-(2-oxo-2-(4-chlorophényl)-éthyl)-indol-2-one (APV-ST413)

Le composé APV-ST413 a été préparé suivant le mode opératoire général D en utilisant la 1-(3-Propyl-(1H)-indol-3-yl)-indol-2,3-dione (80 mg, 0,26 mmol) et la 4'-Chloroacétophenone (48 µL, 0,37 mmol). Le composé indiqué dans le titre a été obtenu après chromatographie éclair suivie d'une trituration dans le pentane sous forme d'un solide beige (119 mg). Rdt = 99 %. Tf = 129,8 - 131,7 °C. RMN ¹H (300 MHz, DMSO-d₆, δ ppm) 10,77 (s, 1H), 7,88 (d, *J* = 8,2 Hz, 2H), 7,54 (d, *J* = 7,9 Hz, 3H), 7,34 (d, *J* = 7,9 Hz, 2H), 7,27 - 7,19 (m, 1H), 7,18 (s, 1H), 7,10 - 7,02 (m, 1H), 6,99 - 6,90 (m, 3H), 6,16 (s, 1H), 4,11 (d, *J* = 17,8 Hz, 1H), 3,84 - 3,67 (m, 2H), 3,64 (d, *J* = 17,6 Hz, 1H), 2,82 (t, *J* = 7,5 Hz, 2H), 2,07 - 1,95 (m, 2H). RMN ¹³C (75 MHz, DMSO-d₆, δ ppm) 196,0, 177,0, 144,2, 138,8, 136,8, 135,2, 131,5, 130,3, 130,3, 129,5, 129,2, 129,2, 127,6, 123,9, 122,7, 122,1, 121,3, 118,8, 118,5, 114,5, 111,8, 108,8, 73,1, 46,5, 39,7, 28,2, 22,6. IR-TF (neat, cm⁻¹)3393, 2921, 1681, 1614, 1588, 1468, 1356, 1213, 1092, 990, 740. HRMS (ESI, m/z) calculé pour C₂₇H₂₄N₂O₃Cl [M+H]⁺ : 459,1475 déterminé 459,1479 et pour C₂₇H₂₃N₂O₃Cl Na [M+Na]⁺ : 481,1295 déterminé 481,1297. *Conditions de chromatographie éclair :* colonne 25 g, débit 18 mL/min, cyclohexane/EtOAc 100/0 à 50/50. R*_{f}* = 0,38 cyclohexane/EtOAc 50/50.

### 1-(3-Propyl-(1H)-indol-3-yl)-3-hydroxy-3-(2-oxo-2-(3-méthylphényl)-éthyl)-indol-2-one (APV-ST414)

Le composé APV-ST414 a été préparé suivant le mode opératoire général D en utilisant la 1-(3-Propyl-(1H)-indol-3-yl)-indol-2,3-dione (80 mg, 0,26 mmol) et la 3'-Méthylacétophenone (50 µL, 0,37 mmol). Le composé indiqué dans le titre a été obtenu après chromatographie éclair suivie d'une trituration dans le pentane sous forme d'un solide beige (114 mg). Rdt = 99 %. Tf = 92,2 - 94,2 °C. RMN ¹H (300 MHz, DMSO-d₆, δ ppm) 10,78 (s, 1H), 7,68 (s, 2H), 7,57 (d, *J* = 7,7 Hz, 1H), 7,45 - 7,32 (m, 4H), 7,26 - 7,18 (m, 2H), 7,11 - 7,04 (m, 1H), 7,01 - 6,89 (m, 3H), 6,14 (s, 1H), 4,12 (d, *J* = 17,6 Hz, 1H), 3,88 - 3,62 (m, 2H), 3,66 (d, *J* = 17,7 Hz, 1H), 2,84 (t, *J =* 7,3 Hz, 2H), 2,33 (s, 3H), 2,11 - 1,95 (m, 2H). RMN ¹³C (75 MHz, DMSO-d₆, δ ppm) 196,9, 177,1, 144,4, 138,5, 136,8, 136,6, 134,5, 131,7, 129,5, 129,0, 128,8, 127,6, 125,6, 123,7, 122,7, 122,1, 121,3, 118,8, 118,5, 114,5, 111,8, 108,8, 73,1, 46,6, 39,7, 28,3, 22,6, 21,2. IR-TF (neat, cm⁻¹)3350, 2929, 1680, 1613, 1468, 1339, 1249, 1163, 1083, 739. HRMS (ESI, m/z) calculé pour C₂₈H₂₇N₂O₃ [M+H]⁺ : 439,2022 déterminé 439,2026 et pour C₂₈H₂₆N₂O₃Na [M+Na]⁺ : 461,1814 déterminé 461,1842. *Conditions de chromatographie éclair* : colonne 25 g, débit 18 mL/min, cyclohexane/EtOAc 100/0 à 50/50. R*_{f}* = 0,36 cyclohexane/EtOAc 50/50.

### 1-(3-Propyl-(1H)-indol-3-yl)-3-hydroxy-3-(2-oxo-2-(3-fluorophényl)-éthyl)-indol-2-one (APV-ST407)

Le composé APV-ST407 a été préparé suivant le mode opératoire général D en utilisant la 1-(3-Propyl-(1H)-indol-3-yl)-indol-2,3-dione (80 mg, 0,26 mmol) et la 3'-Fluoroacétophenone (45 µL, 0,37 mmol). Le composé indiqué dans le titre a été obtenu après chromatographie éclair suivie d'une trituration dans le pentane sous forme d'un solide beige (110 mg). Rdt = 95 %. Tf = 104 - 105,9 °C. RMN ¹H (300 MHz, DMSO-d₆, δ ppm) 10,78 (s, 1H), 7,75 (d, *J* = 7,5 Hz, 1H), 7,63 (d, *J* = 9,6 Hz, 1H), 7,59 - 7,43 (m, 3H), 7,35 (d, *J* = 7,6 Hz, 2H), 7,27 - 7,17 (m, 2H), 7,10 - 7,03 (m, 1H), 7,01 - 6,90 (m, 3H), 6,17 (s, 1H), 4,15 (d, *J* = 17,7 Hz, 1H), 3,87 - 3,66 (m, 3H), 3,68 (d, *J* = 17,6 Hz, 1H), 2,83 (t, *J* = 7,6 Hz, 2H), 2,11 - 1,95 (m, 2H). RMN ¹³C (75 MHz, DMSO-d₆, δ ppm) 195,5, 176,5, 162,1 (d, *J_{C-F}* = 245,1 Hz), 143,8, 138,2 (d, *J_{C-F}* = 5,5 Hz), 136,3, 131,0, 130,9 (d, *J_{C-F}* = 7,6 Hz), 129,1, 127,1, 124,2 (d, *J_{C-F} =* 2,0 Hz), 123,4, 122,3, 121,7, 120,8, 120,3 (d, *J_{C-F}* = 21,7 Hz), 118,3, 118,0, 114,4 (d, *J_{C-F}* = 22,3 Hz), 114,0, 111,3, 108,4, 72,6, 46,2, 39,3, 27,8, 22,1. RMN ¹⁹F (188 MHz, DMSO-d₆, δ ppm) -112,1. IR-TF (neat, cm⁻¹) 3343, 2907, 1687, 1613, 1339, 1248, 1167, 739. HRMS (ESI, m/z) calculé pour C₂₇H₂₄N₂O₃F [M+H]⁺ : 443,1771 déterminé 443,1763 et pour C₂₇H₂₃N₂O₃FNa [M+Na]⁺ : 465,1590 déterminé 465,1581. *Conditions de chromatographie éclair:* colonne 25 g, débit 18 mL/min, cyclohexane/EtOAc 100/0 à 50/50. R*_{f}* = 0,49 cyclohexane/EtOAc 50/50.

### 1-(3-Propyl-(1H)-indol-3-yl)-3-hydroxy-3-(2-oxo-2-(4-fluorophényl)-éthyl)-indol-2-one (APV-ST412)

Le composé APV-ST412 a été préparé suivant le mode opératoire général D en utilisant la 1-(3-Propyl-(1H)-indol-3-yl)-indol-2,3-dione (80 mg, 0,26 mmol) et la 4'-Fluoroacétophenone (45 µL, 0,37 mmol). Le composé indiqué dans le titre a été obtenu après chromatographie éclair suivie d'une trituration dans le pentane sous forme d'un solide beige (99 mg). Rdt = 85 %. Tf = 94,4 - 96,2 °C. RMN ¹H (300 MHz, DMSO-d₆, δ ppm) 10,78 (s, 1H), 7,98 (d, *J* = 5,7 Hz, 1H), 7,95 (d, *J* = 5,5 Hz, 1H), 7,56 (d, *J* = 7,8 Hz, 1H), 7,37 - 7,17 (m, 6H), 7,11 - 7,04 (m, 1H), 7,01 - 6,89 (m, 3H), 6,16 (s, 1H), 4,12 (d, *J* = 17,6 Hz, 1H), 3,88 - 3,67 (m, 2H), 3,66 (d, *J =* 17,6 Hz, 1H), 2,83 (t, *J* = 7,5 Hz, 2H), 2,09 - 1,96 (m, 2H), RMN ¹³C (75 MHz, DMSO-d₆, δ ppm) 195,03, 176,56, 165,10 (d, *J* = 252,1 Hz), 143,81, 136,30, 132,85 (d, *J* = 1,9 Hz), 131,14, 131,02, 130,90, 129,04, 127,13, 123,34, 122,26, 121,62, 120,84, 118,20 (d, *J* = 20,8 Hz, 2*CH), 115,67 (d, *J* = 21,9 Hz, 2*CH), 114,01, 111,32, 108,35, 72,63, 45,98, 39,24, 27,79, 22,13. RMN ¹⁹F (188 MHz, DMSO-d₆, δ ppm)-103,8. IR-TF (neat, cm⁻¹) 3371, 2914, 1681, 1614, 1596, 1468, 1339, 1227, 1157, 1083, 992, 740. HRMS (ESI, m/z) calculé pour C₂₇H₂₄N₂O₃F [M+H]⁺ : 443,1771 déterminé 443,1768 et pour C₂₇H₂₃N₂O₃FNa [M+Na]⁺ : 465,1590 déterminé 465,1597. *Conditions de chromatographie éclair:* colonne 25 g, débit 18 mL/min, cyclohexane/EtOAc 100/0 à 50/50. R*_{f}* = 0,51 cyclohexane/EtOAc 50/50.

### 1-(3-Propyl-(1H)-indol-3-yl)-3-hydroxy-3-(2-oxo-2-((3-trifluorométhyl)-phényl)-éthyl)-indol-2-one (APV-ST420)

Le composé APV-ST420 a été préparé suivant le mode opératoire général D en utilisant la 1-(3-propyl-(1*H*)-indol-3-yl)-indol-2,3-dione (80 mg, 0,26 mmol) et la 3'-(Trifluorométhyl)acétophenone (56 µL, 0,37 mmol). Le composé indiqué dans le titre a été obtenu après chromatographie éclair suivie d'une trituration dans le pentane sous forme d'un solide beige (96 mg). Rdt = 71 %. Tf = 97,7 - 99,6 °C. RMN ¹H (300 MHz, DMSO-d₆, δ ppm) 10,77 (s, 1H), 8,19 (d, *J* = 7,9 Hz, 1H), 8,11 (s, 1H), 7,98 (d, *J* = 7,5 Hz, 1H), 7,78 - 7,69 (m, 1H), 7,55 (d, *J* = 7,8 Hz, 1H), 7,36 (d, *J* = 5,8 Hz, 1H), 7,34 (d, *J* = 7,2 Hz, 1H), 7,27 - 7,20 (m, 1H), 7,18 (br. s, 1H), 7,11 - 7,01 (m, 1H), 7,01 - 6,89 (m, 3H), 6,19 (s, 1H), 4,22 (d, *J* = 17,6 Hz, 1H), 3,86 - 3,66 (m, 2H), 3,73 (d, *J* = 17,6 Hz, 1H), 2,82 (t, *J* = 7,4 Hz, 2H), 2,10 - 1,93 (m, 2H), RMN ¹³C (75 MHz, DMSO-d₆, δ ppm) 195,6, 176,4, 143,7, 136,7, 136,3, 132,0, 131,0, 130,1, 129,9 - 129,6 (m, CH couplé fluor), 129,1, 127,1, 124,5 - 124,1 (m, CH couplé fluor), 123,5, 122,2, 121,7, 120,8, 118,3, 118,0, 114,0, 111,3, 108,4, 72,7, 46,2, 39,3, 27,8, 22,1. NB : Les carbones quaternaires en alpha et beta des fluors ne sont pas visible (-C-CF3). RMN ¹⁹F (188 MHz, DMSO-d₆, δ ppm) -61,7, IR-TF (neat, cm⁻¹) 3368, 3941, 1690, 1612, 1324, 1201, 1165, 1122, 1070, 740. HRMS (ESI, m/z) calculé pour C₂₃H₂₃N₂O₃F₃Na [M+Na]⁺ : 515,1558 déterminé 515,1554. *Conditions de chromatographie éclair* : colonne 25 g, débit 18 mL/min, cyclohexane/EtOAc 100/0 à 50/50. R*_{f}* = 0,40 cyclohexane/EtOAc 50/50.

### 1-(5-Chloro-3-propyl-(1H)-indol-3-yl)-3-hydroxy-3-(2-oxo-2-phenyléthyl)-indol-2-one (APV-ST297)

Le composé APV-ST297 a été préparé suivant le mode opératoire général D en utilisant la 1-(5-Chloro-3-propyl-(1H)-indol-3-yl)-indol-2,3-dione (38 mg, 0,11 mmol) et l'Acétophenone (18 µL, 0,16 mmol). Le composé indiqué dans le titre a été obtenu après chromatographie éclair suivie d'une trituration dans le pentane sous forme d'un solide beige (30 mg). Rdt = 60 %. Tf = 170,7 - 172,4 °C. RMN ¹H (300 MHz, DMSO-d₆, δ ppm) 10,99 (s, 1H), 7,89 (d, *J* = 7,8 Hz, 2H), 7,68 - 7,55 (m, 2H), 7,48 (d, *J* = 7,5 Hz, 1H), 7,46 (d, *J* = 7,7 Hz, 1H), 7,36 (d, *J* = 2,9 Hz, 1H), 7,34 (s, 1H), 7,30 - 7,20 (m, 2H), 7,05 (dd, *J* = 8,3, 1,5 Hz, 1H), 7,02 - 6,87 (m, 2H), 6,14 (s, 1H), 4,14 (d, *J* = 17,7 Hz, 1H), 3,89 - 3,62 (m, 2H), 3,68 (d, *J* = 17,6 Hz, 1H), 2,91 - 2,72 (m, 2H), 2,08 - 1,94 (m, 2H). RMN ¹³C (75 MHz, DMSO-d₆, δ ppm) 196,9, 177,1, 144,3, 136,5, 135,2, 133,9, 131,7, 129,5, 129,1, 129,1, 128,8, 128,4, 128,4, 124,7, 123,8, 123,3, 122,1, 121,2, 118,1, 114,6, 113,3, 108,8, 73,1, 46,5, 39,6, 28,3, 22,3. IR-TF (neat, cm⁻¹) 3301, 2930, 1693, 1613, 1491, 1468, 1349, 1215, 1162, 796. HRMS (ESI, m/z) calculé pour C₂₇H₂₃N₂O₃Cl [M+H]⁺ : 459,1475 déterminé 459,1469 et pour C₂₇H₂₃N₂O₃ClNa [M+Na]⁺ : 481,1295 déterminé 481,1292. *Conditions de chromatographie éclair* : colonne 25 g, débit 18 mL/min, cyclohexane/EtOAc 100/0 à 50/50. R*_{f}* = 0,33 cyclohexane/EtOAc 50/50.

### 1-(5-Chloro-3-propyl-(1H)-indol-3-yl)-3-hydroxy-3-(2-oxo-2-(2-chlorophényl)-éthyl)-indol-2-one (APV-ST385)

Le composé APV-ST385 a été préparé suivant le mode opératoire général D en utilisant la 1-(5-Chloro-3-propyl-(1*H*)-indol-3-yl)-indol-2,3-dione (125 mg, 0,37 mmol) et le 2'-chloroacétophenone (69 µL, 0,52 mmol). Le composé indiqué dans le titre a été obtenu après chromatographie éclair suivie d'une trituration dans le pentane sous forme d'un solide beige (115 mg). Rdt = 63 %. Tf = 135,2 - 137,1 °C. RMN ¹H (300 MHz, DMSO-d₆, δ ppm) 10,98 (s, 1H), 7,56 (s, 1H), 7,50 - 7,42 (m, 3H), 7,41 - 7,32 (m, 3H), 7,30 - 7,22 (m, 2H), 7,05 (dd, *J* = 8,5, 1,3 Hz, 1H), 7,02 - 6,93 (m, 2H), 6,22 (s, 1H), 3,94 (d, *J* = 16,9 Hz, 1H), 3,84 - 3,67 (m, 2H), 3,63 (d, *J* = 17,0 Hz, 1H), 2,80 (t, *J* = 7,4 Hz, 2H), 2,05 - 1,91 (m, 2H). RMN ¹³C (75 MHz, DMSO-d₆, δ ppm) 199,1, 176,7, 144,1, 138,4, 135,2, 132,9, 131,0, 130,9, 130,1, 129,7, 129,7, 128,7, 127,7, 124,7, 124,1, 123,3, 122,3, 121,2, 118,1, 114,5, 113,3, 109,0, 73,1, 50,3, 39,7, 28,2, 22,3. IR-TF (neat, cm⁻¹) 3353, 1701, 16313, 1468, 1160, 1063, 892, 795, 752. HRMS (ESI, m/z) calculé pour C₂₇H₂₃N₂O₃Cl₂ [M+H]⁺ : 493,1086 déterminé 493,1076. *Conditions de chromatographie éclair* : colonne 25 g, débit 18 mL/min, cyclohexane/EtOAc 100/0 à 50/50. R*_{f}* = 0,39 cyclohexane/EtOAc 50/50.

### 1-(5-Chloro-3-propyl-(1H)-indol-3-yl)-3-hydroxy-3-(2-oxo-2-(3-chlorophényl)-éthyl)-indol-2-one (APV-ST298)

Le composé APV-ST298 a été préparé suivant le mode opératoire général D en utilisant la 1-(5-Chloro-3-propyl-(1H)-indol-3-yl)-indol-2,3-dione (38 mg, 0,11 mmol) et le 3'-Chloroacétophenone (21 µL, 0,16 mmol). Le composé indiqué dans le titre a été obtenu après chromatographie éclair suivie d'une trituration dans le pentane sous forme d'un solide beige (36 mg). Rdt = 65 %. Tf = 96,1 - 98,0 °C. RMN ¹H (300 MHz, CDCl₃, δ ppm) 8,16 (s, 1H), 7,86 (s, 1H), 7,75 (d, *J* = 7,8 Hz, 1H), 7,58 - 7,49 (m, 2H), 7,43 - 7,23 (m, 4H), 7,17 - 7,00 (m, 3H), 6,81 (d, *J* = 7,8 Hz, 1H), 3,89 - 3,70 (m, 3H), 3,56 (d, *J* = 17,3 Hz, 1H), 3,08 (br, s), 2,84 (t, *J* = 7,6 Hz, 2H), 2,21 - 2,08 (m, 2H), RMN ¹³C (75 MHz, CDCl₃, δ ppm) 196,5, 176,4, 143,2, 137,8, 135,1, 134,7, 133,6, 130,1, 130,0, 129,8, 128,5, 128,2, 126,3, 124,9, 124,0, 123,3, 123,0, 122,2, 118,3, 114,9, 112,1, 109,0, 74,2, 45,1, 39,8, 27,2, 22,2. IR-TF (neat, cm⁻¹)3343, 2980, 1686, 1613, 1491, 1345, 1209, 1161, 1072, 782, 753. HRMS (ESI, m/z) calculé pour C₂₇H₂₃N₂O₃Cl₂ [M+H]⁺ : 493,1086 déterminé 493,1097 et pour C₂₇H₂₂N₂O₃Cl₂Na [M+Na]⁺ : 515,0905 déterminé 515,0930. *Conditions de chromatographie éclair:* colonne 25 g, débit 18 mL/min, cyclohexane/EtOAc 100/0 à 50/50. R*_{f}* = 0,32 cyclohexane/EtOAc 50/50.

### 1-(5-Chloro-3-propyl-(1H)-indol-3-yl)-3-hydroxy-3-(2-oxo-2-(4-chlorophényl)-éthyl)-indol-2-one (APV-ST422)

Le composé APV-ST422 a été préparé suivant le mode opératoire général D en utilisant la 1-(5-Chloro-3-propyl-(1H)-indol-3-yl)-indol-2,3-dione (80 mg, 0,24 mmol) et le 4'-Chloroacétophenone (43 µL, 0,33 mmol). Le composé indiqué dans le titre a été obtenu après chromatographie éclair suivie d'une trituration dans le pentane sous forme d'un solide beige (84 mg). Rdt = 72 %. Tf = 147,4 - 149,2 °C. RMN ¹H (300 MHz, DMSO-d₆, δ ppm) 11,01 (s, 1H), 7,90 (d, *J* = 8,4 Hz, 2H), 7,59 (s, 1H), 7,53 (d, *J* = 8,3 Hz, 2H), 7,41 - 7,32 (m, 2H), 7,28 (d, *J* = 1,7 Hz, 1H), 7,23 (d, *J* = 7,7 Hz, 1H), 7,06 (dd, *J* = 8,6, 1,4 Hz, 1H), 7,01 - 6,91 (m, 2H), 6,19 (s, 1H), 4,14 (d, *J* = 17,6 Hz, 1H), 3,88 - 3,59 (m, 2H), 3,67 (d, *J* = 17,6 Hz, 1H), 2,84 - 2,76 (m, 2H), 2,08 - 1,94 (m, 2H). RMN ¹³C (75 MHz, DMSO-d₆, δ ppm) 196,0, 177,0, 144,2, 138,9, 135,2, 135,2, 131,5, 130,3, 130,3, 129,5, 129,2, 129,2, 128,8, 124,7, 123,8, 123,4, 122,1, 121,2, 118,1, 114,6, 113,3, 108,9, 73,1, 46,5, 39,7, 28,3, 22,3. IR-TF (neat, cm⁻¹) 3368, 2941, 1680, 1613, 1588, 1467, 1342, 1212, 1092, 1066, 990, 794, 753. HRMS (ESI, m/z) calculé pour C₂₇H₂₂N₂O₃Cl₂Na [M+Na]⁺ : 515,0905 déterminé 515,0911. *Conditions de chromatographie éclair* : colonne 25 g, débit 18 mL/min, cyclohexane/EtOAc 100/0 à 50/50. R*_{f}* = 0,45 cyclohexane/EtOAc 50/50.

### 1-(5-Chloro-3-propyl-(1H)-indol-3-yl)-3-hydroxy-3-(2-oxo-2-(pyridin-3-yl)-éthyl)-indol-2-one (APV-ST302)

Le composé APV-ST302 a été préparé suivant le mode opératoire général D en utilisant la 1-(5-Chloro-3-propyl-(1*H*)-indol-3-yl)-indol-2,3-dione (120 mg, 0,35 mmol) et la 3-Acétylpyridine (55 µL, 0,50 mmol). Le composé indiqué dans le titre a été obtenu après chromatographie éclair suivie d'une trituration dans le pentane sous forme d'un solide beige (107 mg). Rdt = 66 %. Tf = 113,7 - 115,6 °C. RMN ¹H (300 MHz, DMSO-d₆, δ ppm) 11,00 (s, 1H), 9,06 (d, *J* = 1,7 Hz, 1H), 8,76 (d, *J* = 4,8 Hz, 1H), 8,20 (d, *J* = 8,3 Hz, 1H), 7,58 (s, 1H), 7,50 (dd, *J* = 8,1, 4,6 Hz, 1H), 7,37 (d, *J* = 2,8 Hz, 1H), 7,35 (d, *J* = 3,9 Hz, 1H), 7,32 - 7,20 (m, 2H), 7,12 - 6,87 (m, 3H), 6,18 (s, 1H), 4,18 (d, *J* = 17,4 Hz, 1H), 3,90 - 3,59 (m, 2H), 3,69 (t, *J =* 17,7 Hz, 1H), 2,80 (t, *J* = 8,0 Hz, 2H), 1,99 (t, *J* = 6,3 Hz, 2H). RMN ¹³C (75 MHz, DMSO-d₆, δ ppm) 196,6, 177,0, 154,0, 149,7, 144,2, 135,9, 135,2, 131,8, 131,5, 129,6, 128,8, 124,7, 124,2, 123,9, 123,6, 122,2, 121,2, 118,1, 114,5, 113,3, 108,9, 73,1, 46,7, 39,7, 28,3, 22,3. IR-TF (neat, cm⁻¹) 3355, 2953, 1740, 1612, 1468, 1354, 1097, 1068, 860, 753. HRMS (ESI, m/z) calculé pour C₂₆H₂₃N₃O₃Cl [M+H]⁺ : 460,1428 déterminé 460,1420 et pour C₂₆H₂₂N₃O₃ClNa [M+Na]⁺ : 482,1247 déterminé 482,1238. *Conditions de chromatographie éclair:* colonne 25 g, débit 18 mL/min, cyclohexane/EtOAc 100/0 à 50/50. R*_{f}* = 0,05 cyclohexane/EtOAc 50/50.

### 1-(5-Chloro-3-propyl-(1H)-indol-3-yl)-3-hydroxy-3-(2-oxo-2-(3-méthylphényl)-éthyl)-indol-2-one (APV-ST423)

Le composé APV-ST423 a été préparé suivant le mode opératoire général D en utilisant la 1-(5-Chloro-3-propyl-(1H)-indol-3-yl)-indol-2,3-dione (80 mg, 0,24 mmol) et le 3'-Méthylacétophenone (45 µL, 0,33 mmol). Le composé indiqué dans le titre a été obtenu après chromatographie éclair suivie d'une trituration dans le pentane sous forme d'un solide beige (95 mg). Rdt = 85 %. Tf = 125,9 - 127,8 °C. RMN ¹H (300 MHz, DMSO-d₆, δ ppm) 11,00 (s, 1H), 7,68 (br, s, 2H), 7,60 (br, s, 1H), 7,45 - 7,31 (m, 4H), 7,28 (br, s, 1H), 7,22 (d, *J* = 7,7 Hz, 1H), 7,06 (dd, *J* = 8,5, 1,6 Hz, 1H), 7,02 - 6,88 (m, 2H), 6,13 (s, 1H), 4,13 (d, *J* = 17,6 Hz, 1H), 3,91 - 3,59 (m, 2H), 3,66 (d, *J* = 17,5 Hz, 1H), 2,90 - 2,73 (m, 2H), 2,32 (s, 3H), 2,08 - 1,92 (m, 2H). RMN ¹³C (75 MHz, DMSO-d₆, δ ppm) 197,0, 177,1, 144,3, 138,5, 136,6, 135,2, 134,5, 131,7, 129,5, 129,0, 128,8, 128,8, 125,6, 124,7, 123,7, 123,3, 122,1, 121,2, 118,1, 114,6, 113,3, 108,8, 73,1, 46,6, 39,6, 28,3, 22,3, 21,2F. IR-TF (neat, cm⁻¹) 3375, 2941, 1680, 1614, 1467, 1250, 1162, 1067, 799, 752. HRMS (ESI, m/z) calculé pour C₂₃H₂₅N₂O₃ClNa [M+Na]⁺ : 495,1451 déterminé 495,1463. *Conditions de chromatographie éclair* : colonne 25 g, débit 18 mL/min, cyclohexane/EtOAc 100/0 à 50/50. R*_{f}* = 0,45 cyclohexane/EtOAc 50/50.

### 1-(5-Chloro-3-propyl-(1H)-indol-3-yl)-3-hydroxy-3-(2-oxo-2-(3-fluorophényl)-éthyl)-indol-2-one (APV-ST424)

Le composé APV-ST424 a été préparé suivant le mode opératoire général D en utilisant la 1-(5-Chloro-3-propyl-(1H)-indol-3-yl)-indol-2,3-dione (80 mg, 0,24 mmol) et le 3'-Fluoroacétophenone (41 µL, 0,33 mmol). Le composé indiqué dans le titre a été obtenu après chromatographie éclair suivie d'une trituration dans le pentane sous forme d'un solide beige (70 mg). Rdt = 62 %. Tf = 173,7 - 175,6 °C. RMN ¹H (300 MHz, DMSO-d₆, δ ppm) 7,76 (d, *J* = 7,5 Hz, 1H), 7,64 (d, *J* = 9,7 Hz, 1H), 7,59 (s, *J* = 5,6 Hz, 1H), 7,57 - 7,43 (m, 2H), 7,37 (s, 1H), 7,35 (s, 1H), 7,27 (s, 1H), 7,23 (d, *J* = 7,7 Hz, 1H), 7,06 (dd, *J* = 8,6, 1,7 Hz, 1H), 7,02 - 6,90 (m, 2H), 6,17 (s, 1H), 4,16 (d, *J* = 17,7 Hz, 1H), 3,87 - 3,66 (m, 3H), 3,68 (d, *J* = 17,8 Hz, 1H), 2,89 - 2,71 (m, 2H), 2,09 - 1,92 (m, 2H). RMN ¹³C (75 MHz, DMSO-d₆, δ ppm) 195,57, 176,54, 162,10 (d, *J_{C-F}* = 245,5 Hz), 143,79, 138,22 (d, *J_{C-F} =* 6,1 Hz), 134,70, 131,05, 130,89 (d, *J_{C-F}* = 7,7 Hz), 129,09, 128,29, 124,23, 124,23, 123,39, 122,87, 121,69, 120,77, 120,36 (d, *J_{C-F} =* 21,4 Hz), 117,60, 114,40 (d, *J_{C-F}* = 22,4 Hz), 114,09, 112,86, 108,41, 72,59, 46,17, 39,24, 27,86, 21,86. RMN ¹⁹F (188 MHz, DMSO-d₆, δ ppm) -112,5, IR-TF (neat, cm⁻¹) 3324, 2926, 1688, 1612, 1467, 1442, 1340, 1249, 1164, 892, 792, 753. HRMS (ESI, m/z) calculé pour C₂₇H₂₂N₂O₃FClNa [M+Na]⁺ : 499,1201 déterminé 499,1201. *Conditions de chromatographie éclair :* colonne 25 g, débit 18 mL/min, cyclohexane/EtOAc 100/0 à 50/50. R*_{f}* = 0,40 cyclohexane/EtOAc 50/50.

### 1-(5-Chloro-3-propyl-(1H)-indol-3-yl)-3-hydroxy-3-(2-oxo-2-(4-fluorophényl)-éthyl)-indol-2-one (APV-ST421)

Le composé APV-ST421 a été préparé suivant le mode opératoire général D en utilisant la 1-(5-Chloro-3-propyl-(1H)-indol-3-yl)-indol-2,3-dione (80 mg, 0,24 mmol) et le 4'-Fluoroacétophenone (40 µL, 0,33 mmol). Le composé indiqué dans le titre a été obtenu après chromatographie éclair suivie d'une trituration dans le pentane sous forme d'un solide beige (90 mg). Rdt = 80 %. Tf = 111,1 - 112,9 °C. RMN ¹H (300 MHz, DMSO-d₆, δ ppm) 11,00 (s, 1H), 7,99 (d, *J* = 5,7 Hz, 1H), 7,96 (d, *J =* 5,6 Hz, 1H), 7,59 (s, 1H), 7,38 - 7,30 (m, 3H), 7,30 - 7,19 (m, 3H), 7,06 (dd, *J =* 8,6, 1,7 Hz, 1H), 7,02 - 6,90 (m, 2H), 6,15 (s, 1H), 4,14 (d, *J* = 17,7 Hz, 1H), 3,87 - 3,65 (m, 2H), 3,66 (d, *J* = 17,5 Hz, 1H), 2,87 - 2,74 (m, 2H), 2,08 - 1,92 (m, 2H). RMN ¹³C (75 MHz, DMSO-d₆, δ ppm) 195,10, 176,59, 165,11 (d, *J_{C-F}* = 252,1 Hz), 143,80, 134,70, 132,81, 131,14, 130,98 (d, *J_{C-F}* = 9,4 Hz, 2*CH), 129,04, 128,29, 124,23, 123,34, 122,86, 121,64, 120,76, 117,59, 115,66 (d, *J_{C-F}* = 21,9 Hz, 2*CH), 114,09, 112,85, 108,38, 72,61, 45,96, 39,17, 27,82, 21,82. RMN ¹⁹F (188 MHz, DMSO-d₆, δ ppm) -103,8. IR-TF (neat, cm⁻¹) 3338, 3926, 1628, 1614, 1596, 1467, 1345, 1227, 1157, 1066, 794, 753. HRMS (ESI, m/z) calculé pour C₂₇H₂₂N₂O₃FClNa [M+Na]⁺ : 499,1201 déterminé 499,1201. *Conditions de chromatographie éclair :* colonne 25 g, débit 18 mL/min, cyclohexane/EtOAc 100/0 à 50/50. R*_{f}* = 0,4 0cyclohexane/EtOAc 50/50.

### 1-(5-Chloro-3-propyl-(1H)-indol-3-yl)-3-hydroxy-3-(2-oxo-2-((3-trifluorométhyl)-phényl)-éthyl)-indol-2-one (APV-ST425)

Le composé APV-ST425 a été préparé suivant le mode opératoire général D en utilisant la 1-(5-Chloro-3-propyl-(1H)-indol-3-yl)-indol-2,3-dione (80 mg, 0,24 mmol) et la 3'-(Trifluorométhyl)acétophenone (50 µL, 0,33 mmol). Le composé indiqué dans le titre a été obtenu après chromatographie éclair suivie d'une trituration dans le pentane sous forme d'un solide beige (90 mg). Rdt = 72 %. Tf = 143,1 - 144,9 °C, RMN ¹H (300 MHz, DMSO-d₆, δ ppm) 11,00 (s, 1H), 8,19 (d, *J* = 7,3 Hz, 1H), 8,11 (s, 1H), 7,98 (d, *J* = 7,4 Hz, 1H), 7,77 - 7,67 (m, 1H), 7,57 (s, 1H), 7,42 - 7,31 (m, 2H), 7,31 - 7,19 (m, 2H), 7,10 - 6,89 (m, 3H), 6,19 (s, 1H), 4,23 (d, *J* = 17,6 Hz, 1H), 3,86 - 3,67 (m, 2H), 3,74 (d, *J* = 17,5 Hz, 1H), 2,90 - 2,72 (m, 2H), 2,12 - 1,88 (m, 2H). RMN ¹³C (75 MHz, DMSO-d₆, δ ppm) 195,7, 176,5, 143,7, 136,7, 134,7, 132,0, 131,0, 130,1, 129,8 - 129,6 (m, CH couplé fluor), 129,1, 128,3, 124,5 - 124,2 (m, CH couplé fluor), 124,2, 123,5, 122,9, 121,7, 120,8, 117,6, 114,0, 112,9, 108,4, 72,7, 46,2, 39,2, 27,8, 21,8. NB : Les carbones quaternaires en alpha et beta des fluors ne sont pas visible (-C-CF3). RMN ¹⁹F (188 MHz, DMSO-d₆, δ ppm) -61,7. IR-TF (neat, cm⁻¹) 3309, 2941, 1689, 1613, 1468, 1324, 1201, 1164, 1124, 1070, 793, 753, 693. HRMS (ESI, m/z) calculé pour C₂₃H₂₂N₂O₃F₃ClNa [M+Na]⁺ : 549,1169 déterminé 549,1180. *Conditions de chromatographie éclair :* colonne 25 g, débit 18 mL/min, cyclohexane/EtOAc 100/0 à 50/50. R*_{f}* = 0,40 cyclohexane/EtOAc 50/50.

### 1-(5-Chloro-3-éthyl-(1H)-indol-3-yl)-3-hydroxy-3-(2-oxo-2-phenyléthyl)-indol-2-one (APV-ST409)

Le composé APV-ST409 a été préparé suivant le mode opératoire général D en utilisant la 1-(5-Chloro-3-éthyl-(1*H*)-indol-3-yl)-indol-2,3-dione (80 mg, 0,25 mmol) et l'Acétophenone (57 µL, 0,49 mmol). Le composé indiqué dans le titre a été obtenu après chromatographie éclair suivie d'une trituration dans le pentane sous forme d'un solide blanc (18 mg). Rdt = 20 %. Tf = 170,7 - 172,4 °C. RMN ¹H (300 MHz, DMSO-d₆, δ ppm) 11,09 (s, 1H), 7,89 (d, *J* = 7,9 Hz, 2H), 7,67 - 7,59 (m, 2H), 7,51 (d, *J* = 7,5 Hz, 1H), 7,48 (d, *J* = 7,7 Hz, 1H), 7,41 - 7,34 (m, 3H), 7,27 - 7,20 (m, 1H), 7,09 (dd, *J* = 8,5, 1,3 Hz, 1H), 7,01 - 6,89 (m, 2H), 6,17 (s, 1H), 4,13 (d, *J* = 17,5 Hz, 1H), 3,99 - 3,85 (m, 2H), 3,65 (d, *J* = 17,4 Hz, 1H), 3,04 (t, *J* = 7,7 Hz, 2H). RMN ¹³C (75 MHz, DMSO-d₆, δ ppm) 196,9, 177,0, 144,0, 136,6, 135,2, 133,9, 131,5, 129,5, 129,2, 129,2, 128,7, 128,4, 128,4, 125,6, 123,9, 123,6, 122,1, 121,4, 117,9, 113,4, 111,4, 108,8, 73,2, 46,5, 40,4, 23,2. IR-TF (neat, cm⁻¹) 3357, 2919, 1713, 1683, 1614, 1467, 1346, 1217, 1162, 1096, 1022, 752. HRMS (ESI, m/z) calculé pour C₂₆H₂₂N₂O₃Cl [M+H]⁺ : 445,1319 déterminé 445,1324 et pour C₂₆H₂₁N₂O₃ClNa [M+Na]⁺ : 467,1138 déterminé 467,1136. *Conditions de chromatographie éclair:* colonne 25 g, débit 18 mL/min, cyclohexane/EtOAc 100/0 à 50/50. R*_{f}* = 0,35 cyclohexane/EtOAc 50/50.

### 1-(5-Chloro-3-éthyl-(1H)-indol-3-yl)-3-hydroxy-3-(2-oxo-2-(2-chlorophényl)-éthyl)-indol-2-one (APV-ST410)

Le composé APV-ST410 a été préparé suivant le mode opératoire général D en utilisant la 1-(5-Chloro-3-éthyl-(1H)-indol-3-yl)-indol-2,3-dione (80 mg, 0,25 mmol) et le 2'-Chloroacétophenone (45 µL, 0,39 mmol). Le composé indiqué dans le titre a été obtenu après chromatographie éclair suivie d'une trituration dans le pentane sous forme d'un solide blanc (30 mg). Rdt = 25 %. Tf = 120,1 - 122,0 °C. RMN ¹H (300 MHz, DMSO-d₆, δ ppm) 11,10 (s, 1H), 7,64 (d, *J* = 1,4 Hz, 1H), 7,52 - 7,44 (m, 3H), 7,44 - 7,34 (m, 4H), 7,30 - 7,23 (m, 1H), 7,09 (dd, *J* = 8,6, 1,5 Hz, 1H), 7,01 - 6,93 (m, 2H), 6,24 (s, 1H), 3,92 (d, *J* = 16,4 Hz, 1H), 4,01 - 3,77 (m, 2H), 3,60 (d, *J* = 16,8 Hz, 1H), 3,01 (t, *J* = 7,6 Hz, 2H). RMN ¹³C (75 MHz, DMSO-d₆, δ ppm) 199,0, 176,6, 143,8, 138,4, 135,1, 132,9, 130,9, 130,9, 130,1, 129,7, 129,7, 128,7, 127,8, 125,6, 124,3, 123,6, 122,3, 121,4, 117,9, 113,5, 111,3, 109,0, 73,2, 50,3, 40,4, 23,0. IR-TF (neat, cm⁻¹) 3333, 2917, 2850, 1701, 1613, 1468, 1163, 1096, 1061, 753. HRMS (ESI, m/z) calculé pour C₂₆H₂₁N₂O₃Cl₂ [M+H]⁺ : 479,0929 déterminé 479,0929 et pour C₂₆H₂₆N₂O₃Cl₂Na [M+Na]⁺ : 501,0749 déterminé 501,0746. *Conditions de chromatographie éclair* : colonne 25 g, débit 18 mL/min, cyclohexane/EtOAc 100/0 à 50/50. R*_{f}* = 0,41 cyclohexane/EtOAc 50/50.

### 1-(5-Chloro-3-éthyl-(1H)-indol-3-yl)-3-hydroxy-3-(2-oxo-2-(3-chlorophényl)-éthyl)-indol-2-one (APV-ST411)

Le composé APV-ST411 a été préparé suivant le mode opératoire général D en utilisant la 1-(5-Chloro-3-éthyl-(1*H*)-indol-3-yl)-indol-2,3-dione (80 mg, 0,25 mmol) et le 3'-Chloroacétophenone (45 µL, 0,39 mmol). Le composé indiqué dans le titre a été obtenu après chromatographie éclair suivie d'une trituration dans le pentane sous forme d'un solide blanc (25 mg). Rdt = 21 %. Tf = 120,9 - 122,8 °C. RMN ¹H (300 MHz, CDCl₃, δ ppm) 11,10 (s, 1H), 7,91 - 7,82 (m, 2H), 7,73 - 7,63 (m, 2H), 7,56 - 7,48 (m, 1H), 7,42 - 7,33 (m, 3H), 7,29 - 7,20 (m, 1H), 7,09 (dd, *J* = 8,5, 1,2 Hz, 1H), 7,02 - 6,91 (m, 2H), 6,20 (s, 1H), 4,14 (d, *J* = 17,5 Hz, 1H), 4,03 - 3,79 (m, 2H), 3,64 (d, *J* = 17,5 Hz, 1H), 3,03 (t, *J* = 7,7 Hz, 2H). RMN ¹³C (75 MHz, CDCl₃, δ ppm) 195,5, 176,4, 143,5,137,9, 134,7, 133,7, 133,1, 130,9, 130,7, 129,1, 128,2, 127,7, 126,7, 125,2, 123,6, 123,1, 121,7, 120,9, 117,4, 113,0, 110,9, 108,4, 72,7, 46,2, 40,0, 22,6. IR-TF (neat, cm⁻¹) 3338, 2918, 2850, 1687, 1614, 1468, 1344, 1209, 1164, 1097, 1058, 782, 680. HRMS (ESI, m/z) calculé pour C₂₆H₂₁N₂O₃Cl₂ [M+H]⁺ : 479,0929 déterminé 479,0934 et pour C₂₆H₂₀N₂O₃Cl₂Na [M+Na]⁺ : 501,0759 déterminé 501,0753. *Conditions de chromatographie éclair :* colonne 25 g, débit 18 mL/min, cyclohexane/EtOAc 100/0 à 50/50. R*_{f}* = 0,34 cyclohexane/EtOAc 50/50.

### 4-(2-(1-(2-(1H-indol-3-yl)ethyl)-3-hydroxy-2-oxoindolin-3-yl)acetyl)benzonitrile (APV-GG091)

Le composé APV-GG091 a été préparé suivant le mode opératoire général D en utilisant la 1-(5-Chloro-3-éthyl-(1*H*)-indol-3-yl)-indol-2,3-dione (145 mg, 0,5 mmol) et le 4-acetylbenzonitrile (80 mg, 0,55 mmol). Le composé indiqué dans le titre a été obtenu, après chromatographie éclair sur colonne de gel de silice (cyclohexane/EtOAc 80/20 à 50/50), sous forme d'un solide blanc (90 mg). Rdt = 41 %.
RMN ¹H (300 MHz, CDCl₃, δ ppm) 8,10 (s, 1H), 7,87 (d, *J* = 8,3 Hz, 2H), 7,71 (d, *J* = 8,3 Hz, 2H), 7,46 (d, *J* = 7,9 Hz, 1H), 7,35 (d, *J* = 7,7 Hz, 2H), 7,30 (d, *J* = 7,9 Hz, 1H), 7,17 (t, *J* = 7,7 Hz, 1H), 7,11 (d, *J* = 2,2 Hz, 1H), 7,04 (td, *J* = 7,7, 3,0 Hz, 2H), 6,89 (d, *J* = 7,9 Hz, 1H), 4,14-3,94 (m, 2H), 3,64 (d, *J* = 17,3 Hz, 1H), 3,64 (s, 1H), 3,32 (d, *J* = 17,3 Hz, 1H), 3,2 (t, *J* = 7,2 Hz, 2H). RMN ¹³C (75 MHz, DMSO-d₆, δ ppm) 196,0, 176,3, 143,4, 139,2, 138,2, 136,2, 132,7, 130,8, 129,2, 128,8, 128,6, 127,1, 124,4, 123,6, 123,3, 123,2, 121,8, 121,0, 118,4, 118,1, 115,3, 111,5, 110,8, 108,4, 72,7, 46,5, 22,7. HRMS (ESI, m/z) calculé pour C₂₇H₂₁N₃O₃Na [M+Na]⁺ : 458,1481 déterminé 458,1485.

### 1-(2-(1H-indol-3-yl)ethyl)-3-hydroxy-3-(2-oxopropyl)indolin-2-one (APV-GG057)

Le composé APV-GG057 a été préparé suivant le mode opératoire général D en utilisant la 1-(2-(1H-indol-3-yl)ethyl)indoline-2,3-dione (145 mg, 0,5 mmol) et l'actétone (40 µL, 0,54 mmol). Le composé indiqué dans le titre a été obtenu, après chromatographie sur plaques préparatives (2 x 2 mm ; cyclohexane/EtOAc 50/50), sous forme d'un solide beige (79 mg). Rdt = 45 %.
RMN ¹H (300 MHz, CDCl₃, δ ppm) 8,08 (s, 1H), 7,47 (d, *J* = 7,9 Hz, 1H), 7,35 (d, *J* = 7,0 Hz, 2H), 7,30 (d, *J* = 7,9 Hz, 1H), 7,17 (t, *J* = 7,7 Hz, 1H), 7,09 (d, *J* = 2,2 Hz, 1H), 7,05 (td, *J* = 7,5, 3,8 Hz, 2H), 6,83 (d, *J* = 7,9 Hz, 1H), 4,14 (br s, 1H), 4,13-3,88 (m, 2H), 3,24-3,11 (m, 2H), 2,92 (d, *J* = 16,9 Hz, 1H), 2,69 (d, *J* = 16,9 Hz, 1H), 2,07 (s, 3H). RMN ¹³C (75 MHz, CDCl₃, δ ppm) 208,1, 176,0, 143,1, 137,8, 130,1, 128,6, 124,2, 123,4, 123,1, 122,7, 122,3, 119,6, 118,6, 112,5, 111,5, 109,0, 74,3, 48,4, 41,2, 31,6, 23,0. HRMS (ESI, m/z) calculé pour C₂₁H₂₀N₂O₃Na [M+Na]⁺ : 371,1372 déterminé 371,1360.

### 1-(2-(1H-indol-3-yl)ethyl)-3-hydroxy-3-(3-oxobutan-2-yl)indolin-2-one (APV-GG059)

Le composé APV-GG058 a été préparé suivant le mode opératoire général D en utilisant la 1-(2-(1H-indol-3-yl)ethyl)indoline-2,3-dione (145 mg, 0,5 mmol) et la butan-2-one (50 µL, 0,56 mmol). Le composé indiqué dans le titre a été obtenu, après chromatographie sur sur plaques préparatives (2 x 2 mm ; cyclohexane/EtOAc 50/50, double élution), sous forme d'un solide orange (30 mg). Rdt = 17 %.
RMN ¹H (300 MHz, CDCl₃, δ ppm) 8,14 (s, 1H), 7,49 (d, *J* = 7,9 Hz, 1H), 7,41-7,25 (m, 3H), 7,35 (d, *J* = 7,9 Hz, 1H), 7,11-7,03 (m, 3H), 6,85 (d, *J* = 7,9 Hz, 1H), 4,14 (br s, 1H), 4,15-4,02 (m, 1H), 3,99-3,87 (m, 1H), 3,16 (td, 7,2, 3,6 Hz, 2H), 2,93 (q, *J* = 7,2 Hz, 1H), 2,16 (s, 3H), 0,97 (d, *J* = 7,2 Hz, 3H). RMN ¹³C (75 MHz, CDCl₃, δ ppm) 212,8, 176,6, 143,3, 136,4, 130,1, 128,2, 127,6, 125,9, 123,0, 122,6, 122,3, 119,6, 118,6, 112,4, 111,6, 108,9, 50,2, 41,1, 31,5, 29,0, 23,2, 11,6. HRMS (ESI, m/z) calculé pour C₂₂H₂₂N₂O₃Na [M+Na]⁺ : 385,1528 déterminé 385,1525.

### 1-(2-(1H-indol-3-yl)ethyl)-3-hydroxy-3-(2-oxobutyl)indolin-2-one (APV-GG062)

Le composé APV-GG062 a été préparé suivant le mode opératoire général D en utilisant la 1-(2-(1H-indol-3-yl)ethyl)indoline-2,3-dione (145 mg, 0,5 mmol) et la butan-2-one (50 µL, 0,56 mmol). Le composé indiqué dans le titre a été obtenu, après chromatographie sur plaques préparatives (2 x 2 mm ; cyclohexane/EtOAc 50/50, double élution), sous forme d'un solide jaune clair (47 mg). Rdt = 26 %. RMN ¹H (300 MHz, CDCl₃, δ ppm) 8,06 (s, 1H), 7,48 (d, *J* = 7,7 Hz, 1H), 7,38-,27 (m, 3H), 7,17 (t, *J* = 7,4 Hz, 1H), 7,11-7,00 (m, 3H), 6,83 (d, *J* = 7,7 Hz, 1H), 4,28 (s, 1H), 4,12-3,88 (m, 2H), 3,22-3,11 (m, 2H), 2,88 (d, *J* = 16,7 Hz, 1H), 2,66 (d, *J* = 16,7 Hz, 1H), 2,32 (q, *J* = 7,2 Hz, 2H) 0,99 (t, *J* = 7,2 Hz, 3H). RMN ¹³C (75 MHz, CDCl₃, δ ppm) 210,8, 176,2, 143,1, 136,3, 130,1, 127,7, 124,1, 123,1, 122,8, 122,2, 119,5, 118,6, 112,4, 109,1, 74,5, 51,6, 47,3, 41,1, 37,6, 23,1, 11,6, 7,5. HRMS (ESI, m/z) calculé pour C₂₂H₂₂N₂O₃Na [M+Na]⁺ : 385,1528 déterminé 385,1522.

### 1-(2-(1H-indol-3-yl)ethyl)-3-hydroxy-3-(2-oxohexyl)indolin-2-one (APV-GG093)

Le composé APV-GG093 a été préparé suivant le mode opératoire général D en utilisant la 1-(2-(1H-indol-3-yl)ethyl)indoline-2,3-dione (145 mg, 0,5 mmol) et la hexan-2-one (70 µL, 0,57 mmol). Le composé indiqué dans le titre a été obtenu, après chromatographie sur plaques préparatives (2 x 2 mm ; cyclohexane/EtOAc 50/50), sous forme d'un solide beige (64 mg). Rdt = 33 %.
RMN ¹H (300 MHz, CDCl₃, δ ppm) 8,16 (s, 1H), 7,46 (d, *J* = 7,8 Hz, 1H), 7,37-7,27 (m, 2H), 7,30 (d, *J* = 7,9 Hz, 1H), 7,17 (t, *J* = 7,4 Hz, 1H), 7,09-7,00 (m, 3H), 6,83 (d, *J* = 7,8 Hz, 1H), 4,34 (br s, 1H), 4,11-3,88 (m, 2H), 3,22-3,11 (m, 2H), 2,90 (d, *J* = 16,8 Hz, 1H), 2,66 (d, *J* = 16,8 Hz, 1H), 2,28 (t, J = 7,3 Hz, 2H), 1,53-1,40 (m, 2H), 1,32-1,18 (m, 2H), 0,86 (t, J = 7,2 Hz, 3H). RMN ¹³C (75 MHz, CDCl₃, δ ppm) 210,6, 176,3, 143,1, 136,3, 130,1, 127,7, 124,1, 123,1, 122,8, 122,2, 119,5, 118,6, 112,4, 111,6, 109,0, 74,5, 47,5, 44,1, 41,2, 25,5, 23,1 (2C), 22,3, 14,0. HRMS (ESI, m/z) calculé pour C₂₄H₂₆N₂O₃Na [M+Na]⁺ : 413,1841 déterminé 413,1840.

### 1-(2-(1H-indol-3-yl)ethyl)-3-hydroxy-3-(3-methyl-2-oxobutyl)indolin-2-one (APV-GG090)

Le composé APV-GG090 a été préparé suivant le mode opératoire général D en utilisant la 1-(2-(1H-indol-3-yl)ethyl)indoline-2,3-dione (145 mg, 0,5 mmol) et la 3-methylbutan-2-one (60 µL, 0,56 mmol). Le composé indiqué dans le titre a été obtenu, après chromatographie éclair sur colonne de gel de silice (cyclohexane/EtOAc 70/30 à 60/40), sous forme d'un solide beige (100 mg). Rdt = 53 %.
RMN ¹H (300 MHz, CDCl₃, δ ppm) 8,05 (s, 1H), 7,5 (d, *J* = 7,9 Hz, 1H), 7,35 (d, *J* = 8,2 Hz, 1H), 7,31 (d, *J* = 4,8 Hz, 1H), 7,29 (d, *J* = 4,8 Hz, 1H), 7,18 (t, *J* = 7,4 Hz, 1H), 7,12-7,00 (m, 3H), 6,83 (d, *J* = 7,7 Hz, 1H), 4,38 (s, 1H), 4,17-3,90 (m, 2H), 3,17 (t, J = 7,2 Hz, 2H), 3,00 (d, *J=* 17 Hz, 1H), 2,71 (d, *J* = 17 Hz, 1H), 2,50-2,38 (m, 1H), 1,02 (dd, *J* = 6,9, 1,8 Hz, 6H). RMN ¹³C (75 MHz, CDCl₃, δ ppm) 214,2, 176,2, 143,1, 136,4, 130,1, 127,7, 124,1, 123,0, 122,7, 122,2, 119,6, 118,6, 112,5, 111,5, 109,0, 74,5, 45,5, 42,1, 41,1, 23,1, 17,9, 17,5. HRMS (ESI, m/z) calculé pour C₂₃H₂₄N₂O₃Na [M+Na]⁺ : 399,1685 déterminé 399,1687.

### 1-(2-(1H-indol-3-yl)ethyl)-3-hydroxy-3-(2-oxobutyl)indolin-2-one (APV-GG061)

Le composé APV-GG061 a été préparé suivant le mode opératoire général D en utilisant la 1-(2-(1H-indol-3-yl)ethyl)indoline-2,3-dione (145 mg, 0,5 mmol) et la 4-methylpentan-2-one (70 µL, 0,56 mmol). Le composé indiqué dans le titre a été obtenu, après chromatographie sur plaques préparatives (2 x 2 mm ; cyclohexane/EtOAc 50/50, double élution),sous forme d'un solide jaune (60 mg). Rdt = 32 %.
RMN ¹H (300 MHz, CDCl₃, δ ppm) 8,06 (s, 1H), 7,48 (d, *J* = 7,9 Hz, 1H), 7,37-7,27 (m, 3H), 7,17 (t, *J* = 7,3 Hz, 1H), 7,12-6,99 (m, 3H), 6,83 (d, *J* = 7,7 Hz, 1H), 4,28 (s, 1H), 4,12-3,88 (m, 2H), 3,17 (t, *J* = 7,2 Hz, 2H), 2,9 (d, *J* = 17 Hz, 1H), 2,65 (d, *J* = 17 Hz, 1H), 2,20-2,15 (m, 2H), 2,11-2,00 (m, 1H), 0,86 (d, *J* = 6,5 Hz, 6H). RMN ¹³C (75 MHz, CDCl₃, δ ppm) 210,1, 176,3, 143,1, 136,3, 130,1, 127,7, 124,1, 123,1, 122,8, 122,2, 119,5, 118,6, 112,4, 111,6, 109,0, 74,4, 70,7, 53,2, 48,1, 41,1, 24,5, 23,0, 22,6 (2C). HRMS (ESI, m/z) calculé pour C₂₄H₂₆N₂O₃Na [M+Na]⁺ : 413,1841 déterminé 413,1851.

### 1-(2-(1H-indol-3-yl)ethyl)-3-(2-cyclopropyl-2-oxoethyl)-3-hydroxyindolin-2-one (APV-GG072)

Le composé APV-GG072 a été préparé suivant le mode opératoire général D en utilisant la 1-(2-(1H-indol-3-yl)ethyl)indoline-2,3-dione (145 mg, 0,5 mmol) et la 1-cyclopropylethan-1-one (55 µL, 0,56 mmol). Le composé indiqué dans le titre a été obtenu, après chromatographie éclair sur colonne de gel de silice (cyclohexane/EtOAc 70/30 à 50/50), sous forme d'un solide beige (117 mg). Rdt = 62 %.
RMN ¹H (300 MHz, CDCl₃, δ ppm) 8,15 (s, 1H), 7,5 (d, *J* = 7,8 Hz, 1H), 7,33 (d, *J* = 7,6 Hz, 1H), 7,29 (d, *J* = 8,2 Hz, 1H), 7,27 (d, *J* = 7,6 Hz, 1H), 7,17 (t, *J* = 7,4 Hz, 1H), 7,10-6,99 (m, 3H), 6,83 (d, *J* = 7,8 Hz, 1H), 4,04 (s, 1H), 4,07-3,90 (m, 2H), 3,16 (t, J = 7,2 Hz, 2H), 3,11 (d, *J* = 16,8 Hz, 1H), 2,83 (d, *J* = 16,8 Hz, 1H), 1,84-1,74 (m, 1H), 1,08-0,98 (m, 2H), 0,92-0,83 (m, 2H). RMN ¹³C (75 MHz, CDCl₃, δ ppm) 210,0, 176,1, 143,0, 136,4, 130,0, 127,7, 124,3, 123,1, 122,7, 122,2, 119,6, 118,6, 112,4, 111,5, 109,0, 74,5, 60,6, 48,3, 41,1, 23,1, 22,1, 11,9, 11,8. HRMS (ESI, m/z) calculé pour C₂₃H₂₂N₂O₃Na [M+Na]⁺ : 397,1528 déterminé 397,1518.

### 1-(2-(1H-indol-3-yl)ethyl)-3-(2-cyclopentyl-2-oxoethyl)-3-hydroxyindolin-2-one (APV-GG089)

Le composé APV-GG089 a été préparé suivant le mode opératoire général D en utilisant la 1-(2-(1H-indol-3-yl)ethyl)indoline-2,3-dione (145 mg, 0,5 mmol) et la 1-cyclopentylethan-1-one (70 µL, 0,57 mmol). Le composé indiqué dans le titre a été obtenu, après chromatographie éclair sur colonne de gel de silice (cyclohexane/EtOAc 70/30 à 60/40), sous forme d'un solide jaune (130 mg). Rdt = 65 %.
RMN ¹H (300 MHz, CDCl₃, δ ppm) 8,04 (s, 1H), 7,51 (d, *J* = 7,8 Hz, 1H), 7,35 (d, *J* = 8,2 Hz, 1H), 7,32 (d, *J* = 7,8 Hz, 1H), 7,29 (d, *J* = 8,2 Hz, 1H), 7,18 (t, *J* = 7,6 Hz, 1H), 7,11-7,00 (m, 3H), 6,82 (d, *J* = 7,9 Hz, 1H), 4,41 (s, 1H), 4,11-3,90 (m, 2H), 3,17 (t, *J* = 7,4 Hz, 2H), 3,01 (d, *J* = 17,0 Hz, 1H), 2,74 (d, *J* = 17,0 Hz, 1H), 2,76-2,66 (m, 1H), 1,79-1,58 (m, 8H). RMN ¹³C (75 MHz, CDCl₃, δ ppm) 212,6, 176,2, 143,1, 136,4, 130,3, 130,0, 127,7, 124,1, 123,0, 122,7, 122,2, 119,6, 118,6, 112,5, 111,5, 109,0, 74,5, 52,6, 46,8, 41,1, 28,6, 28,5, 27,1, 26,1, 23,1. HRMS (ESI, m/z) calculé pour C₂₅H₂₆N₂O₃Na [M+Na]⁺ : 425,1841 déterminé 425,1845.

### 1-(2-(1H-indol-3-yl)ethyl)-3-(2-cyclohexyl-2-oxoethyl)-3-hydroxyindolin-2-one (APV-GG076)

Le composé APV-GG089 a été préparé suivant le mode opératoire général D en utilisant la 1-(2-(1H-indol-3-yl)ethyl)indoline-2,3-dione (145 mg, 0,5 mmol) et la 1-cyclohexylethan-1-one (80 µL, 0,58 mmol). Le composé indiqué dans le titre a été obtenu, après chromatographie éclair sur colonne de gel de silice (cyclohexane/EtOAc 70/30 à 60/40), sous forme d'un solide jaune (89 mg). Rdt = 43 %.
RMN ¹H (300 MHz, CDCl₃, δ ppm) 8,05 (s, 1H), 7,49 (d, *J* = 7,8 Hz, 1H), 7,35 (d, *J* = 8,2 Hz, 1H), 7,32 (d, *J* = 7,8 Hz, 1H), 7,3 (d, *J* = 7,7 Hz, 1H), 7,18 (t, *J* = 7,4 Hz, 1H), 7,12-6,99 (m, 3H), 6,83 (d, *J* = 7,8 Hz, 1H), 4,49 (s, 1H), 4,13-3,88 (m, 2H), 3,17 (t, *J* = 7,4 Hz, 2H), 2,97 (d, *J* = 17,0 Hz, 1H), 2,66 (d, *J* = 17,0 Hz, 1H), 2,25-2,14 (m, 1H), 1,80-1,68 (m, 4H), 1,32-1,10 (m, 4H). RMN ¹³C (75 MHz, CDCl₃, δ ppm) 213,9, 176,2, 143,1, 136,3, 130,0, 127,7, 124,1, 123,0, 122,8, 122,2, 119,5, 118,6, 112,5, 111,5, 109,0, 74,6, 51,8, 45,6, 41,1, 28,1, 28,0, 25,9, 25,7, 25,6, 26,1, 23,1. HRMS (ESI, m/z) calculé pour C₂₆H₂₈N₂O₃Na [M+Na]⁺ : 439,1998 déterminé 439,2002.

### 1-(2-(1H-indol-3-yl)ethyl)-3-hydroxy-3-((R)-2-oxocyclopentyl)indolin-2-one (APV-GG082)

Le composé APV-GG089 a été préparé suivant le mode opératoire général D en utilisant la 1-(2-(1H-indol-3-yl)ethyl)indoline-2,3-dione (145 mg, 0,5 mmol) et la cyclopentanone (50 µL, 0,57 mmol). Le composé indiqué dans le titre a été obtenu, après chromatographie éclair sur colonne de gel de silice (cyclohexane/EtOAc 70/30), sous forme d'un solide beige (35 mg). Rdt = 19 %.
RMN ¹H (300 MHz, CDCl₃, δ ppm) 8,01 (s, 1H), 7,65 (d, *J* = 7,7 Hz, 1H), 7,38-7,27 (m, 3H), 7,23-7,03 (m, 4H), 6,80 (d, *J* = 7,7 Hz, 1H), 5,63 (s, 1H), 4,13-4,01 (m, 1H), 3,98-3,86 (m, 1H), 3,20-3,10 (m, 2H), 2,72-2,62 (m, 1H), 2,35-2,25 (m, 1H), 1,86-1,73 (m, 1H), 1,72-1,59 (m, 4H).
RMN ¹³C (75 MHz, CDCl₃, δ ppm) 221,8, 176,4, 143,4, 136,4, 130,3, 128,8, 127,5, 124,2, 123,4, 122,6, 122,3, 119,7, 118,8, 112,2, 111,4, 108,9, 62,8, 51,8, 40,7, 39,8, 25,2, 23,4, 20,6.
HRMS (ESI, m/z) calculé pour C₂₃H₂₃N₂O₃Na [M+Na]⁺ : 397,1528 déterminé 397,1531.

### 1-(2-(1H-indol-3-yl)ethyl)-3-(2-(furan-2-yl)-2-oxoethyl)-3-hydroxyindolin-2-one (APV-GG087)

Le composé APV-GG089 a été préparé suivant le mode opératoire général D en utilisant la 1-(2-(1H-indol-3-yl)ethyl)indoline-2,3-dione (145 mg, 0,5 mmol) et la 1-(furan-2-yl)ethan-1-one (55 µL, 0,55 mmol). Le composé indiqué dans le titre a été obtenu, après chromatographie éclair sur colonne de gel de silice (cyclohexane/EtOAc 70/30 à 60/40), sous forme d'un solide orange clair (90 mg). Rdt = 45 %.
RMN ¹H (300 MHz, CDCl₃, δ ppm) 8,05 (s, 1H), 7,60-7,54 (m, 2H), 7,37 (t, *J* = 7,2 Hz, 2H), 7,30-7,06 (m, 6H), 7,03 (t, *J* = 7,5 Hz, 1H), 6,81 (d, *J* = 7,7 Hz, 1H), 6,52 (dd, *J* = 3,4, 1,5 Hz, 1H), 4,19 (s, 1H), 4,09-3,94 (m, 2H), 3,54 (d, *J* = 16,8 Hz, 1H), 3,19 (d, *J* = 16,8 Hz, 1H), 3,18 (t, *J* = 16,8 Hz, 2H). RMN ¹³C (75 MHz, CDCl₃, δ ppm) 186,9, 176,2, 152,4, 147,3, 143,2, 136,4, 130,1, 129,9, 127,6, 124,4, 123,1, 122,7, 122,2, 119,6, 118,6, 118,4, 112,8, 112,4, 111,6, 109,0, 74,6, 44,1, 41,1, 23,2. HRMS (ESI, m/z) calculé pour C₂₄H₂₀N₂O₄Na [M+Na]⁺ : 423,1321 déterminé 423,1318.

### 1-(2-(1H-indol-3-yl)ethyl)-3-(2-(benzofuran-2-yl)-2-oxoethyl)-3-hydroxyindolin-2-one (APV-GG084)

Le composé APV-GG084 a été préparé suivant le mode opératoire général D en utilisant la 1-(2-(1H-indol-3-yl)ethyl)indoline-2,3-dione (145 mg, 0,5 mmol) et la 1-(benzofuran-2-yl)ethan-1-one (88 mg, 0,55 mmol). Le composé indiqué dans le titre a été obtenu, après chromatographie éclair sur colonne de gel de silice (cyclohexane/EtOAc 80/20), sous forme d'un solide orange clair (90 mg). Rdt = 40 %.
RMN ¹H (300 MHz, CDCl₃, δ ppm) 8,03 (s, 1H), 7,69 (d, *J* = 8,0 Hz, 1H), 7,58-7,23 (m, 8H) 7,19 (t, *J* = 7,4 Hz, 1H), 7,13-7,06 (m, 2H), 7,01 (t, *J* = 7,4 Hz, 1H), 6,83 (d,
*J* = 7,8 Hz, 1H), 4,12-3,92 (m, 2H), 4,04 (s, 1H), 3,69 (d, *J* = 16,8 Hz, 1H), 3,31 (d, *J* = 16,8 Hz, 1H), 3,2 (t, *J* = 3,2 Hz, 2H), RMN ¹³C (75 MHz, CDCl₃, δ ppm) 188,8, 176,2, 156,0, 152,1, 143,2, 136,3, 130,2, 129,7, 129,0, 127,6, 127,1, 124,4, 124,3, 123,7, 123,1, 122,7, 122,2, 119,6, 118,6, 114,4, 112,7, 112,5, 111,6, 109,1, 74,6, 44,7, 41,2, 23,2. HRMS (ESI, m/z) calculé pour C₂₈H₂₂N₂O₄Na [M+Na]⁺ : 473,1477 déterminé 473,1482.

### 1-(2-(1H-indol-3-yl)ethyl)-3-hydroxy-3-(2-oxo-2-(thiophen-2-yl)ethyl)indolin-2-one (APV-GG088)

Le composé APV-GG088 a été préparé suivant le mode opératoire général D en utilisant la 1-(2-(1H-indol-3-yl)ethyl)indoline-2,3-dione (145 mg, 0,5 mmol) et la 1-(thiophen-2-yl)ethan-1-one (60 µL, 0,55 mmol). Le composé indiqué dans le titre a été obtenu, après chromatographie éclair sur colonne de gel de silice (cyclohexane/EtOAc 70/30 à 50/50), sous forme d'un solide orange clair (110 mg). Rdt = 53 %.
RMN ¹H (300 MHz, CDCl₃, δ ppm) 8,06 (s, 1H), 7,68 (d, J = 5,0 Hz, 1H), 7,60-7,53 (m, 2H), 7,41 (d, J = 7,6 Hz, 1H), 7,38 (d, J = 8,2 Hz, 1H), 7,33-7,25 (m, 1H), 7,20 (t, *J* = 7,5 Hz, 1H), 7,03 (t, *J* = 7,5 Hz, 1H), 7,15-7,07 (m, 2H), 7,03 (t, *J* = 7,5 Hz, 1H), 6,85 (d, *J* = 7,9 Hz, 1H), 4,26 (s, 1H), 4,15-3,94 (m, 2H), 3,57 (d, *J* = 16,5 Hz, 1H), 3,26 (d, *J* = 16,5 Hz, 1H), 3,20 (t, J = 7,4 Hz, 2H). RMN ¹³C (75 MHz, CDCl₃, δ ppm) 191,1, 176,8, 152,3, 143,8, 143,2, 136,3, 135,1, 133,4, 130,1, 128,5, 124,5, 123,1, 122,7, 122,3, 119,6, 118,7, 118,2, 112,5, 111,5, 109,1, 74,6, 44,9, 41,2, 23,2. HRMS (ESI, m/z) calculé pour C₂₄H₂₀N₂O₃SNa [M+Na]⁺ : 439,1092 déterminé 439,1085.

### 1-(2-(1H-indol-1-yl)ethyl)-3-(2-(2-chlorophenyl)-2-oxoethyl)-3-hydroxyindolin-2-one (APV-GG078)

Le composé APV-GG078 a été préparé suivant le mode opératoire général D en utilisant la 1-(2-(1H-indol-1-yl)ethyl)indoline-2,3-dione (220 mg, 0,76 mmol) et la 1-(2-chlorophenyl)ethan-1-one (110 µL, 0,85 mmol). Le composé indiqué dans le titre a été obtenu, après chromatographie éclair sur colonne de gel de silice (cyclohexane/EtOAc 70/30), sous forme d'un solide orange (130 mg). Rdt = 38 %. RMN ¹H (300 MHz, CDCl₃, δ ppm) 7,52 (d, *J* = 7,8 Hz, 1H), 7,42-7,27 (m, 5H), 7,23 (d, *J* = 8,2 Hz, 1H), 7,19-7,06 (m, 3H), 7,0 (q, *J* = 7,9 Hz, 2H), 6,39 (m, 2H), 4,54-4,40 (m, 2H), 4,22-4,11 (m, 1H), 4,06-3,94 (m, 1H), 3,85 (s, 1H), 3,56 (d, *J* = 17,3 Hz, 1H), 3,35 (d, *J* = 17,3 Hz, 1H). RMN ¹³C (75 MHz, CDCl₃, δ ppm) 201,3, 176,4, 142,7, 138,5, 136,1, 132,6, 131,4, 130,8, 130,3, 129,7, 129,0, 129,3, 128,3, 127,2, 124,3, 123,4, 121,8, 121,5, 119,8, 108,9, 108,4, 102,4, 74,4, 48,5, 43,7, 40,5. HRMS (ESI, m/z) calculé pour C₂₆H₂₁N₂O₃NaCl [M+Na]⁺ : 467,1138 déterminé 467,1142.

### 1-(4-(1H-indol-1-yl)butyl)-3-(2-(2-chlorophenyl)-2-oxoethyl)-3-hydroxyindolin-2-one (APV-GG079)

Le composé APV-GG079 a été préparé suivant le mode opératoire général D en utilisant la 1-(4-(1H-indol-1-yl)butyl)indoline-2,3-dione (100 mg, 0,31 mmol) et la 1-(2-chlorophenyl)ethan-1-one (45 µL, 0,35 mmol). Le composé indiqué dans le titre a été obtenu, après chromatographie éclair sur colonne de gel de silice (cyclohexane/EtOAc 60/40), sous forme d'un solide orange (45 mg). Rdt = 31 %. RMN ¹H (300 MHz, CDCl₃, δ ppm) 7,64 (d, *J* = 7,7 Hz, 1H), 7,43-7,24 (m, 7H), 7,22-7,14 (m, 1H), 7,12-7,03 (m, 3H) 6,71 (d, *J* = 7,9 Hz, 1H), 6,46 (d, *J* = 3,0 Hz, 1H), 4,17 (t, *J* = 6,8 Hz, 2H), 4,11 (s, 1H), 3,78 (d, *J* = 16,8 Hz, 1H), 3,74-3,62 (m, 2H), 3,51 (d, *J* = 16,8 Hz, 1H), 2,01-1,88 (m, 2H), 1,78-1,65 (m, 2H). RMN ¹³C (75 MHz, CDCl₃, δ ppm)201,2, 176,5, 143,1,138,6, 136,0, 132,7, 130,8,130,3, 129,8, 127,9, 127,3, 124,4, 123,2, 121,7, 121,2, 120,3, 119,6, 119,5, 109,8, 109,6, 109,0, 101,4, 74,7, 49,0, 45,9, 39,7, 27,6, 24,8. HRMS (ESI, m/z) calculé pour C₂₈H₂₅N₂O₃NaCl [M+Na]⁺ : 495,1451 déterminé 495,1453.

### Exemple 9 : Tests biologiques et méthodes

### Cultures cellulaires

Des rats Wistar femelles en gestation avec des embryons de 17 jours (E17) ont été obtenues de JANVIER LABS. Le jour de la réception des animaux, le rat est sacrifié et les embryons sont disséqués dans des conditions stériles pour enlever leur cerveau, qui est ensuite micro-disséqué dans GBSS (solution saline équilibrée de Gey) moyen pour obtenir la structure hippocampique des deux hémisphères, tout en rejetant les méninges. Les tissus prélevés d'au moins 5 embryons sont placés dans 2 ml de milieu DMEM (marque déposée) (milieu de Eagle modifié par Dulbecco - chez Life Technologies) avec 2% de sérum de veau foetal, 2 mM de glutamine, 0,1 mg / mL de mélange antibiotique pénicilline-streptomycine (Life Technologies) et soumise à une dissociation mécanique des cellules par passage à travers des pipettes en verre aux extrémités arrondies et de diamètre intérieur décroissant de 0,5 à 0,2 mm. Les cellules sont comptées et 100 µl de suspension contenant 25 x 103 cellules sont placés sur une lamelle de verre revêtue de polylysine (poly-D-lysine bromohydrate de 75 à 150 kDa, de Sigma) de Ø 12 mm, dans des plaques de culture de 12 puits et incubées pendant 2 heures à 37°C dans une atmosphère contenant 5% de CO₂ pour permettre l'adhésion cellulaire. Un volume de 2 mL de milieu de culture est ajouté doucement, Neurobasal (marque déposée) avec 1/50 (v: v) supplément sans sérum B-27® (Life Technologies), 2 mM de glutamine, 0,1 mg / ml de mélange antibiotique pénicilline-streptomycine (de Life Technologies) et on fait incuber encore dans les mêmes conditions. La moitié du milieu est remplacé deux fois par semaine pour l'entretien. Les cellules neuronales vont pouvoir se différencier pendant une période de 12 à 30 jours. Les cultures sont suivies par microscopie à contraste de phase: l'extension des neurites et des connexions synaptiques sont observées pour les neurones qui représentent la majorité des cellules, tandis que quelques astrocytes sont également présents.

### Electrophysiologie

### Système Patch-clamp

Le poste d'électrophysiologie est constitué par un microscope inversé à contraste de phase modèle Olympus IMT-2, placé sur une table anti-vibration TMC dans une cage de Faraday. L'installation comprend également un système de perfusion multicanal Warner modèle SF-77B, un système 3D-micromanipulateur modèle Burleigh PCS-5000 tenant la sonde de microélectrodes, un amplificateur de patch-clamp modèle Warner PC-505, et une interface analogique-numérique 8 canaux modèle PowerLab 8/35 de ADInstruments. La microélectrode de verre est fabriquée avec des tubes capillaires en borosilicate à paroi mince (1,5 OD x 1,17 ID x 100 L mm, de Harvard Apparatus) et préparée avec l'étireuse d'électrode verticale Narishige modèle PC10 pour obtenir une résistance électrique autour de 5 MOhm. L'électrode de la pipette est remplie avec le milieu intracellulaire composé de (en mM): 100 CsF, 40 CsCl, 1,2 CaCl₂, 20 HEPES, 10 EGTA, pH = 7,4 (ajusté avec du CsOH).

La culture sur sa lamelle est placée dans une boîte de pétri de 60 mm avec 2 ml de milieu extracellulaire composé de (en mM): 140 NaCl, 5 KCl, 1,2 CaCl2, 1,5 MgCl2, 12 HEPES, 12 Na-HEPES, 33 glucose, pH = 7,4.

Le neurone sélectionné est patché dans la configuration cellule entière (« whole cell configuration »). Le potentiel de maintien est fixé à -60 mV.

### Procédure d'application des composés test

Les microtubes de verre assemblés de section carrée du système de perfusion fournissent chacun un flux stable de composition différente. Le milieu par défaut « TTX » correspond au milieu extracellulaire auquel est ajouté 1 µM tétrodotoxine. Le milieu d'application « NMDA » est le « TTX » additionné de 100 µM de NMDA (N-méthyl-D-aspartate) comme agoniste, et 5 µM de L-glycine en tant que co-agoniste. Le milieu « NMDA + composé test » correspond au « NMDA » ajouté avec 10 µM du composé test. Le «lavage NMDA » est identique au milieu « NMDA ».

En règle générale, trois applications de chaque composé test sont effectuées sur la cellule patchée, pendant 2 secondes à des intervalles de 3 minutes (pour permettre l'inversion de désensibilisation). La séquence des applications est: 1) « NMDA », 2) « NMDA + composé test », 3) «lavage NMDA ». Le milieu « TTX » est appliqué par défaut, avant et après chaque application de composé test.

Dans la plupart des cellules, une courte équilibration en montée (run-up) du courant NMDA se produit. Aussi, la première application NMDA considérée est celle après cette équilibration, habituellement de 3 à 6 min. Certaines cellules montrant un run-down de longue durée du courant NMDA après cette courte période qui a précédé, sont donc éliminées.

UBP141 est obtenu de ABcam, DQP 1105 de Tocris et la tétrodotoxine (libre de citrate) de Latoxan. Tous les autres produits chimiques commerciaux sont de Sigma-Aldrich.

### Analyses des données

Les données électrophysiologiques sont numériquement collectées par le logiciel LabChart Pro 8 et analysées par le module d'analyse de pic (ADInstruments). Les paramètres de pic mesurés sont les suivants:

| **Propriété de transmission NMDA** | **Paramètre** | **Unité** | **Signification physiologique** |
|---|---|---|---|
| Courant NMDA | **Hauteur** | pA | Courant maximum |
| | **Largeur50** | ms | Durée du courant au-dessus de 50 % de Hauteur |
| | **SurfacePic** | pA.s | Courant total intégré dans le temps |
| Cinétique d'ouverture des canaux | **TempsAuPic** | ms | Temps pour atteindre le courant maximum |
| | **TMontée** | ms | Temps d'ouverture entre 10 et 90 % du courant max. |
| | **PenteMontée** | pA/s | Vitesse moyenne d'ouverture pendant TMontée |
| | **PenteMin** | pA/s | Vitesse d'ouverture la plus grande |
| Cinétique de fermeture des canaux | **TChute** | ms | Temps de fermeture entre 90 et 10 % du courant max |
| | **PenteChute** | pA/s | Vitesse moyenne de fermeture pendant TChute |
| | **PenteMax** | pA/s | Vitesse de fermeture la plus grande |
| | **Tau** | s | Temps de fermeture de 50% des canaux (demi-vie) |
| Désensibilisation | **Pente** | pA/s | Vitesse de désensibilisation pendant l'application de NMDA |

### Validation de la méthode avec des actifs de référence

Les deux composés référence UBP141 et DQP1105 sont testés en premier pour valider la possibilité technique de montrer un effet antagoniste sur les courants NMDA totaux des neurones embryonnaires des rats dans les cultures. À une concentration de 10 µM, les deux références montrent un effet antagoniste de plus de -20 % de l'amplitude de courant maximale. À une concentration inférieure à 1 µM, l'effet antagoniste est maintenu au même niveau tout au moins pour DQP 1105. Les molécules références présentent également des modifications dans la cinétique d'ouverture et de fermeture du canal NMDA, ainsi que sur la désensibilisation. Le tableau 3 montre l'effet de la référence, le DQP1105. Une série de 12 paramètres de pic sont choisis par rapport à l'amplitude du courant et différentes propriétés cinétiques du canal NMDA, comme mentionné ci-dessus. Ces paramètres représentent un profil d'effets sur la neurotransmission NMDA.

### Procédures statistiques

Pour chaque cellule testée, la moyenne de la série de paramètres de pic de courant, comme mentionné ci-dessus, est calculée pour les applications médicamenteuses répétées (typiquement n = 3) dans chaque condition à savoir : « NMDA », « NMDA + composé test » et « lavage NMDA ». L'effet du composé est évalué sur plusieurs neurones (n = 6 à 16) avec un t-test apparié unilatéral, en comparant les conditions de «NMDA + composé test» aux conditions du «NMDA ». La persistance de l'effet composé après le retrait est évaluée avec un t-test apparié unilatéral, en comparant les conditions de «lavage NMDA» à «NMDA + composé test ». La signification statistique est atteinte pour des valeurs p < 0,05. Toutes les analyses statistiques sont effectuées avec le logiciel JMP 12 (SAS Institute Inc.).

Le tableau 2 ci-dessous résume les formules des composés utilisés et l'indicatif utilisé dans le tableau de résultats :

| **Molécule** | **Formule** |
|---|---|
| 1 | 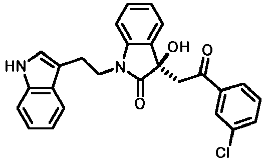 |
| 2 | 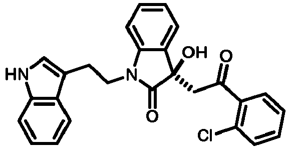 |
| 3 | 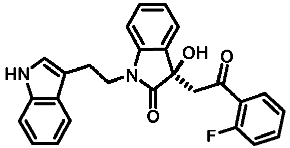 |

Le tableau 3 ci-dessous résume les résultats obtenus in-vitro sur les courants NMDA, la cinétique d'ouverture, de fermeture et la désensibilisation éventuelle des canaux, avec des exemples de composés selon l'invention

| A. Courant NMDA | | | | | |
|---|---|---|---|---|---|
| **Molécule** | **Hauteur (pA)** | **Largeur50 (ms)** | **SurfacePic (pA.s)** | **Lavable** | **Résumé** |
| 1 | -43 ± 8% (7)** | 0 ± 9% (7) | -37 ± 12% (7)* | Non | Antagoniste (4+) |
| 2 | -66 ± 10% (7)*** | -49 ± 14% (7)** | -65 ± 13% (7)** | Oui | Antagoniste (6+) |
| 3 | -24 ± 7% (13)** | -10 ± 7% (13) | -16 ± 8% (13)* | (Non/Oui ) | Antagoniste (2+) |
| | | | | | |

| B. Cinétique d'ouverture des canaux | | | | | |
|---|---|---|---|---|---|
| **Molécule** | **TempsAuPic (ms)** | **TMontée (ms)** | **PenteMontée (pA/s)** | **PenteMin (pA/s)** | **Lavable** |
| 1 | 1 ± 4% (7) | 3 ± 3% (7) | -45 ± 7% (7)*** | -35 ± 12% (7)* | Non |
| 2 | -7 ± 4% (7) | -16 ± 13% (7) | -61 ± 10% (7)*** | -25 ± 9% (7)* | (Oui/Non ) |
| 3 | -5 ± 3% (13)* | -8 ± 6% (13) | -23 ± 5% (13)*** | -22 ± 4 (13)**** | (Oui/Non ) |
| | | | | | |

| C. Cinétique de fer meture des canaux | | | | | |
|---|---|---|---|---|---|
| **Molécule** | **TChute (ms)** | **PenteChute (pA/s)** | **Pente Max (pA/s)** | **Tau (s)** | **Lavable** |
| 1 | 12 ± 6% (7) | -48 ± 8% (7)*** | -27 ± 10% (7)* | 29 ± 13% (6)* | Non |
| 2 | -15 ± 5% (7)* | -63 ± 13% (7)** | -28 ± 8% (7)** | 44 ± 28% (7) | (Oui/Non ) |
| 3 | 2 ± 4% (13) | -30 ± 8% (13)** | -22 ± 4% (13)**** | 17 ±5 (13)** | Non |
| | | | | | |

| D. Désensibilisation | | | | | |
|---|---|---|---|---|---|
| **Molécule** | | **Pente (pA/s)** | | | **Lavable** |
| 1 | | -59 ± 8% (7)*** | | | Non |
| 2 | | -70 ± 17% (7)** | | | (Oui) |
| 3 | | -43 ± 8% (13)**** | | | Non |

Dans le tableau ci-dessus les éléments suivants signifient :
entre () : Nombre de cellules, (pas d'étoile) NS, * p<0,05, ** p<0,01, ***p<0,001, ****p<0,0001 et NA : Non applicable.

Cet exemple démontre donc clairement que des exemples de composés selon l'invention sont des antagonistes des récepteurs NMDA qui peuvent être utiles dans la prévenir ou à traiter des pathologies impliquant les récepteurs NMDA du système nerveux central, en particulier l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure.

### Liste de référence

1. Chen, H.-S. V.; Lipton, A. S. The chemical biology of clinically tolerated NMDA receptor antagonists. J. Neurochem. 2006, 97, 1611-1626.
2. Hallett, P. J.; Standaert, D. G. Rationale for and use of NMDA receptor antagonists in Parkinson's disease. Pharmacol. Ther. 2004, 102, 155-174.
3. Vance, K. M.; N., S.; Traynelis, S. F.; Furukawa, H. Ligand specific deactivation time course of GluN1/GluN2D NMDA receptors. Nat. Commun. 2011, 2, 294.
4. Goff, D. C.; Cather, C.; Gottlieb, J. D.; Evins, A. E.; Walsh, J.; Raeke, L.; Otto, M. W.; Schoenfeld, D.; Green, M. F. Once-weekly Dcycloserine effects on negative symptoms and cognition in schizophrenia: an exploratory study. Schizophr. Res. 2008, 106, 320-327).
5. approach for the synthesis of indole-3-propanol and its acetates from dihydropyran" Monatsh Chem. 2008, 139, 1475-1478.
6. Campos, K. R.; Woo, J. C. S.; Lee, S.; Tillyer, R. D. "A General Synthesis of Substituted Indoles from Cyclic Enol Ethers and Enol Lactones" Org. Lett. 2004, 6, 79-82.
7. Yang, J.-M.; Li, P.-H.; Wei, Y.; Tang, X.-Y.; Shi, M. « Gold(I)-catalyzed highly stereoselective synthesis of polycyclic indolines: the construction of four contiguous stereocenters » Chem. Commun. 2016, 52, 346-349
8. Kounosuke, O.; Abe, J.; Kanai, M. « Manganese-catalyzed aerobic dehydrogenative cyclization toward ring-fused indole skeletons » Org. Biomol. Chem. 2013, 11, 4569-4572.
9. Campaigne, E.; Homfeld, E. « Benzo[b]thiophene derivatives. XXV.Condensation and reductive alkylation of 3-aminoalkylbenzo[b]thiophenes with formaldehyde » J. Heterocycl. Chem. 1979, 16, 1321-1324.
10. Zhang , H. C.; Ye, H.; Moretto , A. F.; Brumfield, K. K.; Maryanoff, B. E. « Facile Solid-Phase Construction of Indole Derivatives Based on a Traceless, Activating Sulfonyl Linker » Org. Lett. 2000, 2, 89-92.
11. Lee, S. S.; Shen, W.; Zheng, X.; Jacobsen, I. C. « Preparation of tetrahydropyridinylthiophenecarboxylic acid derivatives for use as hepatitis c virus polymerase inhibitors »WO2014055142 A1.
12. Caroff, E.; Keller, M.; Kimmerlin, T.; Meyer, E.; « Preparation of 4-(benzoimidazol-2-yl)thiazole compounds and related aza derivatives as modulators of the CXCR3 receptor » WO2013114332 A1.
13. Kruegel, A. C.; Rakshit, S.; Li, X.; Sames, D.; « Constructing Iboga Alkaloids via C-H Bond Functionalization: Examination of the Direct and Catalytic Union of Heteroarenes and Isoquinuclidine Alkenes » J. Org. Chem. 2015, 80, 2062-2071.
14. Van Epps, D. E.; Jiang, G.-L.; Collette, A. L.; Horan, R.L.; Chen, J. S.; Altman, G. H.; Im, W.-B. « Compositions and improved soft tissue replacement methods » WO2013123272 A1.
15. Contour-Galcéra, M.-O. ; Sidhu, A. ; Plas, P. ; Roubert, P. "3-Thio-1,2,4-triazoles, novel somatostatin sst2/sst5 agonists" Bioorg. Med. Chem. Lett. 2005, 15, 3555-3559.
16. Rong, Z.; Wang, W.; Jiang, Z.j.; Wang, K.; Zheng, X.-I.; Fu, H.-y.; Chen, H.; Li, R.-x. « One-pot synthesis of 2-substituted benzo[b]furans via Pd-tetraphosphinecatalyzed coupling of 2-halophenols with alkynes » Chem. Commun. 2014, 50, 6023-6026.
17. Banerjee, T. S.; Paul, S.; Sinha, S.; Das, S. « Synthesis of iboga-like isoquinuclidines: Dual opioid receptors agonists having antinociceptive properties » Bioorg. Med. Chem. 2014, 22, 6062-6070.
18. Kruegel, A. C.; Rakshit, S.; Li, X.; Sames, D.; « Constructing Iboga Alkaloids via C-H Bond Functionalization: Examination of the Direct and Catalytic Union of Heteroarenes and Isoquinuclidine Alkenes » J. Org. Chem. 2015, 80, 2062-2071.
19. Contour-Galcéra, M.-O. ; Sidhu, A. ; Plas, P. ; Roubert, P. "3-Thio-1,2,4-triazoles, novel somatostatin sst2/sst5 agonists" Bioorg. Med. Chem. Lett. 2005, 15, 3555-3559.
20. S. M. Berge et al., J. Pharmaceutical Sciences, 1977, 66, 1-19
21. Remington Pharmaceutical Sciences, seizième édition, E. W. Martin (Mack Publishing Co., Easton, Pa., 1980)

## Revendications

1. Composé de formule **(I)** : 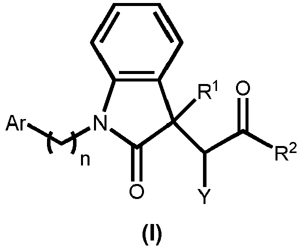 ou un sel pharmaceutiquement acceptable de celui-ci;
dans laquelle :
**R¹** représente H ou OH ;
**R²** représente un radical C₁₋₆alkyle linéaire, ramifié ou cyclique ; C₆₋₁₀aryle éventuellement substitué par un ou plusieurs substituants choisis parmi un atome d'halogène, un radical C₁₋₆alkyle linéaire, ramifié ou cyclique, un radical C₁₋₆alkoxyle linéaire, ramifié ou cyclique, un radical C₁₋₆haloalkyle linéaire, ramifié ou cyclique, un radical C₁₋₆haloalkoxyle linéaire, ramifié ou cyclique ; un radical C₆₋₁₀hétéroaryle éventuellement substitué par un ou plusieurs substituants choisis parmi un atome d'halogène, un radical C₁₋₆alkyle linéaire, ramifié ou cyclique, un radical C₁₋₆alkoxyle linéaire, ramifié ou cyclique, un radical C₁₋₆haloalkyle linéaire, ramifié ou cyclique, un radical C₁₋₆haloalkoxyle linéaire, ramifié ou cyclique ; ou un radical C₂₋₅hétérocycle, de préférence C₄₋₅hétérocycle, saturé ou insaturé; de préférence insaturé, comprenant de 1 à 3 hétéroatome(s) choisi(s) dans le groupe comprenant O, N et S,
**Y** représente H ou un radical C₁₋₆alkyle linéaire, ramifié ou cyclique ; ou bien Y pris ensemble avec R² forme un cycle à 5 ou 6 chainons ;
**n** représente 0, 1, 2, 3 ou 4 ;
**Ar** représente un hétérocyle bicyclique de structure : 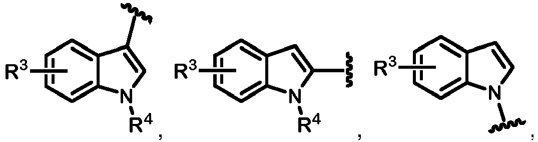 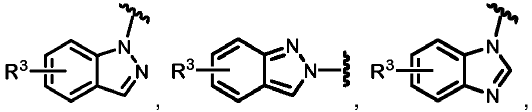 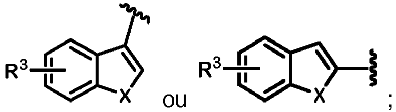 dans laquelle :
**R³** représente H, un atome d'halogène, un radical -OR⁵ ou -NR^{6A}R^{6B} ; dans lequel R⁵ représente un radical H, C₁₋₆alkyle linéaire, ramifié ou cyclique ; et R^{6A} et R^{6B} représentent indépendamment H, C₁₋₆alkyle linéaire, ramifié ou cyclique ou C₆₋₁₀aryle ;
**R⁴** représente H ; un radical C₁₋₆alkyle linéaire, ramifié ou cyclique ; ou un radical -C(=O)R⁷ dans lequel R⁷ représente un radical C₁₋₆alkyle linéaire, ramifié ou cyclique ; et
**X** représente O ou S.

2. Composé selon la revendication 1, ayant la configuration suivante:

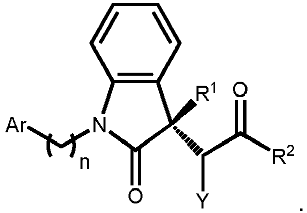

3. Composé selon la revendication 1 ou 2, dans lequel n représente 2 ou 3.

4. Composé selon l'une quelconque des revendications 1 à 3, dans lequel R¹ représente OH.

5. Composé selon l'une quelconque des revendications 1 à 4, dans lequel Y représente H, méthyle ou bien Y pris ensemble avec R² forme un cycle à 5 chainons ; de préférence Y représente H.

6. Composé selon l'une quelconque des revendications 1 à 4, dans lequel lorsque **Y** pris ensemble avec R² forme un cycle à 5 ou 6 chainons ; le carbone en alpha de la fonction cétone qui porte Y est un centre de chiralité de configuration R ou S, ou bien racémique.

7. Composé selon l'une quelconque des revendications 1 à 6, dans lequel Ar répond à la formule suivante : 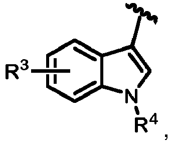 dans laquelle
R³ représente H ; un atome d'halogène choisi parmi F, Cl ou Br, de préférence Cl; un radical -OR⁵ où R⁵ représente H, méthyle ou éthyle ; ou un radical -NR^{6A}R^{6B} où R^{6A} et R^{6B} représentent indépendamment H, méthyle ou éthyle; et
R⁴ représente H ou méthyle, ou 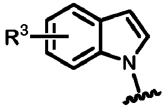 dans laquelle
R³ représente H ; un atome d'halogène choisi parmi F, Cl ou Br, de préférence Cl; un radical -OR⁵ où R⁵ représente H, méthyle ou éthyle ; ou un radical -NR^{6A}R^{6B} où R^{6A} et R^{6B} représentent indépendamment H, méthyle ou éthyle.

8. Composé selon la revendication 7, dans lequel Ar répond à la formule suivante : 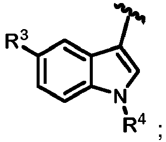 dans laquelle R³ et R⁴ sont tels que définis à la revendication 7.

9. Composé selon la revendication 7, dans lequel Ar répond à la formule suivante : 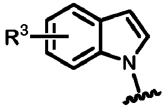 dans laquelle R³ représente H.

10. Composé selon l'une quelconque des revendications 1 à 9 dans lequel R² représente :
- méthyle, éthyle, propyle, n-butyle, iso-propyle, iso-butyle, cyclopropyle, cyclobutyle, cyclopentyle ou cyclohexyle ;
- un radical phényle éventuellement substitué par un ou plusieurs substituants choisis parmi F, Cl, Br, méthyle, éthyle, CN, CF₃, OH, méthoxy, NH₂ ou OCF₃;
- un radical pyridyle éventuellement substitué par un ou plusieurs substituants choisis parmi F, Cl, Br, méthyle, éthyle, CF₃, OH, méthoxy, NH₂ ou OCF₃; ou
- un radical benzofuryle de structure 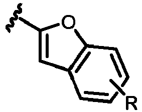 éventuellement substitué par un ou plusieurs substituants R choisis parmi F, Cl, Br, méthyle, éthyle, CN, CF₃, OH, méthoxy, NH₂ ou OCF₃.
- un radical furyle de structure 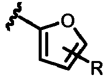 éventuellement substitué par un ou plusieurs substituants R choisis parmi F, Cl, Br, méthyle, éthyle, CN, CF₃, OH, méthoxy, NH₂ ou OCF₃.
- un radical thiophene de structure 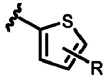 éventuellement substitué par un ou plusieurs substituants R choisis parmi F, Cl, Br, méthyle, éthyle, CN, CF₃, OH, méthoxy, NH₂ ou OCF₃.
- un radical benzofuryle de structure 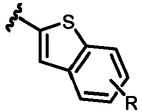 éventuellement substitué par un ou plusieurs substituants R choisis parmi F, Cl, Br, méthyle, éthyle, CN, CF₃, OH, méthoxy, NH₂ ou OCF₃.

11. Composé selon l'une quelconque des revendications 1 à 9 dans lequel R² représente :
- méthyle, éthyle, iso-propyle, iso-butyle, cyclopropyle, ou cyclohexyle ;
- un radical phényle non substitué ou mono-substitué en ortho, méta ou para par un substituant choisi parmi F, Cl, Br, méthyle, éthyle, CN, CF₃, OH, méthoxy, NH₂ ou OCF₃; ou
- un radical pyridyle 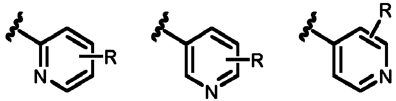 éventuellement substitué par un substituant R choisi parmi H, F, Cl, Br, méthyle, éthyle, CF₃, OH, méthoxy, NH₂ ou OCF₃, de préférence H ; ou
- un radical benzofuryle de structure 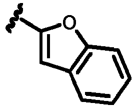
- un radical furyl de structure

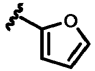

12. Composé selon l'une quelconque des revendications 1 à 10 répondant à l'une des structures suivantes:

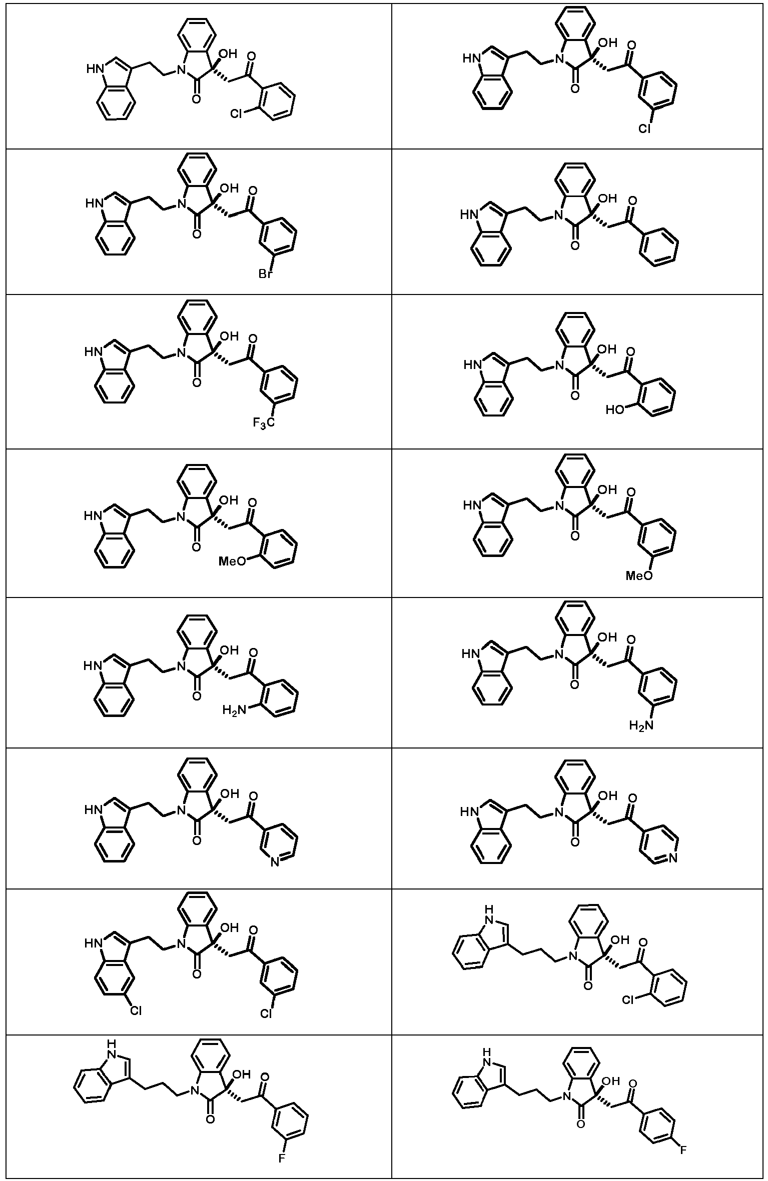

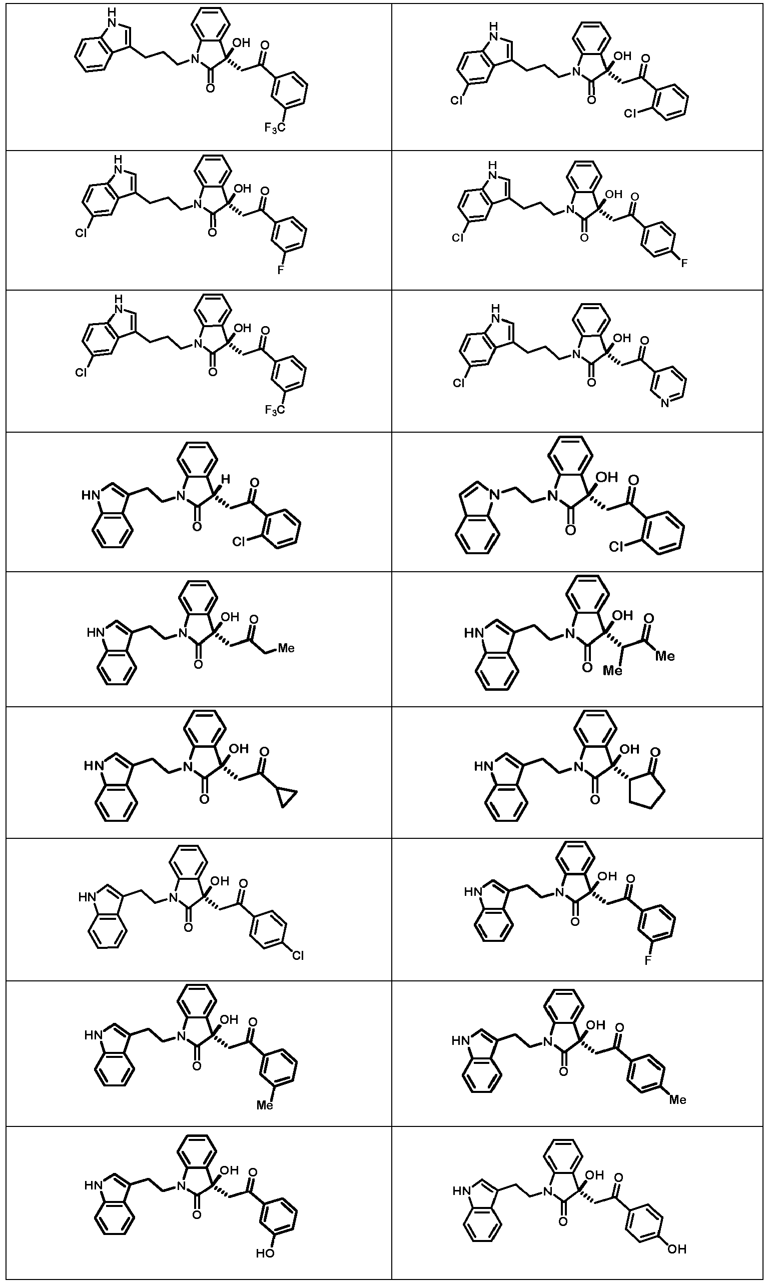

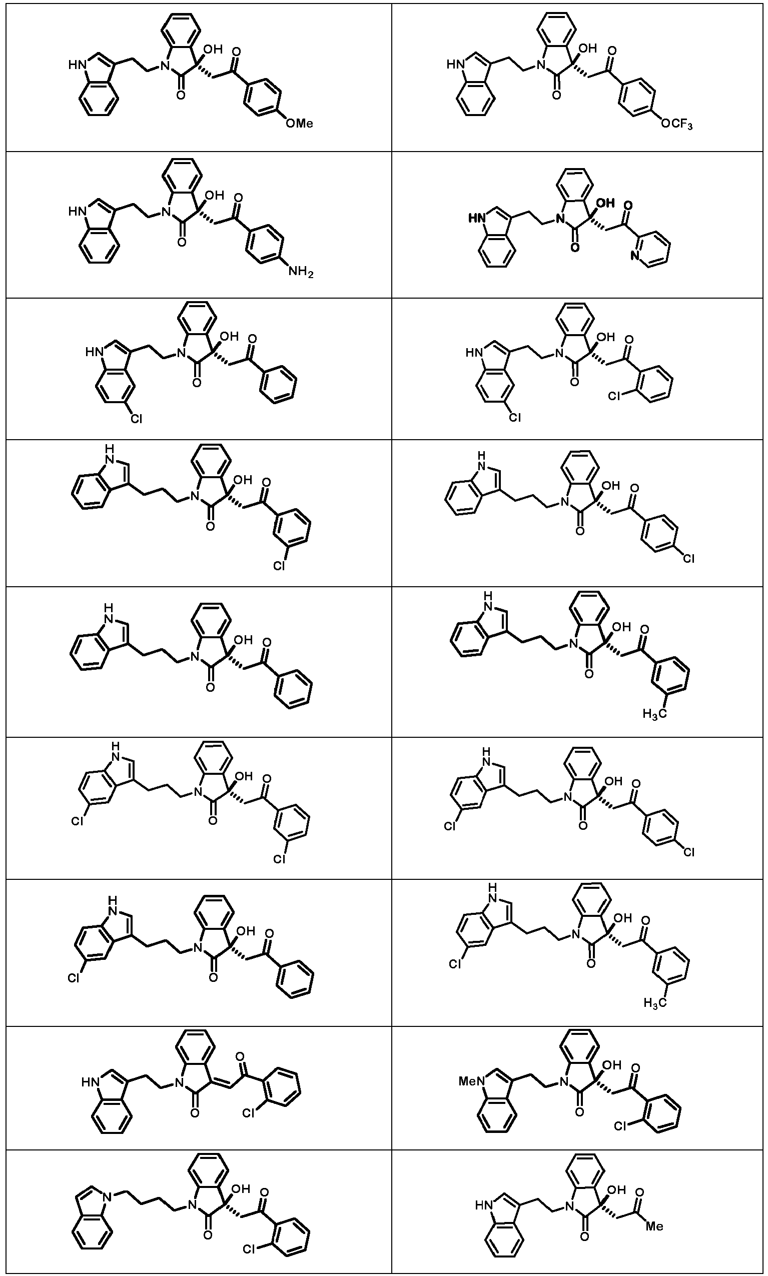

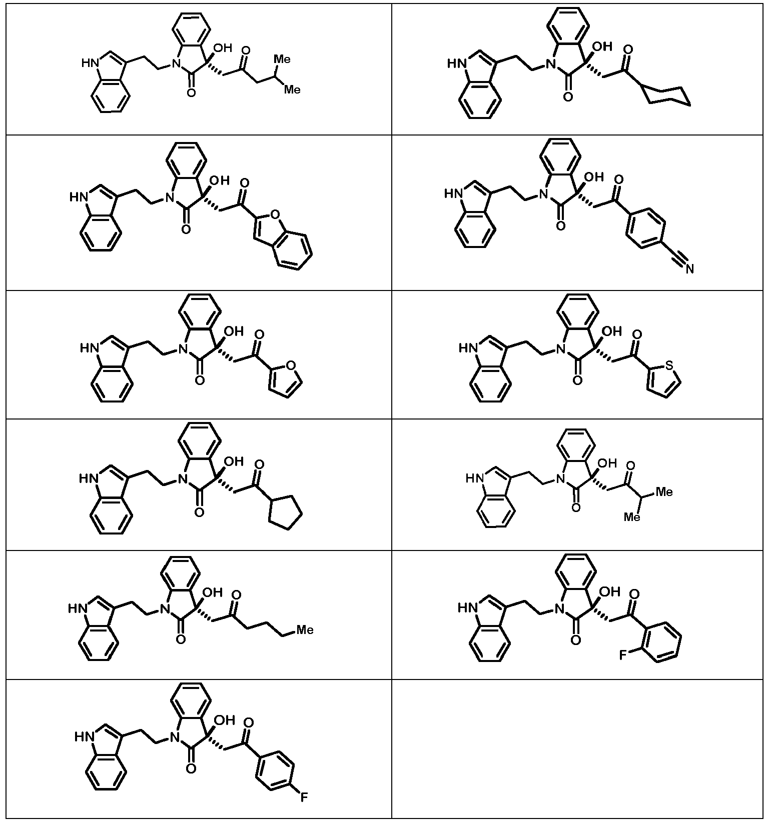

13. Composition pharmaceutique comprenant un composé selon l'une quelconque des revendications 1 à 11 et un véhicule pharmaceutiquement acceptable.

14. Composé selon l'une quelconque des revendications 1 à 11, pour son utilisation comme médicament.

15. Composé pour son utilisation selon la revendication 13 dans le traitement de maladies ou pathologies impliquant un récepteur N-Méthyl-D-Aspartate du système nerveux central, telles que l'épilepsie grave/résistante et les troubles cognitifs qui en résultent, notamment l'autisme, mais aussi les accidents vasculaires cérébraux, la schizophrénie, les maladies dégénératives impliquant l'activation des récepteurs NMDA tels que les maladies de Parkinson et d'Alzheimer, le syndrome de Rett ou la sclérose latérale amyotrophique, la migraine, la démence et la dépression majeure.

## Patentansprüche

1. Eine Verbindung der Formel (I): 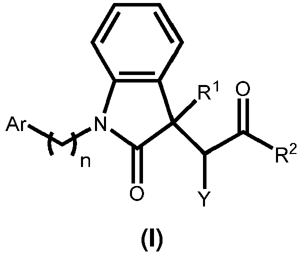 oder ein pharmazeutisch akzeptables Salz davon;
wobei :
**R1** stellt H oder OH dar;
**R2** einen linearen, verzweigten oder cyclischen C₁₋₆-Alkylrest; C₆₋₁₀-Aryl, gegebenenfalls substituiert durch einen oder mehrere Substituenten, ausgewählt aus einem Halogenatom, einem linearen, verzweigten oder cyclischen C₁₋₆-Alkylrest, einem linearen, verzweigten oder cyclischen C₁₋₆-Alkoxylrest, einem linearen, verzweigten oder cyclischen C₁₋₆-Halogenalkylrest, einem linearen, verzweigten oder cyclischen C₁₋₆-Halogenalkoxylrest; einen Rest C₆₋₁₀-Heteroaryl, der gegebenenfalls durch einen oder mehrere Substituenten substituiert ist, ausgewählt aus einem Halogenatom, einem linearen, verzweigten oder cyclischen C₁₋₆-Alkylrest, einem linearen, verzweigten oder cyclischen C₁₋₆-Alkoxylrest, einem linearen, verzweigten oder cyclischen oder einem C₂₋₅-Heterocyclus-Rest, vorzugsweise einem C₄₋₅-Heterocyclus, gesättigt oder ungesättigt; vorzugsweise ungesättigt, umfassend 1 bis 3 Heteroatom(e), ausgewählt aus der Gruppe bestehend aus O, N und S;
**Y** H oder einen linearen, verzweigten oder cyclischen C₁₋₆-Alkylrest darstellt; oder Y zusammen mit R2 einen 5- oder 6-gliedrigen Ring bildet;
**n** 0, 1, 2, 3 oder 4 ist;
Ar einen bicyclischen Heterocyclus der Struktur darstellt: 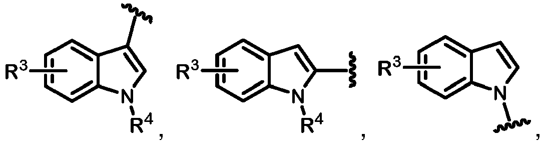 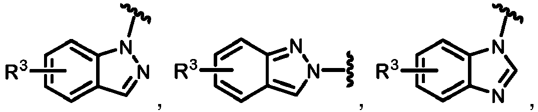 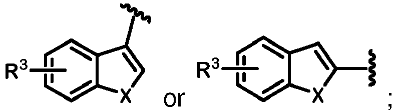
worin:
**R₃** H, Halogen, -OR⁵ oder -NR6AR6B darstellt; wobei R⁵ H, lineares, verzweigtes oder cyclisches C₁₋₆-Alkyl darstellt; und R^{6A} und R^{6B} unabhängig voneinander H, lineares, verzweigtes oder cyclisches C₁₋₆-Alkyl oder C6-10-Aryl darstellen;
**R₄** H, lineares, verzweigtes oder cyclisches C₁₋₆-Alkyl oder -C(=O)R⁷ ist, wobei R⁷ lineares, verzweigtes oder cyclisches C₁₋₆-Alkyl ist; und
X O oder S ist.

2. Verbindung nach Anspruch 1, die die folgende Konfiguration aufweist:

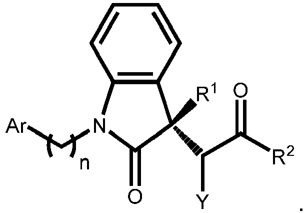

3. Die Verbindung nach Anspruch 1 oder 2, wobei n 2 oder 3 ist.

4. Die Verbindung nach einem der Ansprüche 1 bis 3, worin R¹ für OH.

5. Verbindung nach einem der Ansprüche 1 bis 4, wobei Y für H, Methyl steht oder Y zusammen mit R² einen 5-gliedrigen Ring bildet; vorzugsweise steht Y für H.

6. Verbindung nach einem der Ansprüche 1 bis 4, wobei, wenn Y zusammen mit R² einen 5- oder 6-gliedrigen Ring bildet; das Alpha-Kohlenstoffatom der Ketonfunktion, das Y trägt, ein Chiralitätszentrum der R- oder S-Konfiguration ist, oder racemisch.

7. Die Verbindung nach einem der Ansprüche 1 bis 6, wobei Ar die folgende Formel hat: 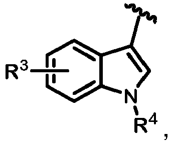 worin
R³ H; ein Halogenatom, ausgewählt aus F, Cl oder Br, vorzugsweise Cl; einen Rest -OR⁵, worin R⁵ H, Methyl oder Ethyl bedeutet; oder einen Rest - NR⁶AR^{6B}, worin R^{6A} und R^{6B} unabhängig voneinander H, Methyl oder Ethyl bedeuten; und
R⁴ H oder Methyl ist, oder 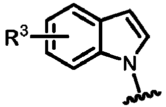 in der
R³ H; ein Halogenatom, ausgewählt aus F, Cl oder Br, vorzugsweise Cl; einen Rest -OR5, worin R⁵ H, Methyl oder Ethyl darstellt; oder einen Rest - N^{R6}AR^{6B}, worin R6A und R^{6B} unabhängig voneinander H, Methyl oder Ethyl darstellen.

8. Die Verbindung nach Anspruch 7, wobei Ar die folgende Formel hat: 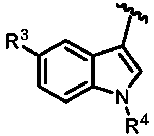 worin R³ und R⁴ wie in Anspruch 7 definiert sind.

9. Die Verbindung nach Anspruch 7, wobei Ar die folgende Formel hat: 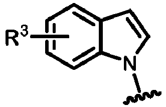 worin R³ für H steht.

10. Verbindung nach einem der Ansprüche 1 bis 9, worin R2 darstellt:
- Methyl, Ethyl, Propyl, n-Butyl, iso-Propyl, iso-Butyl, Cyclopropyl, Cyclobutyl, Cyclopentyl oder Cyclohexyl;
- einen Phenylrest, gegebenenfalls substituiert durch einen oder mehrere Substituenten, ausgewählt aus F, Cl, Br, Methyl, Ethyl, CN, CF₃, OH, Methoxy, NH₂ oder OCF₃;
- einen Pyridylrest, der gegebenenfalls mit einem oder mehreren Substituenten, ausgewählt aus F, Cl, Br, Methyl, Ethyl, CF₃, OH, Methoxy, NH₂ oder OCF₃, substituiert ist; oder
- einen Benzofurylrest der Struktur 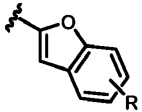 gegebenenfalls substituiert durch einen oder mehrere Substituenten R, ausgewählt aus F, Cl, Br, Methyl, Ethyl, CN, CF₃, OH, Methoxy, NH₂ oder OCF₃;
- ein Furylradikal der Struktur 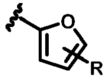
- gegebenenfalls substituiert durch einen oder mehrere Substituenten R, ausgewählt aus F, Cl, Br, Methyl, Ethyl, CN, CF₃, OH, Methoxy, NH₂ oder OCF₃;
- a Thiophenrest der Struktur 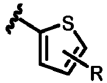 gegebenenfalls substituiert durch einen oder mehrere Substituenten R, ausgewählt aus F, Cl, Br, Methyl, Ethyl, CN, CF₃, OH, Methoxy, NH₂ oder OCF₃;
- einen Benzofurylrest der Struktur 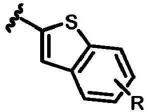 gegebenenfalls substituiert durch einen oder mehrere Substituenten R, ausgewählt aus F, Cl, Br, Methyl, Ethyl, CN, CF₃, OH, Methoxy, NH₂ oder OCF₃.

11. Die Verbindung nach einem der Ansprüche 1-9, worin R² darstellt:
- Methyl, Ethyl, iso-Propyl, iso-Butyl, Cyclopropyl oder Cyclohexyl;
- einen Phenylrest, der unsubstituiert oder in ortho-, meta- oder para-Stellung durch einen Substituenten ausgewählt aus F, Cl, Br, Methyl, Ethyl, CN, CF₃, OH, Methoxy, NH₂ oder OCF₃ monosubstituiert ist; oder
- einer Pyridyl-Radikalitätl 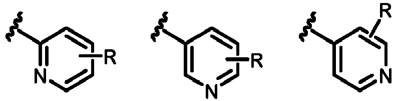 gegebenenfalls substituiert durch einen oder mehrere Substituenten R, ausgewählt aus F, Cl, Br, Methyl, Ethyl, CF₃, OH, Methoxy, NH₂ oder OCF₃, vorzugsweise H; oder;
- einen Benzofurylrest der Struktur 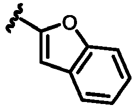
- einen Furylrest der Struktur

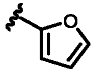

12. Die Verbindung nach einem der Ansprüche 1 bis 10 mit einer der folgenden Strukturen:

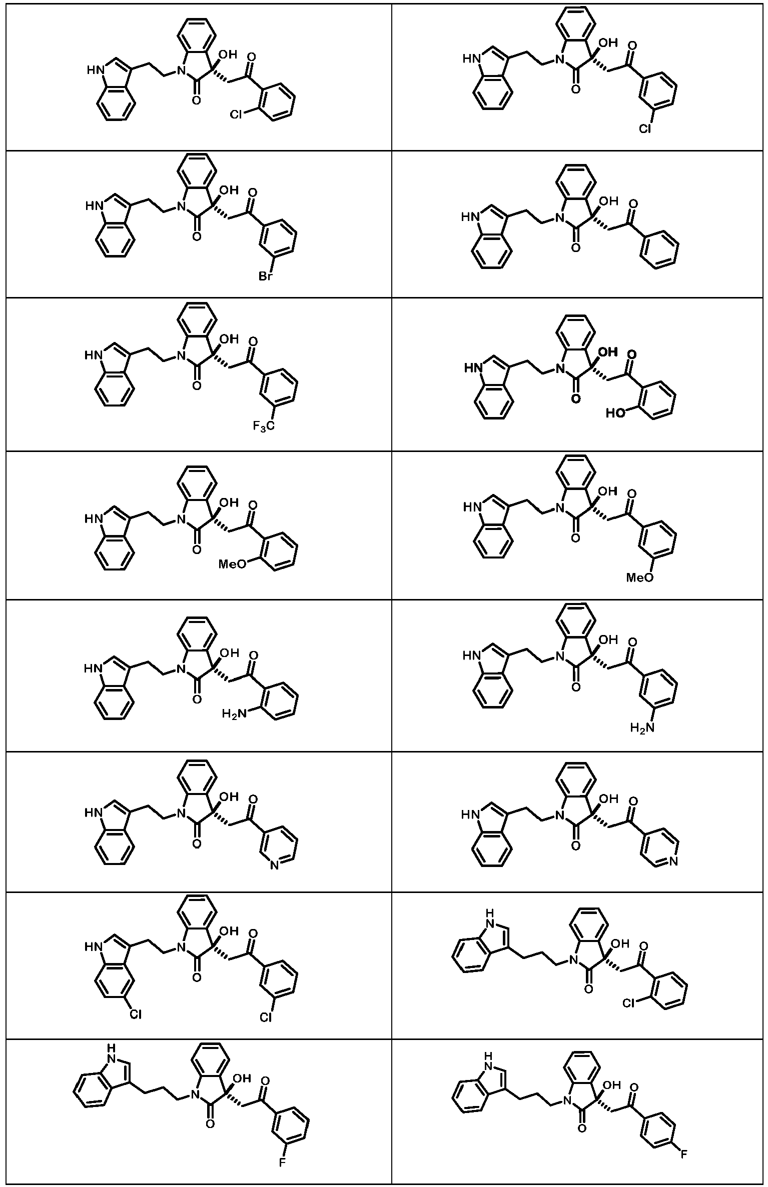

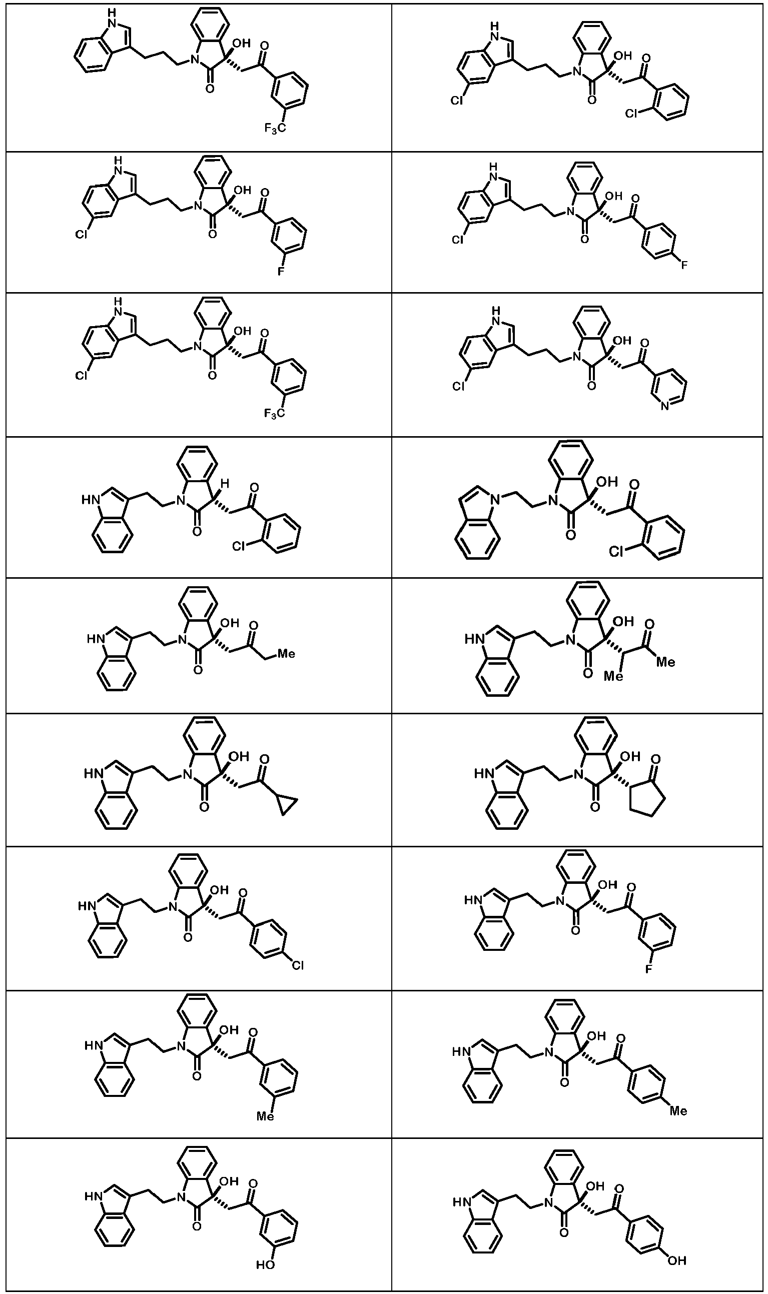

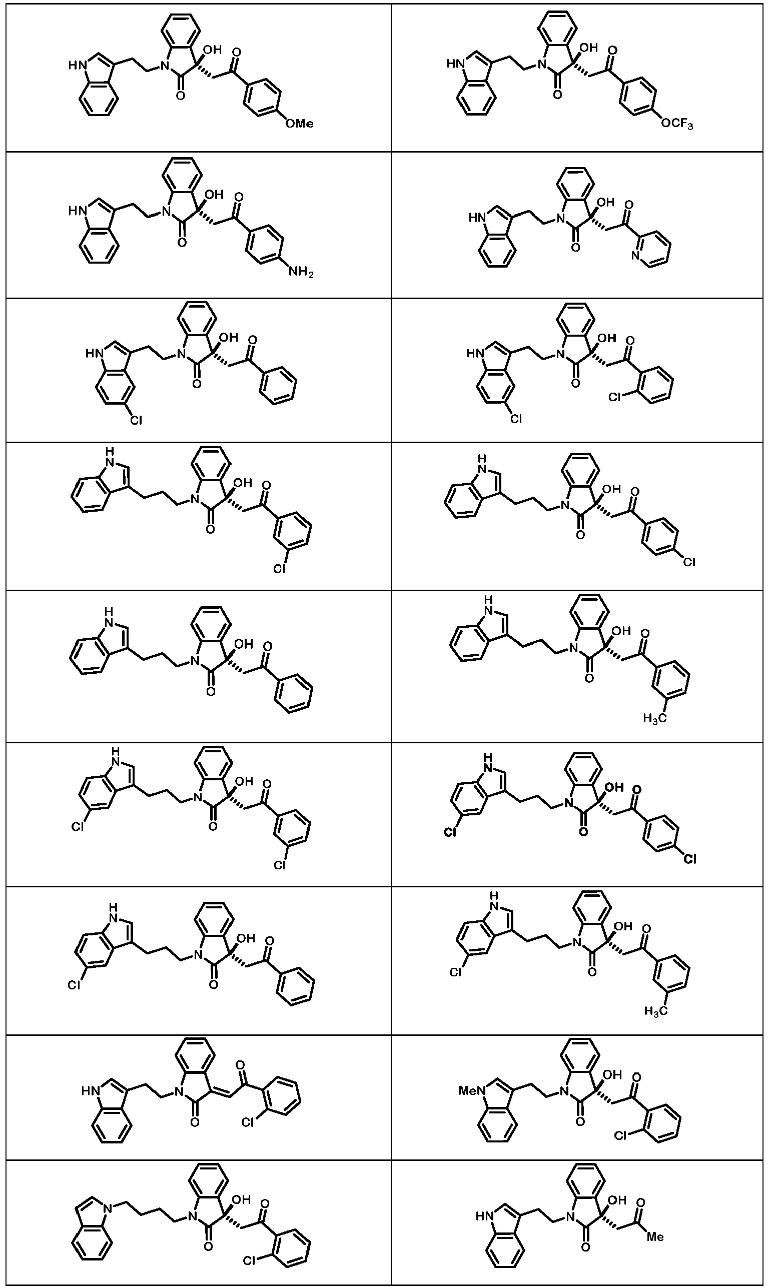

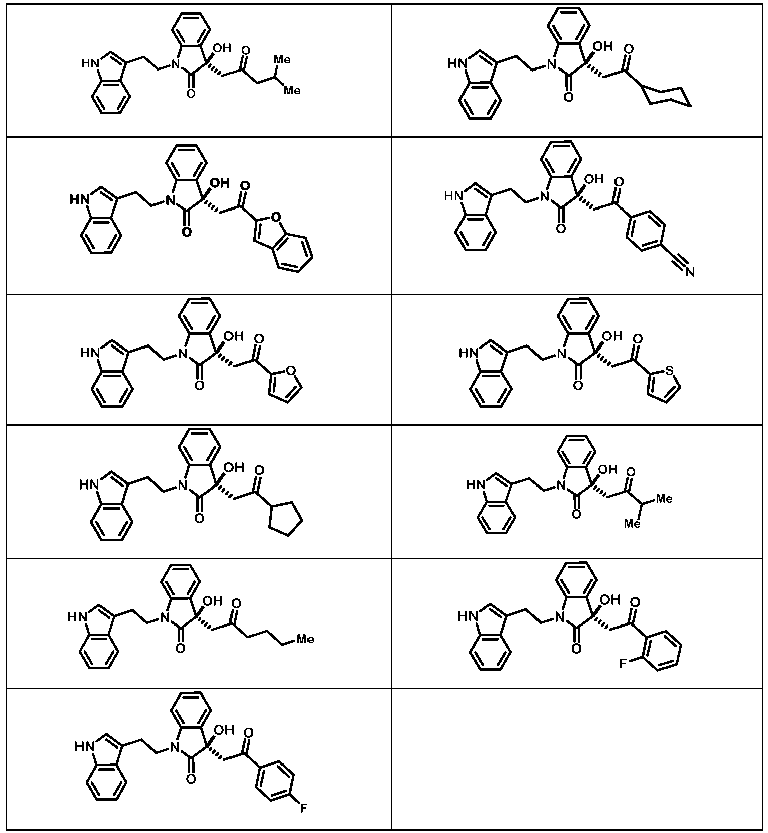

13. Pharmazeutische Zusammensetzung, umfassend eine Verbindung nach einem der Ansprüche 1 bis 11 und einen pharmazeutisch akzeptablen Träger.

14. Die Verbindung nach einem der Ansprüche 1 bis 11 zur Verwendung als Medikament.

15. Verbindung zur Verwendung nach Anspruch 13 bei der Behandlung von Krankheiten oder Pathologien, an denen ein N-Methyl-D-Aspartat-Rezeptor des Zentralnervensystems beteiligt ist, wie schwere/resistente Epilepsie und die daraus resultierenden kognitiven Störungen, einschließlich Autismus, aber auch Schlaganfälle, Schizophrenie, degenerative Krankheiten, an denen eine NMDA-Rezeptoraktivierung beteiligt ist, wie Parkinson- und Alzheimer-Krankheit, Rett-Syndrom oder amyotrophe Lateralsklerose, Migräne, Demenz und schwere Depression.

## Claims

1. A compound of formula (I): 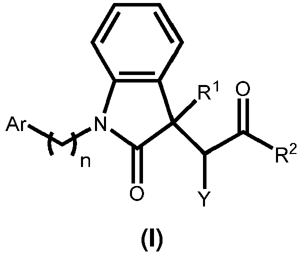 or a pharmaceutically acceptable salt thereof;
wherein :
**R1** represents H or OH;
**R2** represents a linear, branched or cyclic C₁₋₆alkyl radical; C₆₋₁₀aryl optionally substituted by one or more substituents selected from a halogen atom, a linear, branched or cyclic C₁₋₆alkyl radical, a linear, branched or cyclic C₁₋₆alkoxyl radical, a linear, branched or cyclic C₁₋₆haloalkyl radical, a linear, branched or cyclic C₁₋₆haloalkoxyl radical; a radical C₆₋₁₀heteroaryl optionally substituted by one or more substituents selected from a halogen atom, a linear, branched or cyclic C₁₋₆alkyl radical, a linear, branched or cyclic C₁₋₆alkoxyl radical, a linear, branched or cyclic or a C₂₋₅heterocycle radical, preferably C₄₋₅heterocycle, saturated or unsaturated; preferably unsaturated, comprising from 1 to 3 heteroatom(s) selected from the group consisting of O, N and S;
**Y** represents H or a linear, branched or cyclic C₁₋₆alkyl radical; or Y taken together with R² forms a 5 or 6 membered ring;
**n** is 0, 1, 2, 3 or 4;
**Ar** represents a bicyclic heterocycle of the structure: 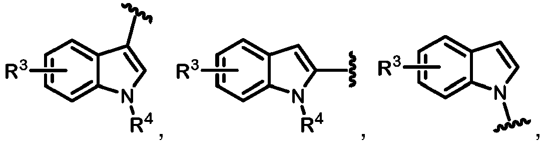 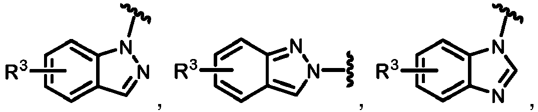 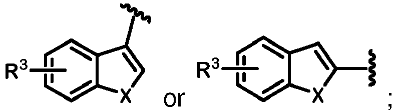 wherein:
**R³** represents H, halogen, -OR⁵ or -NR⁶AR^{6B}; wherein R⁵ represents H, linear, branched or cyclic C¹⁻⁶alkyl; and R^{6A} and R^{6B} independently represent H, linear, branched or cyclic C₁₋₆alkyl or C₆₋₁₀aryl;
**R⁴** is H; linear, branched or cyclic C₁₋₆alkyl; or -C(=O)R⁷ wherein R⁷ is linear, branched or cyclic C¹⁻⁶alkyl; and
X is O or S.

2. The compound of claim 1, having the following configuration:

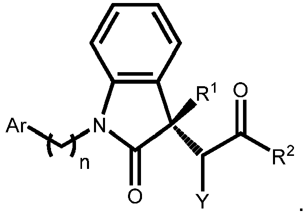

3. The compound of claim 1 or 2, wherein n is 2 or 3.

4. The compound of any one of claims 1 to 3, wherein R¹ represents OH.

5. The compound of any one of claims 1 to 4, wherein Y represents H, methyl or Y taken together with R² forms a 5-membered ring; preferably Y represents H.

6. The compound of any one of claims 1 to 4, wherein when Y taken together with R² forms a 5- or 6-membered ring; the alpha carbon of the ketone function that bears Y is a center of chirality of R or S configuration, or racemic.

7. The compound of any one of claims 1 to 6, wherein Ar has the following formula: 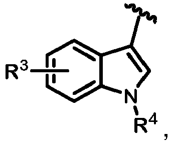 wherein
R³ represents H; a halogen atom selected from F, Cl or Br, preferably Cl; a radical -OR⁵ where R⁵ represents H, methyl or ethyl; or a radical -NR⁶AR^{6B} where R^{6A} and R^{6B} independently represent H, methyl or ethyl; and
R⁴ is H or methyl, or 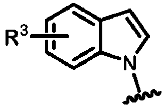 in which
R³ represents H; a halogen atom selected from F, Cl or Br, preferably Cl; a radical -OR⁵ where R⁵ represents H, methyl or ethyl; or a radical -NR⁶AR^{6B} where R^{6A} and R^{6B} independently represent H, methyl or ethyl.

8. The compound of claim 7, wherein Ar has the following formula: 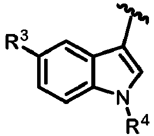 wherein R³ and R⁴ are as defined in claim 7.

9. The compound of claim 7, wherein Ar has the following formula: 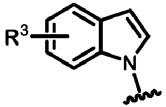 wherein R³ represents H.

10. The compound of any one of claims 1-9 wherein R2 represents:
- methyl, ethyl, propyl, n-butyl, iso-propyl, iso-butyl, cyclopropyl, cyclobutyl, cyclopentyl or cyclohexyl;
- a phenyl radical optionally substituted by one or more substituents selected from F, Cl, Br, methyl, ethyl, CN, CF₃, OH, methoxy, NH₂ or OCF₃;
- a pyridyl radical optionally substituted by one or more substituents selected from F, Cl, Br, methyl, ethyl, CF₃, OH, methoxy, NH₂ or OCF₃; or
- a benzofuryl radical of structure 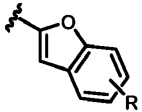 optionally substituted by one or more substituents R selected from F, Cl, Br, methyl, ethyl, CN, CF₃, OH, methoxy, NH₂ or OCF₃.
- a furyl radical of structure 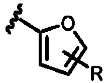 optionally substituted with one or more substituents R selected from F, Cl, Br, methyl, ethyl, CN, CF₃, OH, methoxy, NH₂ or OCF₃
- a thiophene radical of the structure 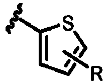 optionally substituted with one or more substituents R selected from F, Cl, Br, methyl, ethyl, CN, CF₃, OH, methoxy, NH₂ or OCF₃
- a benzofuryl radical of the structure 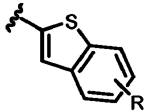 optionally substituted with one or more substituents R selected from F, Cl, Br, methyl, ethyl, CN, CF₃, OH, methoxy, NH₂ or OCF₃.

11. The compound of any one of claims 1-9 wherein R² represents:
- methyl, ethyl, iso-propyl, iso-butyl, cyclopropyl, or cyclohexyl;
- a phenyl radical unsubstituted or mono-substituted in the ortho, meta or para position by a substituent selected from F, Cl, Br, methyl, ethyl, CN, CF₃, OH, methoxy, NH₂ or OCF₃; or
- a pyridyl radical 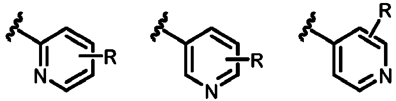 optionally substituted by one or more substituents R selected from F, Cl, Br, methyl, ethyl, CF₃, OH, methoxy, NH₂ or OCF₃, preferably H; or
- a benzofuryl radical of the structure 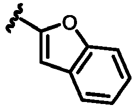
- a furyl radical of the structure

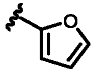

12. The compound of any one of claims 1 to 10 having one of the following structures:

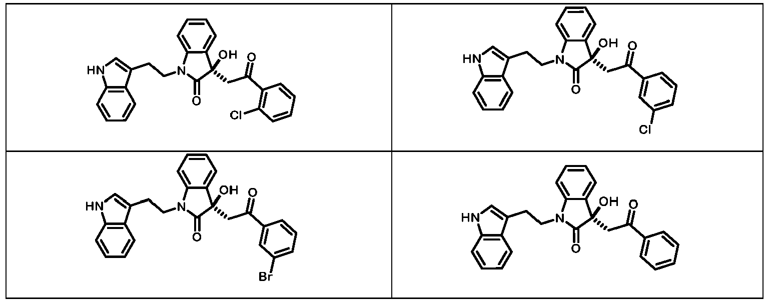

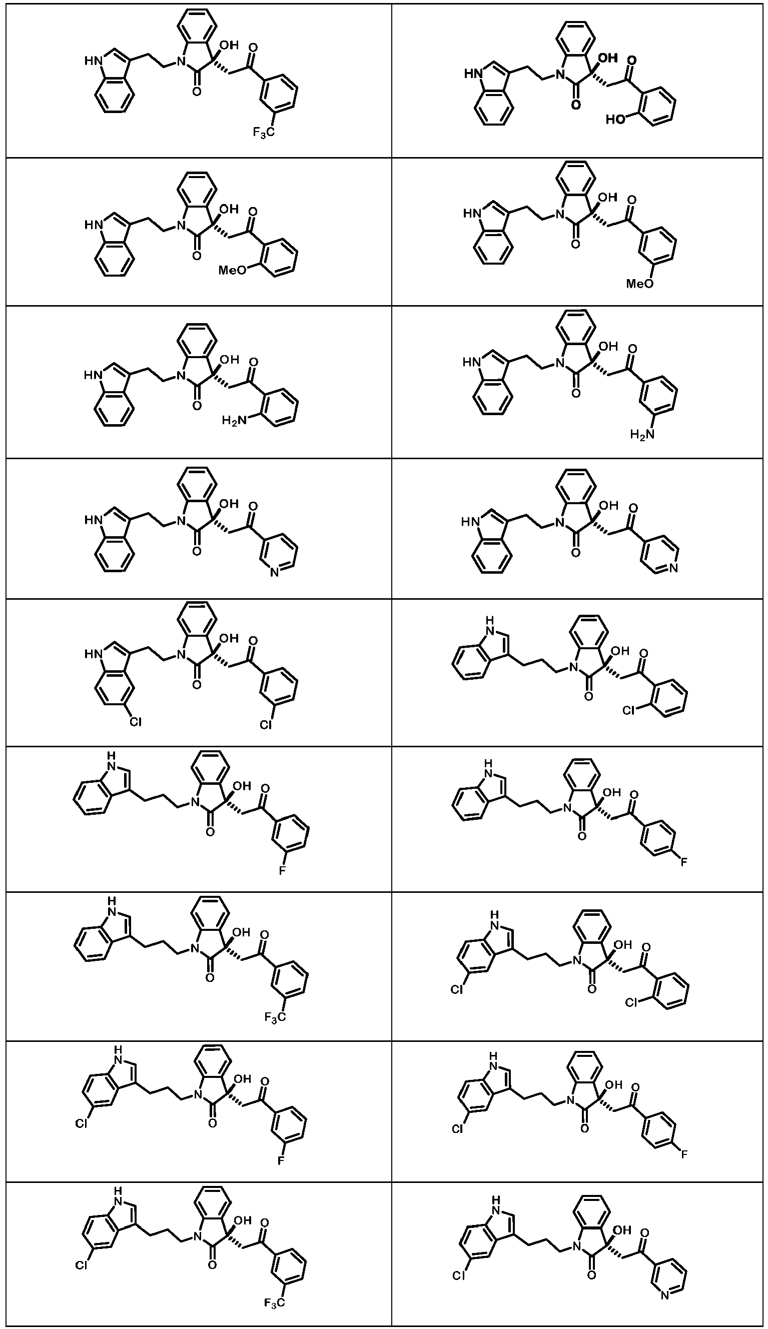

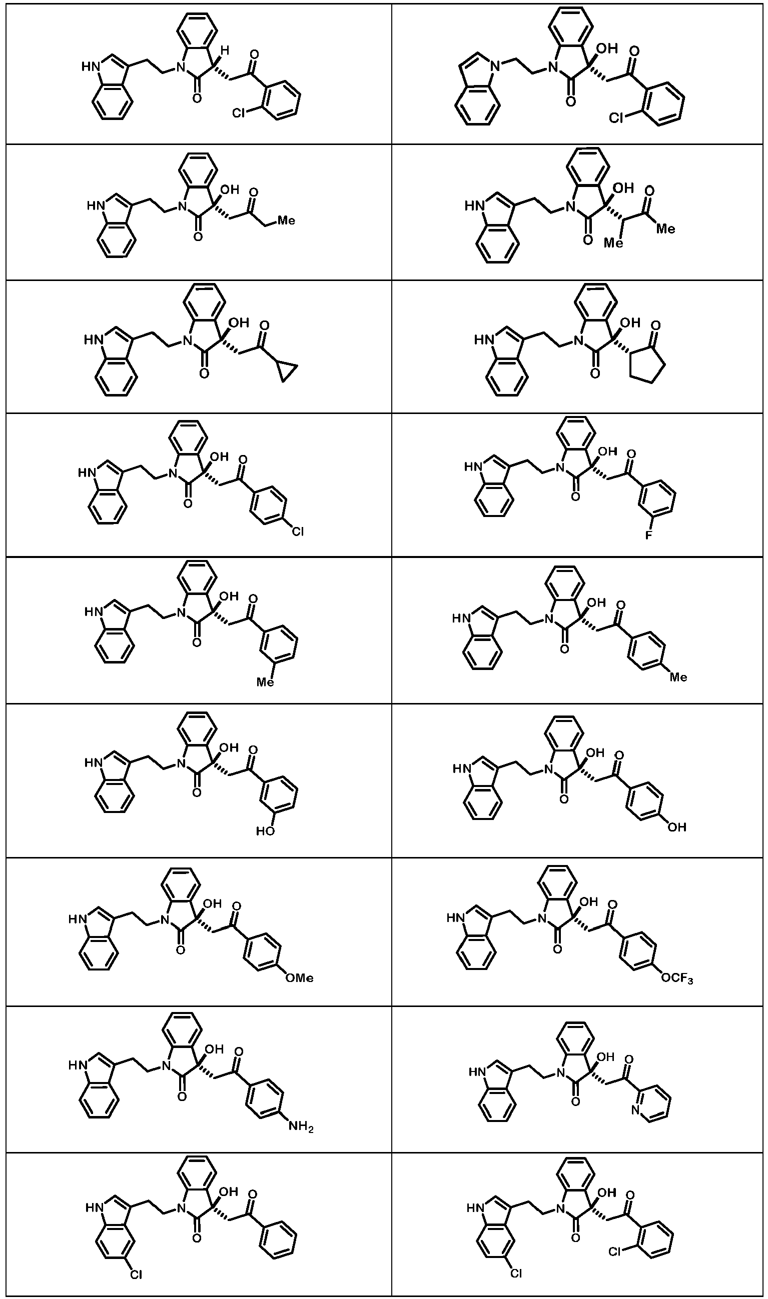

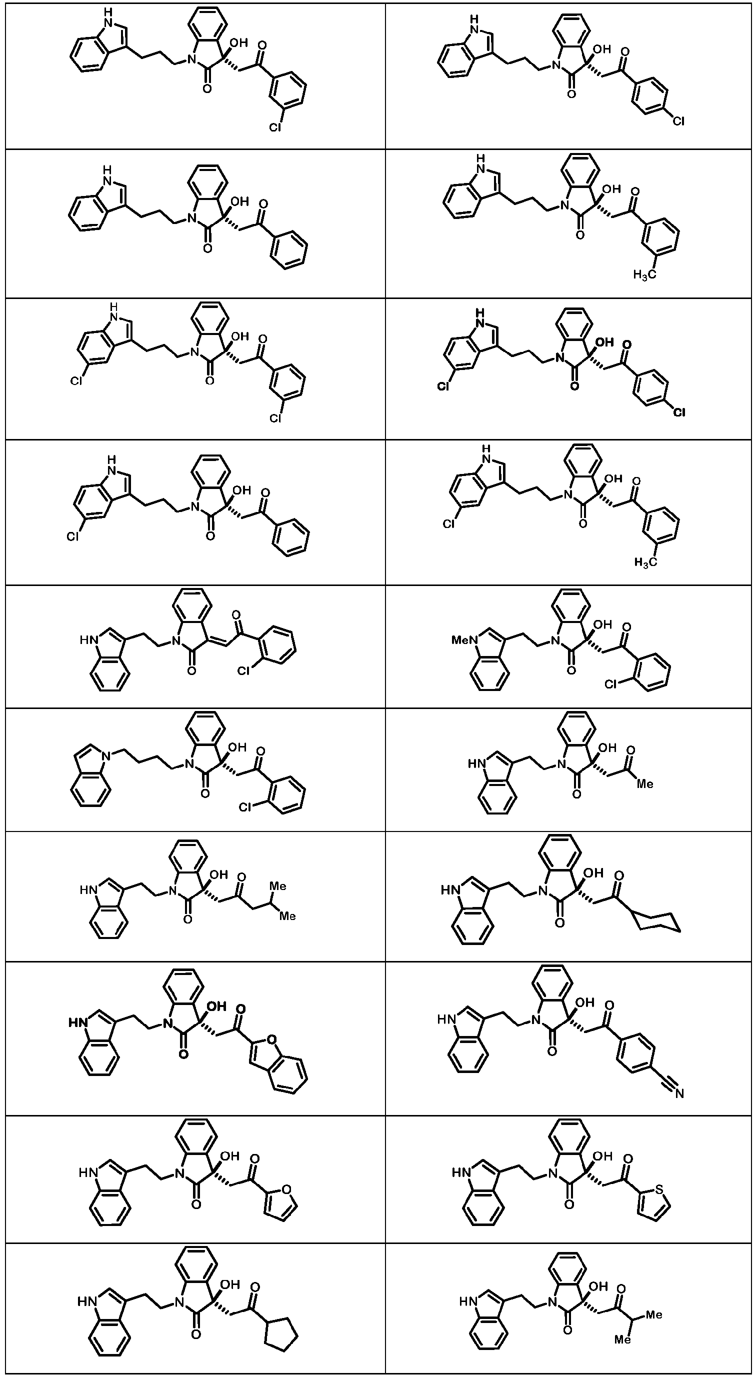

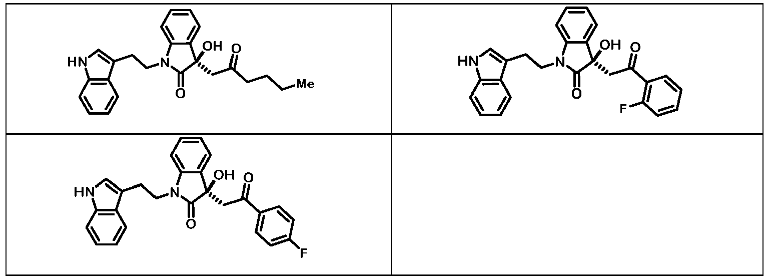

13. A pharmaceutical composition comprising a compound of any one of claims 1 to 11 and a pharmaceutically acceptable carrier.

14. The compound of any one of claims 1 to 11, for use as a medicament.

15. A compound for use according to claim 13 in the treatment of diseases or pathologies involving an N-Methyl-D-Aspartate receptor of the central nervous system, such as severe/resistant epilepsy and the resulting cognitive disorders, including autism, but also strokes, schizophrenia, degenerative diseases involving NMDA receptor activation such as Parkinson's and Alzheimer's diseases, Rett syndrome or amyotrophic lateral sclerosis, migraine, dementia and major depression.
